(19) Europäisches Patentamt
European Patent Office
Office européen des brevets

(11) **EP 4 389 771 A1**

(12) **EUROPEAN PATENT APPLICATION**
published in accordance with Art. 153(4) EPC

(43) Date of publication:
**26.06.2024 Bulletin 2024/26**

(21) Application number: **22857934.8**

(22) Date of filing: **22.08.2022**

(51) International Patent Classification (IPC):
**C07K 19/00** (2006.01)     **C07K 14/71** (2006.01)
**C07K 14/495** (2006.01)     **C07K 16/28** (2006.01)
**A61K 38/17** (2006.01)     **A61P 35/00** (2006.01)
**A61K 39/00** (2006.01)

(52) Cooperative Patent Classification (CPC):
**A61K 39/00; A61K 38/17; A61P 35/00;**
**C07K 14/495; C07K 14/71; C07K 16/28;**
**C07K 19/00;** Y02A 50/30

(86) International application number:
**PCT/CN2022/113882**

(87) International publication number:
**WO 2023/020625 (23.02.2023 Gazette 2023/08)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.08.2021   CN 202110961038**

(71) Applicant: **Akeso Biopharma, Inc.**
**Zhongshan, Guangdong 528437 (CN)**

(72) Inventors:
• **WANG, Zhongmin**
  **Guangdong 528437 (CN)**
• **ZHANG, Peng**
  **Guangdong 528437 (CN)**
• **LI, Baiyong**
  **Guangdong 528437 (CN)**
• **XIA, Yu**
  **Guangdong 528437 (CN)**

(74) Representative: **Berggren Oy**
**P.O. Box 16**
**Eteläinen Rautatiekatu 10A**
**00101 Helsinki (FI)**

Remarks:
The complete document including Reference Table(s) and the Sequence Listing(s) can be downloaded from the EPO website

(54) **FUSION PROTEIN CONTAINING ANTI-TIGIT ANTIBODY AND TGF-BETA-R, AND PHARMACEUTICAL COMPOSITION AND USE THEREOF**

(57)     A fusion protein containing an anti-TIGIT antibody and TGF-βR, and a pharmaceutical composition and the use thereof. Specifically, disclosed is a fusion protein containing a first protein functional region for targeting TIGIT, and a second protein functional region having a TGF-β binding activity, wherein the first protein functional region is an anti-TIGIT antibody or an antigen-binding fragment thereof; and the heavy chain variable region of the anti-TIGIT antibody contains HCDR1-HCDR3 with amino acid sequences as respectively shown in SEQ ID NOs: 3-5, and the light chain variable region thereof contains LCDR1-LCDR3 with amino acid sequences as respectively shown in SEQ ID NOs: 8-10. The fusion protein can simultaneously inhibit TIGIT and reduce the TGF-β level, and has good potential for preparing an anti-tumor drug.

FIG. 7

**Description**

TECHNICAL FIELD

The present invention belongs to the field of biomedicines, and relates to a fusion protein containing an anti-TIGIT antibody and TGF-βR, and a pharmaceutical composition and use thereof.

BACKGROUND

TIGIT (T cell Ig and ITIM domain, also known as WUCAM, Vstm3, or VSIG9) is a member of the poliovirus receptor (PVR)/Nectin family. TIGIT consists of an extracellular immunoglobulin variable region (IgV) domain, a type I transmembrane domain, and an intracellular domain with a classical immunoreceptor tyrosine-based inhibitory motif (ITIM) and an immunoglobulin tail tyrosine (ITT) motif. TIGIT is highly expressed in lymphocytes, especially in effector and regulatory CD4+ T cells (Treg cells), follicular helper CD4+ T cells and effector CD8+ T cells, as well as natural killer (NK) cells (Yu X, Harden K, Gonzalez L C, et al., The surface protein TIGIT suppresses T cell activation by promoting the generation of mature immunoregulatory dendritic cells. [J]. Nature immunology, 2009, 10(1):48). CD155 (also known as PVR, Necl5, or Tage4), CD112 (also known as PVRL2/nectin 2), and CD113 (also known as PVRL3) are ligands to which TIGIT binds (Martinet L, Smyth M J., Balancing natural killer cell activation through paired receptors.[J]. Nature Reviews Immunology, 2015, 15(4):243-254), wherein CD155 is a high-affinity ligand for TIGIT. In NK cells, TIGIT binding to ligands CD155 and CD112 can inhibit the killing effect of NK cells on CD155 and CD112 high expression cells (Stanietsky N, Simic H, Arapovic J, et al., The interaction of TIGIT with PVR and PVRL2 inhibits human NK cell cytotoxicity.[J]. Proceedings of the National Academy of Sciences, 2009, 106(42):17858-17863). It was reported that the killing effect of CD8+ T cells can be enhanced when PD-1 and TIGIT are blocked simultaneously (Johnston R J, Comps-Agrar L, Hackney J, et al., The immunoreceptor TIGIT regulates antitumor and antiviral CD8+ T cell effector function.[J]. Cancer cell, 2014, 26(6):923-937). Recent research revealed that TIGIT, as an immune checkpoint of NK cells, can cause NK cell exhaustion in the process of tumor development, and proved that anti-TIGIT monoclonal antibodies can reverse NK cell exhaustion and be used for immunotherapy of a variety of tumors such as non-small cell lung cancer, small cell lung cancer, breast cancer, ovarian cancer, colorectal cancer, melanoma, pancreatic cancer, cervical tumor, multiple myeloma, non-Hodgkin's lymphoma, B-lymphoma, and plasma cell cancer (Zhang Q, Bi J, Zheng X, et al., Blockade of the checkpoint receptor TIGIT prevents NK cell exhaustion and elicits potent anti-tumor immunity.[J]. Nature immunology, 2018, 19(7):723-732).

In addition, it was reported that TIGIT blockers alone or in combination with PD-1 blockers plus CD96 blockers could significantly reduce the growth of B16 melanoma in wild-type and CD155-/- mouse models (Li X-Y, Das I, Lepletier A, et al., CD155 loss enhances tumor suppression via combined host and tumor-intrinsic mechanisms. J Clin Invest, 2018, 128:2613-25). CD112R blockers alone or in combination with TIGIT blockers and/or PD-1 blockers could increase cytokine production ability of TILs in ovarian cancer, endometrial cancer, and lung tumor (Whelan S, Ophir E, Kotturi MF, et al., PVRIG and PVRL2 Are Induced in Cancer and Inhibit CD8+ T-cell function. Cancer Immunol Res, 2019, 7:257-68).

The transforming growth factor-β (TGF-β) superfamily is a class of functionally diverse cytokines, which are further divided into subfamilies such as TGF-β, activins, inhibins, growth differentiation factors (GDFs), glial cell line-derived neurotrophic factors (GDNFs), Nodal, Lefty, and anti-Miillerian hormone (Table A). Three types of proteins for TGF-β subfamilies are known as TGF-β1, TGF-β2, and TGF-β3, respectively, with TGF-β1 being the most highly expressed subtype. TGF-β1, TGF-β2, and TGF-β3, when binding to receptors, mediate a range of biological responses through Smad and non-Smad signaling pathways, including epithelial-mesenchymal transitions (EMTs), pro-tissue fibrosis, pro-angiogenesis, tumor-promoting immune escape, cancer inhibition and promotion dual action, etc. (J Massagué. TGFbeta in Cancer. [J]. Cell, 2008, 134(2):215-230).

TGF-β receptors (TGF-βR) are widely distributed on the surfaces of normal and tumor cells in humans, including 3 types of receptor superfamilies, wherein the classical TGF-β receptor family type I includes ALK1-7, wherein TGF-βRI (also written as TβRI) is also known as ALK5; the classical TGF-β receptor family type II includes TGF-βRII (also written as TβRII), ActRII, ActRIIB, AMHRII, and BMPRII; the TGF-β receptor superfamily type III includes Betaglycan (also known as TGF-βRIII) and Endoglin; wherein TGF-βRI, TGF-βRII, and TGF-βRIII are each able to bind to TGF-β1, TGF-β2, and TGF-β3 (Pawlak John B, Blobe Gerard C, TGF-β superfamily co-receptors in cancer. DevDyn, 2021). The TGF-β family and receptors are shown in Table A (summarized by review based on Carl-Henrik Heldin1 and Aristidis Moustakas. Cold Spring Harb Perspect Biol. 2016). TGF-βRI and TGF-βRII are serine/threonine protein kinase receptors. TGF-βRII, as a key molecule of signal transduction in a TGF-β signaling pathway, can bind to TGF-β with high affinity to form a heterotetramer receptor complex with TGF-βRI dimer, and then is phosphorylated through its self-phosphorylation and activates TGF-βRI. The activated TGF-βRI phosphorylates downstream Smad pathway-related proteins, regulates the transcription and translation of downstream target genes, and further causes biological responses related to diseases. TGF-βRIII has a weaker affinity for TGF-β than TGF-βRI and TGF-βRII, and has no intracellular segment, thus it cannot be connected to downstream signaling pathways. Its function is to capture and present TGF-β to TGF-βRII. Other TGF-β receptors can also bind to TGF-β (Sang Xiaohong et al., Advances in researches on small molecule inhibitors targeting TGF-β and receptors. [J]. Journal of Pharmacy, 2019(9)).

Table A: TGF-β family and receptors

| Protein name | Gene name | Other names | Receptor | |
|---|---|---|---|---|
| TGF-β1 | TGFB1 | CIF-A (cartilage-inducing factor-A), differentiation-inhibiting factor | ActRL1/ALK-1; TβRI/ALK-5 | TβRII |
| TGF-β2 | TGFB2 | G-TsF (glioblastoma-derived T-cell suppresso rfactor), BSC-1GI (BSC-1 cell-growth inhibitor), polyergin, CIF-B (cartilage-inducingfactor -B) | | |
| TGF-β3 | TGFB3 | | | |
| Inhibinα | INHA | InhibinA | ActRIB/ALK-4 | ActRIIB; ActRII |
| InhibinβA | INHBA | FRP (follicle-stimulating hormone-releasing protein), EDF (erythroid differentiation factor), XTC-MIF (Xenopus XTC cell mesoderm-inducing factor) | ActRIB/ALK-4; ActRIC/ALK-7 | |
| InhibinβB | INHBB | InhibinB and activinB or AB,a,b XTC-MIF | | |
| InhibinβC | INHBC | ActivinCc | | |
| InhibinβE | INHBE | ActivinEc | | |
| Nodal | NODAL | BMP-16 | ActRIB/ALK-4 | ActRIIB; ActRII |
| Myostatin | MSTN | GDF-8 | TβRI/ALK-5; ActRIB/ALK-4 | ActRIIB |
| BMP-2 | BMP2 | | BMPRIA/ALK-3; BMPRIB/ALK-6 | BMPRII; ActRIIB; ActRII |
| BMP-3 | BMP3 | Osteogenin | | |
| BMP-4 | BMP4 | BMP-2B | BMPRIA/ALK-3; BMPRIB/ALK-6 | BMPRII; ActRIIB; ActRII |
| BMP-5 | BMP5 | | BMPRIB/ALK-6; BMPRIA/ALK-3; ActRIA/ALK-2 | BMPRII; ActRIIB; ActRII |
| BMP-6 | BMP6 | Vgr1 (Vg1-related protein) | BMPRIB/ALK-6; BMPRIA/ALK-3; ActRIA/ALK-2 | BMPRII; ActRIIB; ActRII |
| BMP-7 | BMP7 | OP-1 (osteogenic protein-1) | BMPRIB/ALK-6; BMPRIA/ALK-3; ActRIA/ALK-2 | BMPRII; ActRIIB; ActRII |
| BMP-8A | BMP8A | OP-2 | BMPRIB/ALK-6; BMPRIA/ALK-3; ActRIA/ALK-2 | BMPRII; ActRIIB; ActRII |
| BMP-8B | BMP8B | OP-3 | BMPRIB/ALK-6; BMPRIA/ALK-3; ActRIA/ALK-2 | BMPRII; ActRIIB; ActRII |

(continued)

| Protein name | Gene name | Other names | Receptor | |
|---|---|---|---|---|
| BMP-9 | GDF2 | GDF-2 | BMPRIB/ALK-6; ActRL1/ALK-1 | BMPRII; ActRIIB; ActRII |
| BMP-10 | BMP10 | | BMPRIB/ALK-6; ActRL1/ALK-1 | BMPRII; ActRIIB; ActRII |
| GDF-1 | GDF1 | | ActRIB/ALK-4; ActRIC/ALK-7 | ActRIIB; ActRII |
| GDF-3 | GDF3 | Vgr2 | ActRIB/ALK-4; ActRIC/ALK-7 | ActRIIB; ActRII |
| GDF-5 | GDF5 | CDMP-1 (cartilage-derived morpho genetic protein-1), BMP-14 | BMPRIB/ALK-6; BMPRIA/ALK-3 | BMPRII; ActRIIB; ActRII |
| GDF-6 | GDF6 | CDMP-2, BMP-13 | BMPRIB/ALK-6; BMPRIA/ALK-3 | BMPRII; ActRIIB; ActRII |
| GDF-7 | GDF7 | CDMP-3, BMP-12 | BMPRIB/ALK-6; BMPRIA/ALK-3 | BMPRII; ActRIIB; ActRII |
| GDF-9 | GDF9 | GDF-9A | TβRI/ALK-5; ActRIB/ALK-4; ActRIC/ALK-7 | ActRIIB |
| GDF-9B | BMP15 | BMP-15 | BMPRIB/ALK-6 | |
| GDF-10 | GDF10 | BMP-3b | ActRIB/ALK-4; ActRIB/ALK-4 | ActRII |
| GDF-11 | GDF11 | BMP-11 | TβRI/ALK-5; ActRIB/ALK-4 | ActRIIB; ActRII |
| GDF-15 | GDF15 | Placental TGF-β, placental BMP (PLAB), PDF (prostate-derivedfactor), NAG-1 (nonsteroidalanti-inflam matorydrug-activatedge ne-1), MIC-1 (macrophage inhibitory cytokine-1) | BMPRIA/ALK-3 | ActRIIB |
| MIS (Miillerian-i nhibiting substance) | AMH | AMH (anti-Miillerian hormone) | ActRIA/ALK-2; | AMHRII |
| LeftyA | LEFTY2 | EBAF (endometrial bleeding-associated factor), TGF-β4, Stra3 | | |
| LeftyB | LEFTY1 | | | |

During tumor progression, tumor cells, mesenchymal fibroblasts, and other cells secrete large quantities of TGF-β in the tumor microenvironment (TME), mediating immunosuppression. TGF-β inhibits the differentiation of naive T cells to Th1 which mediates anti-tumor activity, and TGF-βRII-deficient T cells have an enhanced Th1 response (Eduard, Batlle, Joan, et al., Transforming Growth Factor-β Signaling in Immunity and Cancer. [J]. Immunity, 2019). Granzyme A, granzyme B, perforin, γ-interferon (IFN-y) and FasL of effector T cells having tumor killing activity have reduced expression levels under the action of TGF-β, resulting in immune escape of tumor cells. Tregs are key cells for mediating tumor immunosuppression by a tumor microenvironment (TME) and capable of inhibiting the functions of effector T cells with tumor killing activity; TGF-β produced by tumor cells induces the emergence of a large quantity of Tregs in the TME, enhancing the tolerance of tumor antigens and promoting the generation of tumor immune escape (Chen Dan, Ran Yan. Advance in researches on the regulation and control of tumor cells and tumor-associated immune cells by TGF-β.[J]. Modern Medicine & Health, 2020, 36(09):1354-1358). In-research antibody-based pharmaceuticals targeting TGF-β molecules, which are currently under clinical trials, are indicated for melanoma, renal cell carcinoma, breast cancer, cervical cancer, advanced non-small cell lung cancer, prostate cancer, pancreatic ductal adenocarcinoma, advanced solid tumors, and metastatic solid tumors.

Fc receptors belong to an immunoglobulin family that are expressed on the surfaces of specific immune cells to recognize antibody Fc regions and mediate immune responses. After the Fab region recognizes an antigen, the Fc region of the antibody binds to the Fc receptor on the immune cell (e.g., a killer cell) to initiate the response function of the immune cell, such as phagocytosis and ADCC.

According to the type of antibody recognized by the Fc receptor and the type of expression cells, Fc receptors are mainly classified into three types, FcγR, FcαR and FcεR. FcγR can be further classified into four subtypes, FcγRI (CD64), FcγRII (CD32), FcγRIII (CD16) and FcRn (neonatal Fc receptor). Among these, FcγRI, FcγRII and FcγRIII are closely associated with ADCC effects. FcγRIII is the most predominant molecule mediating ADCC, with two highly homologous subtypes, FcγRIIIa and FcγRIIIb, in different cell types. In FcγRIIIa populations, two subtypes distinguished by sites of single-nucleotide polymorphism (SNP), FcγRIIIa_V158 with high affinity and FcγRIIIa_F158 with low affinity, are present. FcγRI has higher affinity for the Fc region of IgG and participates in ADCC process; FcγRII comprises three subtypes, FcγRIIa, FcγRIIb and FcγRIIc (also referred to as CD32a, CD32b and CD32c, respectively), among which FcγRIIa has ADCC activity; for FcγRIIa, two subtypes, FcγRIIa_H131 and FcγRIIa_R131, are present in humans due to single nucleotide mutation; FcγRIIb is an inhibitory receptor, and is a typical inhibitory FcγR that inhibits nearby ITAM pathways. For example, after the binding of the immune complex to BCR, the Fc fragment binds to FcγRIIb on the same cell, negatively regulating B cell activation and decreasing secretion of antibodies and cytokines (Hogarth PM, Pietersz GA., 2012, NATURE REVIEWS DRUG DISCOVERY, 11(4):311-331).

Currently, there is a need to develop a drug that inhibits both TIGIT and TGF-β signaling pathways.

SUMMARY

The inventor has made a fusion protein containing an anti-TIGIT antibody and TGF-βR through intensive research and creative work, and surprisingly found that the fusion protein can effectively bind to TIGIT and TGF-β at the same time and has the potential of preparing anti-tumor drugs. The present invention is detailed below.

One aspect of the present invention relates to a fusion protein, comprising:

a first protein functional region targeting TIGIT, and
a second protein functional region having TGF-β binding activity;
wherein,
the first protein functional region is an anti-TIGIT antibody or an antigen-binding fragment thereof;
a heavy chain variable region of the anti-TIGIT antibody comprises HCDR1-HCDR3 with amino acid sequences set forth in SEQ ID NOs: 3-5, respectively, and a light chain variable region thereof comprises LCDR1-LCDR3 with amino acid sequences set forth in SEQ ID NOs: 8-10, respectively.

In some embodiments of the present invention, the fusion protein is provided, wherein the heavy chain variable region of the anti-TIGIT antibody comprises an amino acid sequence selected from SEQ ID NO: 1, SEQ ID NO: 11, SEQ ID NO: 13, SEQ ID NO: 15, and SEQ ID NO: 17; and

the light chain variable region of the anti-TIGIT antibody comprises an amino acid sequence selected from SEQ ID NO: 6, SEQ ID NO: 19, SEQ ID NO: 21, SEQ ID NO: 23, and SEQ ID NO: 25.

In some embodiments of the present invention, the fusion protein is provided, wherein

the heavy chain variable region of the anti-TIGIT antibody comprises an amino acid sequence set forth in SEQ ID NO: 1, and the light chain variable region thereof comprises an amino acid sequence set forth in SEQ ID NO: 6;
the heavy chain variable region of the anti-TIGIT antibody comprises an amino acid sequence set forth in SEQ ID NO: 11, and the light chain variable region thereof comprises an amino acid sequence set forth in SEQ ID NO: 19;
the heavy chain variable region of the anti-TIGIT antibody comprises an amino acid sequence set forth in SEQ ID NO: 17, and the light chain variable region thereof comprises an amino acid sequence set forth in SEQ ID NO: 19;
the heavy chain variable region of the anti-TIGIT antibody comprises an amino acid sequence set forth in SEQ ID NO: 13, and the light chain variable region thereof comprises an amino acid sequence set forth in SEQ ID NO: 21;
the heavy chain variable region of the anti-TIGIT antibody comprises an amino acid sequence set forth in SEQ ID NO: 13, and the light chain variable region thereof comprises an amino acid sequence set forth in SEQ ID NO: 23;

the heavy chain variable region of the anti-TIGIT antibody comprises an amino acid sequence set forth in SEQ ID NO: 15, and the light chain variable region thereof comprises an amino acid sequence set forth in SEQ ID NO: 21;
the heavy chain variable region of the anti-TIGIT antibody comprises an amino acid sequence set forth in SEQ ID NO: 15, and the light chain variable region thereof comprises an amino acid sequence set forth in SEQ ID NO: 23;
the heavy chain variable region of the anti-TIGIT antibody comprises an amino acid sequence set forth in SEQ ID NO: 11, and the light chain variable region thereof comprises an amino acid sequence set forth in SEQ ID NO: 25; or
the heavy chain variable region of the anti-TIGIT antibody comprises an amino acid sequence set forth in SEQ ID NO: 17, and the light chain variable region thereof comprises an amino acid sequence set forth in SEQ ID NO: 25.

In some embodiments of the present invention, the fusion protein is provided, wherein the anti-TIGIT antibody or the antigen-binding fragment thereof is selected from Fab, Fab', F(ab')$_2$, Fd, Fv, dAb, a complementarity determining region fragment, a single chain antibody, a humanized antibody, a chimeric antibody, and a diabody.

In some embodiments of the present invention, the fusion protein is provided, wherein the anti-TIGIT antibody comprises a non-CDR region derived from a species other than murine, such as from a human antibody.

In some embodiments of the present invention, the fusion protein is provided, wherein a heavy chain constant region of the anti-TIGIT antibody is Ig gamma-1 chain C region (e.g., NCBI ACCESSION: P01857), or Ig gamma-4 chain C region (e.g., NCBI ACCESSION: P01861.1); a light chain constant region thereof is Ig kappa chain C region (e.g., NCBI ACCESSION: P01834). In some embodiments of the present invention, the fusion protein is provided, wherein the fusion protein has a stronger capacity of binding to the ligand CD155-hFc-Biotin than the control antibody RG6058(hG4).

In some embodiments of the present invention, the fusion protein is provided, wherein the fusion protein binds to TIGIT-mFc with an EC$_{50}$ of less than 0.08 nM or less than 0.10 nM; preferably, the EC$_{50}$ is measured by indirect ELISA. In some embodiments of the present invention, the fusion protein is provided, wherein the fusion protein binds to TGF-β1, TGF-β2, and/or TGF-β3 with an EC$_{50}$ of less than 0.3 nM, less than 0.4 nM, less than 0.5 nM, or less than 0.6 nM; preferably, the EC$_{50}$ is measured by indirect ELISA.

In some embodiments of the present invention, the fusion protein is provided, wherein the anti-TIGIT antibody is an antibody produced by a hybridoma cell line LT019 deposited at China Center for Type Culture Collection (CCTCC) under CCTCC NO. C2020208.

In some embodiments of the present invention, the fusion protein is provided, wherein

according to the EU numbering system, the heavy chain constant region of the anti-TIGIT antibody has one of the combinations of the following mutations: L234A and L235A;
L234A and G237A;
L235A and G237A; or
L234A, L235A, and G237A.

In some embodiments of the present invention, the fusion protein is provided, wherein

the second protein functional region is a TGF-β receptor, an extracellular region thereof, or a truncated fragment of a TGF-β receptor extracellular region with TGF-β receptor functions;
preferably, the TGF-β receptor is TGF-βRI, TGF-βRII, or TGF-βRIII, or is an extracellular region of TGF-βRI, TGF-βRII, or TGF-βRIII, or is a truncated fragment of the extracellular region of TGF-βRI, TGF-βRII, or TGF-βRIII;
preferably, the second protein functional region comprises an amino acid sequence set forth in any one of SEQ ID NOs: 33-39.

In some embodiments of the present invention, the fusion protein is provided, wherein
a heavy chain of the anti-TIGIT antibody comprises an amino acid sequence set forth in SEQ ID NO: 27 or SEQ ID NO: 31, and a light chain thereof comprises an amino acid sequence set forth in SEQ ID NO: 29.

In some embodiments of the present invention, the fusion protein is provided, wherein the first protein functional region and the second protein functional region are linked directly or via a linker fragment.

In some embodiments of the present invention, the fusion protein is provided, wherein the linker fragment is (GGGGS)n or (GGGGS)nG, n being a positive integer; preferably, n is 1, 2, 3, 4, 5, or 6.

In some embodiments of the present invention, the fusion protein is provided, wherein the numbers of the first protein functional region and the second protein functional region are each independently 1, 2, or more.

In some embodiments of the present invention, the fusion protein is provided, wherein the TGF-β receptor or the extracellular fragment thereof is linked to the C-terminus of the heavy chain of the anti-TIGIT antibody.

The present invention relates to a fusion protein, comprising:

a first protein functional region targeting TIGIT, and

a second protein functional region having TGF-β binding activity;

wherein,

the number of the first protein functional region is 1, and the number of the second protein functional region is 2;

the first protein functional region is an anti-TIGIT antibody or an antigen-binding fragment thereof, and the second protein functional region is a TGF-β receptor, an extracellular region thereof, or a truncated fragment of a TGF-β receptor extracellular region with TGF-β receptor functions;

a heavy chain of the anti-TIGIT antibody comprises an amino acid sequence set forth in SEQ ID NO: 27 or SEQ ID NO: 31, and a light chain thereof comprises an amino acid sequence set forth in SEQ ID NO: 29;

the second protein functional region comprises an amino acid sequence set forth in SEQ ID NO: 33;

the second protein functional region is linked to the C-terminus of the heavy chain of the anti-TIGIT antibody via a linker fragment;

preferably, the linker fragment comprises an amino acid sequence set forth in SEQ ID NO: 44.

In some embodiments of the present invention, the fusion protein is used for treating and/or preventing a tumor;

preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;

preferably, the non-small cell lung cancer is advanced non-small cell lung cancer.

In a broad sense, the fusion protein of the present invention may also be referred to as an antibody.

Another aspect of the present invention relates to an isolated nucleic acid molecule encoding the fusion protein according to any embodiment of the present invention.

Yet another aspect of the present invention relates to a vector comprising the isolated nucleic acid molecule of the present invention.

Yet another aspect of the present invention relates to a host cell comprising the isolated nucleic acid molecule of the present invention or the vector of the present invention.

Yet another aspect of the present invention relates to a conjugate comprising a fusion protein moiety and a conjugated moiety, wherein the fusion protein moiety is the fusion protein according to any embodiment of the present invention, and the conjugated moiety is a detectable label; preferably, the conjugated moiety is a radioisotope, a fluorescent substance, a luminescent substance, a colored substance, or an enzyme.

In some embodiments of the present invention, the conjugate is used for treating and/or preventing a tumor;

preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;

preferably, the non-small cell lung cancer is advanced non-small cell lung cancer.

Yet another aspect of the present invention relates to use of the fusion protein of the present invention or the conjugate of the present invention in the preparation of a kit for detecting the presence or level of TIGIT and/or TGF-β in a sample.

Yet another aspect of the present invention relates to a pharmaceutical composition comprising the fusion protein according to any embodiment of the present invention or the conjugate of the present invention; optionally, the pharmaceutical composition further comprises a pharmaceutically acceptable carrier and/or excipient.

In some embodiments of the present invention, the pharmaceutical composition further comprises one or more anti-tumor chemotherapeutic drugs;

preferably, the anti-tumor chemotherapeutic drug is a tyrosine kinase inhibitor; more preferably, the anti-tumor chemotherapeutic drug is anlotinib or a pharmaceutically acceptable salt thereof (e.g., hydrochloride salt), or lenvatinib or a pharmaceutically acceptable salt thereof (e.g., mesylate salt).

In some embodiments of the present invention, the pharmaceutical composition is provided, wherein the unit dose of the pharmaceutical composition is 100 mg-1500 mg, 200 mg-1000 mg, 200 mg-800 mg, 300 mg-600 mg, 400 mg-500 mg, or 450 mg, based on the mass of the fusion protein.

In some embodiments of the present invention, the pharmaceutical composition is an injection.

Yet another aspect of the present invention relates to a combination product comprising a first product and a second product in separate packages, wherein,

the first product comprises the fusion protein according to any embodiment of the present invention, the conjugate of the present invention, or the pharmaceutical composition according to any embodiment of the present invention; the second product comprises one or more anti-tumor chemotherapeutic drugs; preferably, the anti-tumor chemotherapeutic drug is a tyrosine kinase inhibitor; more preferably, the anti-tumor chemotherapeutic drug is anlotinib or a pharmaceutically acceptable salt thereof (e.g., hydrochloride salt), or lenvatinib or a pharmaceutically acceptable salt thereof (e.g., mesylate salt);

preferably, the first product and the second product further independently comprise one or more pharmaceutically acceptable auxiliary materials; preferably, the combination product further comprises a package insert.

In some embodiments of the present invention, the combination product is provided, wherein the unit dose of the first product is 100 mg-1500 mg, 200 mg-1000 mg, 200 mg-800 mg, 300 mg-600 mg, 400 mg-500 mg, or 450 mg, based on the mass of the fusion protein.

In some embodiments of the present invention, the combination product is provided, wherein the unit dose of the second product is 0.1 mg-100 mg, 0.5 mg-50 mg, 1 mg-20 mg, 2 mg-15 mg, 4 mg-12 mg, or 8 mg-12 mg, based on the mass of an active ingredient.

In some embodiments of the present invention, the therapeutic combination is provided, wherein the first product and the second product are independently injections.

Yet another aspect of the present invention relates to use of the fusion protein according to any embodiment of the present invention or the conjugate of the present invention in the preparation of a medicament for treating and/or preventing a tumor;

preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;

preferably, the non-small cell lung cancer is advanced non-small cell lung cancer.

Yet another aspect of the present invention relates to a method for treating and/or preventing a tumor, comprising a step of administering to a subject in need thereof an effective amount of the fusion protein according to any embodiment of the present invention or the conjugate of the present invention; preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;

preferably, the non-small cell lung cancer is advanced non-small cell lung cancer.

In some embodiments of the present invention, the method is provided, wherein a step of administering to a subject in need thereof an effective amount of the pharmaceutical composition according to any embodiment of the present invention is before or after surgical treatment, and/or before or after radiation treatment.

In some embodiments of the present invention, the method is provided, wherein

the unit dose of the fusion protein of the present invention is 0.1-100 mg, preferably 1-10 mg per kg body weight; or, the unit dose of the fusion protein is 10-1000 mg, preferably 50-500 mg, 100-400 mg, 150-300 mg, 150-250 mg, or 200 mg in each subject;

preferably, the dose is given once every 3 days, 4 days, 5 days, 6 days, 10 days, 1 week, 2 weeks, or 3 weeks;

preferably, the route of administration is intravenous drip infusion or intravenous injection.

The variable regions of the light chain and the heavy chain determine the binding of the antigen; the variable region of each chain contains three hypervariable regions called complementarity determining regions (CDRs), wherein CDRs of the heavy chain (H) comprise HCDR1, HCDR2, and HCDR3, and CDRs of the light chain (L) comprise LCDR1, LCDR2, and LCDR3. In the present invention, the CDRs are defined by the IMGT numbering system, see Ehrenmann F, Kaas Q, and Lefranc M P., IMGT/3Dstructure-DB and IMGT/DomainGapAlign: a database and a tool for immunoglobulins or antibodies, T cell receptors, MHC, IgSF and MhcSF.[J]. Nucleic acids research, 2009; 38(suppl_1):D301-D307.

The amino acid sequences of the CDRs of the monoclonal antibody below are analyzed by technical means well known to those skilled in the art, for example, according to the IMGT definition, and the results are as follows:

HCDRs and LCDRs of mouse TIGIT monoclonal antibody
HCDR1: GHSFTSDYA (SEQ ID NO: 3)
HCDR2: ISYSDST (SEQ ID NO: 4)
HCDR3: ARLDYGNYGGAMDY (SEQ ID NO: 5)

LCDR1: QHVSTA (SEQ ID NO: 8)
LCDR2: SAS (SEQ ID NO: 9)
LCDR3: QQHYITPWT (SEQ ID NO: 10)

Three HCDRs and three LCDRs of a humanized TIGIT monoclonal antibody are the same as those of a mouse TIGIT monoclonal antibody.

In the present invention, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present invention, the definitions and explanations of the relevant terms are provided below.

As used herein, the term $EC_{50}$ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into $\kappa$ and $\lambda$ light chains. Heavy chains are classified into $\mu$, $\delta$, y, $\alpha$, or $\varepsilon$. Isotypes of antibodies are defined as IgM, IgD, IgG, IgA, and IgE. In light chains and heavy chains, the variable region and the constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of classical complement system. The VH and VL regions can be further subdivided into hypervariable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol., 1987; 196:901-917; Chothia et al., Nature, 1989; 342:878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF.[J]. Nucleic acids research, 2009; 38(suppl_1):D301-D307.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the term "mAb" or "monoclonal antibody" refers to an antibody or a fragment of an antibody that is derived from a group of highly homologous antibody molecules, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C., Continuous cultures of fused cells secreting antibody of predefined specificity.[J]. Nature, 1975; 256(5517):495), but can also be obtained using recombinant DNA technology (see, e.g., U.S. Patent 4,816,567).

As used herein, the term "humanized antibody" refers to an antibody or antibody fragment obtained when all or a part of CDRs of a human immunoglobulin (receptor antibody) are replaced by the CDRs of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321:522-525; Reichmann et al., Nature, 1988; 332:323-329; Presta, Curr. Op. Struct. Biol., 1992; 2:593-596; and Clark, Immunol. Today, 2000; 21:397-402. In some cases, the antigen-binding fragment of the antibody is a diabody, in which the $V_H$ and $V_L$ domains are expressed on a single polypeptide chain. However, the linker used is too short to allow the pairing of the two domains on the same chain. Thus the domains are forced to pair with the complementary domains on the other chain and two antigen-binding sites are generated (see, e.g., Holliger P. et al., Proc. Natl. Acad. Sci. USA, 1993; 90:6444-6448 and Poljak R.J. et al., Structure, 1994; 2:1121-1123).

The fusion protein as described herein is a protein product of co-expression of two genes by DNA recombination.

Methods for producing and purifying antibodies and antigen-binding fragments are well known in the art, for example, those described in chapters 5-8 and 15 of Antibodies: A Laboratory Manual, Cold Spring Harbor Press.

As used herein, the term "single chain fragment variable (ScFv)" refers to a molecule in which the antibody heavy chain variable region ($V_H$) and the antibody light chain variable region ($V_L$) are linked by a linker. The $V_L$ and $V_H$ domains are paired to form a monovalent molecule by a linker that enables them to produce a single polypeptide chain (see, e.g., Bird et al., Science, 1988; 242:423-426 and Huston et al., Proc. Natl. Acad. Sci. USA, 1988; 85:5879-5883). Such scFv molecules may have the general structure: NH2-$V_L$-linker fragment-$V_H$-COOH or NH2-$V_H$-linker fragment-$V_L$-COOH. An appropriate linker in prior art consists of GGGGS amino acid sequence repeats or a variant thereof. For example, a linker having the amino acid sequence (GGGGS)$_4$ may be used, but variants thereof may also be used (Holliger et al., Proc. Natl. Acad. Sci. USA, 1993; 90:6444-6448). Other linkers that can be used in the present invention are described by Alfthan et al., Protein Eng., 1995; 8:725-731, Choi et al., Eur. J. Immunol., 2001; 31:94-106, Hu et al., Cancer Res., 1996; 56:3055-3061, Kipriyanov et al., J. Mol. Biol., 1999; 293:41-56 and Roovers et al., Cancer Immunology, Immunotherapy, 2001, 50(1):51-59.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, GS cells, COS cells, NSO cells, HeLa cells, BHK cells, HEK 293 cells, or human cells.

In the present invention, the term "ADCP" refers to antibody-dependent cellular phagocytosis. The Fc fragment of an antibody that binds to a cell surface antigen binds to the Fc receptor of a phagocytically active cell, such as a macrophage, which in turn mediates phagocytosis of the antibody-bound cells by phagocytic cells.

In the present invention, the term "ADCC" refers to antibody-dependent cell-mediated cytotoxicity. The Fab fragment of an antibody binds to an epitope of a virus-infected cell or a tumor cell, and the Fc fragment of the antibody binds to an Fc receptor (FcR) on the surface of a killer cell (NK cell, macrophage, etc.) to mediate direct killing of the target cells by killer cells.

In the present invention, the term "CDC" refers to complement-dependent cytotoxicity. When an antibody specifically binds to a corresponding antigen on a cell membrane surface, a complex is formed and the complement system is activated, and then an MAC is formed on the surface of a target cell, resulting in subsequent target cell lysis. Complements may cause lysis of various bacteria and other pathogenic organisms, and are an important defense mechanism against pathogenic organism infections.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity ($K_D$) of less than about $10^{-5}$ M, e.g., less than about $10^{-6}$ M, $10^{-7}$ M, $10^{-8}$ M, $10^{-9}$ M, or $10^{-10}$ M or less.

As used herein, the term "$K_D$" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, an antibody binds to an antigen (e.g., TIGIT protein) with a dissociation equilibrium constant ($K_D$) of less than about $10^{-5}$ M, e.g., less than about $10^{-6}$ M, $10^{-7}$ M, $10^{-8}$ M, $10^{-9}$ M, or $10^{-10}$ M or less. $K_D$ can be determined using methods known to those skilled in the art, e.g., using a Fortebio system.

As used herein, the terms "monoclonal antibody" and "mAb" have the same meaning and can be used interchangeably; the terms "polyclonal antibody" and "pAb" have the same meaning and can be used interchangeably. Besides, as used herein, amino acids are generally represented by single-letter or three-letter abbreviations known in the art. For example, alanine can be represented by A or Ala.

As used herein, the term "pharmaceutically acceptable auxiliary material" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to: pH regulators, surfactants, adjuvants and ionic strength enhancers. For example, the pH regulators include, but are not limited to phosphate buffer; the surfactants include, but are not limited to cationic, anionic, or non-ionic surfactants, such as Tween-80; the ionic strength enhancers include, but are not limited to sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop the disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

In the present invention, the terms "first" (e.g., first protein functional region, or first product) and "second" (e.g., second protein functional region, or second product) are used for distinguishing or clarity in expression and do not carry typical sequential meanings, unless otherwise specified.

Beneficial effects of the present invention

The present invention achieves any one or more of the following technical effects (1) to (9):

(1) The fusion proteins of the present invention (e.g., TF01 or TF02) can simultaneously inhibit TIGIT and reduce TGF-β level.

(2) The fusion proteins of the present invention (e.g., TF01 or TF02) can very specifically bind to TIGIT, and can very effectively block the binding of TIGIT to CD155, and specifically relieve the immunosuppression of TIGIT on the organism.

(3) The fusion proteins of the present invention (e.g., TF01 or TF02) can very specifically bind to TGF-β, and can very effectively block the binding of TGF-β to a TGF-β receptor, and specifically relieve the immunosuppression of the organism by TGF-β and activate immune responses.

(4) The fusion proteins of the present invention have good potential for preparing anti-tumor drugs.

(5) The first protein functional region and the second protein functional region of the fusion proteins of the present invention have a synergistic effect which is superior to that of the fusion protein in which one of the protein functional regions is used alone.

(6) The fusion proteins of the present invention, particularly TF01, completely eliminate the binding activity thereof to Fc receptors FcγRI, FcγRIIb, FcγRIIa_H131 and/or FcγRIIa_R131, FcγRIIIa_V158 and/or FcγRIIIa_F158, and further eliminate the ADCC activity or ADCP activity thereof.

(7) The fusion proteins of the present invention, particularly TF01, completely eliminate the binding activity thereof to a complement C1q, and further completely eliminate the CDC activity thereof.

(8) The fusion proteins of the present invention, particularly TF01 and TF02, both can effectively block the immunosuppression of immune cells induced by TIGIT and induce the cells to secrete IL-2.

(9) The fusion proteins of the present invention, particularly TF01, can effectively block the immunosuppression of immune cells induced by TGF-β and induce the cells to secrete INF-y.

BRIEF DESCRIPTION OF THE DRAWINGS

FIG. 1: Assays for binding activity of TF01, TF02, 26B12H2L2(hG4DM), and RG6058(hG4) to an antigen TIGIT-mFc.

FIG. 2: Assays for binding activity of TF01, TF02, and TGF-βRII-mFc to TGF-β1·

FIG. 3: Assays for binding activity of TF01, TF02, and TGF-βRII-mFc to TGF-β2.

FIG. 4: Assays for binding activity of TF01, TF02, and TGF-βRII-mFc to TGF-β3.

FIG. 5: Assays for activity of the fusion protein of the anti-TIGIT antibody and TGF-βR competing with TGF-βRII-His-Biotin for binding to human TGF-β1.

FIG. 6: Assays for activity of the fusion protein of the anti-TIGIT antibody and TGF-βR competing with TGF-βRII-His-Biotin for binding to human TGF-β3.

FIG. 7: Assays for activity of the fusion protein of the anti-TIGIT antibody and TGF-βR competing with human CD155-hFc-Biotin for binding to human TIGIT-mFC.

FIG. 8: Assays for binding activity of the fusion protein of the anti-TIGIT antibody and TGF-βR to TIGIT on the 293T-TIGIT membrane surface.

FIG. 9: Assays of TF01 competing with CD155 for binding to an antigen TIGIT on the cell membrane surface.

FIG. 10: Assays of TF01 competing with CD112 for binding to an antigen TIGIT on the cell membrane surface.

FIG. 11: Assays for affinity constants of TF01 to FcγRI.

FIG. 12: Assays for affinity constants of 26B12H2L2(hG1WT) to FcγRI. FIG. 13: Assays for affinity constants of TF01 to FcγRIIIa_V158.

FIG. 14: Assays for affinity constants of 26B12H2L2(hG1WT) to FcγRIIIa_V158.

FIG. 15: Assays for affinity constants of TF01 to FcγRIIIa_F158.

FIG. 16: Assays for affinity constants of 26B12H2L2(hG1WT) to FcγRIIIa_F158.

FIG. 17: Assays for affinity constants of TF01 to FcγRIIa_H131.

FIG. 18: Assays for affinity constants of 26B12H2L2(hG1WT) to FcγRIIa_H131.

FIG. 19: Assays for affinity constants of TF01 to FcγRIIa_R131.

FIG. 20: Assays for affinity constants of 26B12H2L2(hG1WT) to FcγRIIa_R131.

FIG. 21: Assays for affinity constants of TF01 to FcγRIIb.

FIG. 22: Assays for affinity constants of 26B12H2L2(hG1WT) to FcγRIIb.

FIG. 23: Assays for affinity constants of TF01 to C1q.

FIG. 24: Assays for affinity constants of 26B12H2L2(hG1WT) to C1q.

FIG. 25: Assays for ADCP effects of RG6058(hG1WT), RG6058(hG4WT), 26B12H2L2(hG1WT), and TF01.

FIG. 26: Evaluation of blocking activity of the fusion protein of the anti-TIGIT antibody and TGF-βR on CD112 inhibition against IL-2 secretion in the Jurkat-TIGIT and THP-1 cell co-culture system.

FIG. 27: Evaluation of blocking activity of the fusion protein of the anti-TIGIT antibody and TGF-βR on CD155 inhibition against IL-2 secretion in the Jurkat-TIGIT and THP-1 cell co-culture system.

FIG. 28: Evaluation of bioactivity of the fusion protein of the anti-TIGIT antibody and TGF-βR in promotion of IL-2 secretion in the Jurkat-TIGIT and HT1080-aCD3scFv cell co-culture system.

FIG. 29: Evaluation of blocking activity of the fusion protein of the anti-TIGIT antibody and TGF-βR on TGF-β1 inhibition against IFN-y secretion in process of PBMC secondary immune response to CMV antigen.

FIG. 30: Efficacy of the fusion protein of the anti-TIGIT antibody and TGF-βR for treating MDA-MB-231 xenograft tumor in a Scid Beige mouse model. Results were expressed as mean ± standard error and were subjected to two-way ANOVA (Bonferroni test), which were *p<0.05, **p<0.01, and ***p<0.001, compared to isotype control.

FIG. 31: Effect of the fusion protein of the anti-TIGIT antibody and TGF-βR on the body weight of a Scid Beige mouse model with MDA-MB-231 xenograft tumor.

Deposited biological material:

Hybridoma cell line LT019 (TIGIT-26B12) was deposited at China Center for Type Culture Collection (CCTCC) on Oct. 23, 2020, under CCTCC NO. C2020208, the depository address being Wuhan University, Wuhan, China, postal code: 430072.

The present invention relates to the following sequences 1 to 48:

1. The amino acid sequence of 26B12VH

**EVQLQESGPGLVKPSQSLSLTCTVTGHSFTSDYAWNWIRQFPGNRLEW**

**MGYISYSDSTNYNPSLKSRISITRDTSKNQFFLQMNSVTTEDTATYYCA**

**RLDYGNYGGAMDYWGQGTSVTVSS (SEQ ID NO: 1)**

2. The nucleotide sequence of 26B12VH

GAGGTGCAGCTGCAGGAGTCTGGACCTGGCCTGGTGAAACCCTCT
CAGTCTCTGTCCCTCACCTGCACTGTCACTGGCCACTCATTCACCA
GTGATTATGCCTGGAACTGGATCCGGCAGTTCCAGGAAACAGACT
GGAGTGGATGGGCTACATAGCTACAGTGATAGCACTAACTACAAC
CCATCTCTCAAAAGTCGAATCTATCACTCGAGACACATCCAAGA
ACCAGTTCTTCTTGCAGATGAATTCTGTGACTACTGAGGACACAGC
CACATATTACTGTGCAAGATTGGACTATGGTAACTACGGTGGGGCT
ATGGACTACTGGGGTCAAGGGACCTCAGTCACCGTCTCCTCA   (SEQ
ID NO: 2)

3. HCDR1:
GHSFTSDYA (SEQ ID NO: 3)

4. HCDR2:
ISYSDST (SEQ ID NO: 4)

5. HCDR3:
ARLDYGNYGGAMDY (SEQ ID NO: 5)

6. The amino acid sequence of 26B12VL

DIVLTQSHEFMSTSLRDRVSITCKSSQHVSTAVAWYQQKPGQSPKLLIY
SASYRYTGVPDRFTGSGSGTDFTFTISSVKAEDLAVYYCQQHYITPWTF
GGGTKLEIK (SEQ ID NO: 6)

7. The nucleotide sequence of 26B12VL

GATATTGTGCTAACTCAGTCTCACGAATTCATGTCCACCTCATTACG
AGACAGGGTCAGCATCACCTGCAAATCCAGTCAACATGTGAGTACT
GCTGTAGCCTGGTATCAACAGAAACCAGGACAATCTCCTAAACTAC
TGATTTACTCGGCATCCTACCGGTACACTGGAGTCCCTGATCGCTT
CACTGGCAGTGGATCTGGGACGGATTTCACTTTCACCATCAGCAGT
GTGAAGGCTGAAGACCTGGCAGTTTATTACTGTCAGCAACATTATA
TTACTCCGTGGACGTTCGGTGGAGGCACCAAGCTGGAAATAAAA
(SEQ ID NO: 7)

8. LCDR1:
QHVSTA (SEQ ID NO: 8)

9. LCDR2:
SAS (SEQ ID NO: 9)

10. LCDR3:
QQHYITPWT (SEQ ID NO: 10)

11. The amino acid sequence of 26B12H1VH

## DVQLQESGPGLVKPSQTLSLTCTVSGHSFTSDYAWNWIRQFPGKGLE WIGYISYSDSTNYNPSLKSRITISRDTSKNQFFLQLNSVTAADTATYYCA RLDYGNYGGAMDYWGQGTSVTVSS (SEQ ID NO: 11)

12. The nucleotide sequence of 26B12H1VH

## GATGTGCAGCTGCAGGAGAGCGGCCCCGGACTGGTGAAGCCTTCC CAGACCCTGTCTCTGACCTGTACAGTGTCTGGCCACAGCTTCACAT CCGACTACGCCTGGAACTGGATCAGGCAGTTTCCAGGCAAGGGCCT GGAGTGGATCGGCTACATCTCTTATAGCGACTCCACCAACTATAAT CCCTCTCTGAAGAGCCGGATCACCATCAGCAGAGATACATCCAAGA ACCAGTTCTTTCTGCAGCTGAACAGCGTGACAGCCGCCGACACCGC CACATACTATTGCGCCCGGCTGGACTACGGCAATTATGGCGGAGCC ATGGATTACTGGGGCCAGGGCACCTCCGTGACAGTGAGCTCC (SEQ ID NO: 12)

13. The amino acid sequence of 26B12H2VH

## DVQLQESGPGLVKPSQTLSLTCTVSGHSFTSDYAWSWIRQPPGKGLEW IGYISYSDSTNYNPSLKSRVTISRDTSKNQFSLKLSSVTAADTAVYYCAR LDYGNYGGAMDYWGQGTSVTVSS (SEQ ID NO: 13)

14. The nucleotide sequence of 26B12H2VH

GATGTGCAGCTGCAGGAGTCTGGCCCAGGACTGGTGAAGCCAAGC
CAGACCCTGTCCCTGACCTGTACAGTGTCCGGCCACTCTTTTACAA
GCGACTACGCCTGGTCTTGGATCAGGCAGCCCCCTGGCAAGGGAC
TGGAGTGGATCGGCTACATCCTATTCTGACAGCACCAACTATAA
TCCCTCCCTGAAGTCTCGGGTGACCATCTCTAGAGATACAAGCAAG
AACCAGTTCTCCCTGAAGCTGAGCTCCGTGACCGCAGCAGACACAG
CCGTGTACTATTGCGCCCGGCTGGACTACGGCAATTATGGCGGAGC
CATGGATTACTGGGGCCAGGGCACCAGCGTGACAGTGTCTAGC
(SEQ ID NO: 14)

15. The amino acid sequence of 26B12H3VH

DVQLQESGPGLVKPSQTLSLTCTVSGHSFTSDYAWSWIRQPPGKGLEW
IGYISYSDSTNYNPSLKSRVTISVDTSKNQFSLKLSSVTAADTAVYYCAR
LDYGNYGGAMDYWGQGTSVTVSS (SEQ ID NO: 15)

16. The nucleotide sequence of 26B12H3VH

GATGTGCAGCTGCAGGAGTCTGGCCCAGGACTGGTGAAGCCAAGC
CAGACCCTGTCCCTGACCTGTACAGTGTCCGGCCACTCTTTTACAA
GCGACTACGCCTGGTCTTGGATCAGACAGCCCCCTGGCAAGGGACT
GGAGTGGATCGGCTACATCTCCTATTCTGACAGCACCAACTATAAT
CCCTCCCTGAAGTCTAGAGTGACCATCTCTGTGGATACAAGCAAGA
ACCAGTTCTCCCTGAAGCTGAGCTCCGTGACCGCAGCAGACACAGC
CGTGTACTATTGCGCCCGGCTGGACTACGGCAATTATGGCGGAGCC
ATGGATTACTGGGGCCAGGGCACCAGCGTGACAGTGTCTAGC  (SEQ
ID NO: 16)

17. The amino acid sequence of 26B12H4VH

DVQLQESGPGLVKPSQTLSLTCTVSGHSFTSDYAWNWIRQFPGKGLE
WMGYISYSDSTNYNPSLKSRITISRDTSKNQFFLQLNSVTAADTATYYC
ARLDYGNYGGAMDYWGQGTSVTVSS (SEQ ID NO: 17)

18. The nucleotide sequence of 26B12H4VH

GATGTGCAGCTGCAGGAGAGCGGCCCCGGACTGGTGAAGCCTTCC
CAGACCCTGTCTCTGACCTGTACAGTGTCTGGCCACAGCTTCACAT
CCGACTACGCCTGGAACTGGATCAGGCAGTTTCCAGGCAAGGGCCT
GGAGTGGATGGGCTACATCTCTTATAGCGACTCCACCAACTATAAT
CCCTCTCTGAAGAGCCGGATCACCATCAGCAGAGATACATCCAAGA
ACCAGTTCTTTCTGCAGCTGAACAGCGTGACAGCCGCCGACACCGC
CACATACTATTGCGCCCGGCTGGACTACGGCAATTATGGCGGAGCC
ATGGATTACTGGGGCCAGGGCACCTCCGTGACAGTGAGCTCC (SEQ

ID NO: 18)

19. The amino acid sequence of 26B12L1VL

DIQMTQSPKSLSTSVGDRVTITCRSSQHVSTAVAWYQQKPGKSPKLLIY
SASYRYSGVPDRFSGSGSGTDFTFTISSVQPEDFATYYCQQHYITPWTF
GGGTKLEIK (SEQ ID NO: 19)

20. The nucleotide sequence of 26B12L1VL

GACATCCAGATGACCCAGTCCCCTAAGTCCCTGTCTACAAGCGTGG
GCGATCGGGTGACCATCACATGTAGAAGCTCCCAGCACGTGTCTAC
CGCAGTGGCATGGTACCAGCAGAAGCCAGGCAAGAGCCCTAAGCT
GCTGATCTATTCCGCCTCTTACAGGTATTCCGGAGTGCCAGACCGG
TTTAGCGGCTCCGGCTCTGGCACCGATTTCACCTTTACAATCTCTA
GCGTGCAGCCAGAGGACTTCGCCACATACTATTGCCAGCAGCACTA
CATCACCCCATGGACCTTCGGCGGCGGCACAAAGCTGGAGATCAA
G (SEQ ID NO: 20)

21. The amino acid sequence of 26B12L2VL

DIQMTQSPSSLSASVGDRVTITCRSSQHVSTALAWYQQKPGKSPKLLIY
SASSRYSGVPDRFSGSGSGTDFTFTISSLQPEDFATYYCQQHYITPWTFG
GGTKLEIK (SEQ ID NO: 21)

22. The nucleotide sequence of 26B12L2VL

GACATCCAGATGACCCAGTCCCCTAGCTCCCTGTCTGCCAGCGTGG
GCGATAGGGTGACCATCACATGTAGATCTAGCCAGCACGTGTCTAC
AGCCCTGGCATGGTACCAGCAGAAGCCAGGCAAGAGCCCTAAGCT
GCTGATCTACTCCGCCTCCTCTAGGTATTCTGGAGTGCCAGACCGG
TTTTCCGGCTCTGGCAGCGGCACCGATTTCACCTTTACAATCAGCT
CCCTGCAGCCAGAGGACTTCGCCACATACTATTGCCAGCAGCACTA
TATCACCCCATGGACCTTCGGCGGCGGCACCAAGCTGGAGATCAAG
(SEQ ID NO: 22)

23. The amino acid sequence of 26B12L3VL

DIQMTQSPSSLSASVGDRVTITCRASQHVSTALAWYQQKPGKAPKLLIY
SASSLQSGVPSRFSGSGSGTDFTLTISSLQPEDFATYYCQQHYITPWTFG
GGTKLEIK (SEQ ID NO: 23)

24. The nucleotide sequence of 26B12L3VL

GACATCCAGATGACCCAGTCCCCTAGCTCCCTGAGCGCCTCCGTGG
GCGATAGGGTGACCATCACATGTAGAGCCTCTCAGCACGTGAGCAC
AGCCCTGGCATGGTACCAGCAGAAGCCAGGCAAGGCCCCTAAGCT
GCTGATCTATAGCGCCTCTAGCCTGCAGTCCGGAGTGCCATCTCGG
TTCTCTGGCAGCGGCTCCGGAACCGACTTTACCCTGACAATCTCCT
CTCTGCAGCCAGAGGATTTCGCCACATACTATTGCCAGCAGCACTA
CATCACCCCATGGACCTTCGGCGGCGGCACCAAGCTGGAGATCAA
G (SEQ ID NO: 24)

25. The amino acid sequence of 26B12L4VL

DIQMTQSPKSMSTSVGDRVTITCRSSQHVSTAVAWYQQKPGKSPKLLI
YSASYRYSGVPDRFSGSGSGTDFTFTISSVQPEDFATYYCQQHYITPWTF
GGGTKLEIK (SEQ ID NO: 25)

26. The nucleotide sequence of 26B12L4VL

GACATCCAGATGACCCAGTCCCCTAAGTCCATGTCTACAAGCGTGGGCGACAGGGTGACCATCACATGTAGAAGCTCCCAGCACGTGTCTACCGCAGTGGCATGGTACCAGCAGAAGCCAGGCAAGAGCCCTAAGCTGCTGATCTATTCCGCCTCTTACAGGTATTCCGGAGTGCCAGACCGGTTTAGCGGCTCCGGCTCTGGCACCGATTTCACCTTTACAATCTCTA

GCGTGCAGCCAGAGGACTTCGCCACATACTATTGCCAGCAGCACTACATCACCCCATGGACCTTCGGCGGCGGCACAAGCTGGAGATCAAG (SEQ ID NO: 26)

27. The amino acid sequence of heavy chain of 26B12H2L2(hG4DM)

DVQLQESGPGLVKPSQTLSLTCTVSGHSFTSDYAWSWIRQPPGKGLEWIGYISYSDSTNYNPSLKSRVTISRDTSKNQFSLKLSSVTAADTAVYYCARLDYGNYGGAMDYWGQGTSVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEAAGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLG (SEQ ID NO: 27)

28. The nucleotide sequence of heavy chain of 26B12H2L2(hG4DM)

GATGTGCAGCTGCAGGAGTCTGGCCCAGGACTGGTGAAGCCAAGC
CAGACCCTGTCCCTGACCTGTACAGTGTCCGGCCACTCTTTTACAA
GCGACTACGCCTGGTCTTGGATCAGGCAGCCCCCTGGCAAGGGAC
TGGAGTGGATCGGCTACATCTCCTATTCTGACAGCACCAACTATAA
TCCCTCCCTGAAGTCTCGGGTGACCATCTCTAGAGATACAAGCAAG
AACCAGTTCTCCCTGAAGCTGAGCTCCGTGACCGCAGCAGACACAG
CCGTGTACTATTGCGCCCGGCTGGACTACGGCAATTATGGCGGAGC
CATGGATTACTGGGGCCAGGGCACCAGCGTGACAGTGTCTAGCGC
CTCCACAAAGGGGCCCTCGGTCTTCCCCCTGGCGCCCTGCTCCAGG
AGCACCTCCGAGAGCACAGCCGCCCTGGGCTGCCTGGTCAAGGAC
TACTTCCCCGAACCGGTGACGGTGTCGTGGAACTCAGGCGCCCTGA
CCAGCGGCGTACACACCTTCCCGGCTGTCCTACAGTCCTCAGGACT

CTACTCCCTCAGCAGCGTGGTGACCGTGCCCTCCAGCAGCTTGGGC
ACGAAGACCTACACCTGCAACGTAGATCACAAGCCCAGCAACACCA
AGGTGGACAAGAGAGTTGAGTCCAAATATGGTCCCCCATGCCCACC
ATGCCCAGCACCTGAGGCCGCTGGGGGACCATCAGTCTTCCTGTTC
CCCCCAAAACCCAAGGACACTCTCATGATCTCCGGACCCCTGAGG
TCACGTGCGTGGTGGTGGACGTGAGCCAGGAAGACCCCGAGGTCC
AGTTCAACTGGTACGTGGATGGCGTGGAGGTGCATAATGCCAAGAC
AAAGCCGCGGGAGGAGCAGTTCAACAGCACGTACCGTGTGGTCAG
CGTCCTCACCGTCCTGCACCAGGACTGGCTGAACGGCAAGGAGTAC
AAGTGCAAGGTCTCCAACAAAGGCCTCCCGTCCTCCATCGAGAAAA
CCATCTCCAAAGCCAAAGGGCAGCCCCGAGAGCCACAGGTGTACA
CCCTGCCCCCATCCCAGGAGGAGATGACCAAGAACCAGGTCAGCCT
GACCTGCCTGGTCAAAGGCTTCTACCCCAGCGACATCGCCGTGGAG
TGGGAGAGCAATGGGCAGCCGGAGAACAACTACAAGACCACGCCT
CCCGTGCTGGACTCCGACGGCTCCTTCTTCCTCTACAGCAGGCTAA
CCGTGGACAAGAGCAGGTGGCAGGAGGGGAATGTCTTCTCATGCT
CCGTGATGCATGAGGCTCTGCACAACCACTACACACAGAAGAGCCT
CTCCCTGTCTCTGGGT (SEQ ID NO: 28)

29. The amino acid sequence of light chain of 26B12H2L2(hG4DM)

DIQMTQSPSSLSASVGDRVTITCRSSQHVSTALAWYQQKPGKSPKLLIY
SASSRYSGVPDRFSGSGSGTDFTFTISSLQPEDFATYYCQQHYITPWTFG
GGTKLEIKRTVAAPSVFIFPPSDEQLKSGTASVVCLLNNFYPREAKVQW
KVDNALQSGNSQESVTEQDSKDSTYSLSSTLTLSKADYEKHKVYACEV
THQGLSSPVTKSFNRGEC (SEQ ID NO: 29)

30. The nucleotide sequence of light chain of 26B12H2L2(hG4DM)

GACATCCAGATGACCCAGTCCCCTAGCTCCCTGTCTGCCAGCGTGG
GCGATAGGGTGACCATCACATGTAGATCTAGCCAGCACGTGTCTAC

AGCCCTGGCATGGTACCAGCAGAAGCCAGGCAAGAGCCCTAAGCT
GCTGATCTACTCCGCCTCCTCTAGGTATTCTGGAGTGCCAGACCGG
TTTTCCGGCTCTGGCAGCGGCACCGATTTCACCTTTACAATCAGCT
CCCTGCAGCCAGAGGACTTCGCCACATACTATTGCCAGCAGCACTA
TATCACCCCATGGACCTTCGGCGGCGGCACCAAGCTGGAGATCAAG
CGTACGGTGGCAGCCCCATCTGTCTTCATTTTTCCCCCTAGTGACG
AGCAGCTGAAATCCGGAACAGCCTCTGTGGTCTGTCTGCTGAACAA
TTTCTACCCTCGCGAAGCCAAGGTGCAGTGGAAAGTCGATAACGCT
CTGCAGAGTGGCAATTCACAGGAGAGCGTGACTGAACAGGACTCC
AAGGATTCTACCTATAGTCTGAGCTCCACTCTGACCCTGTCCAAAG
CAGATTACGAAAAGCACAAAGTGTATGCCTGTGAGGTCACCCACCA
GGGGCTGAGTTCTCCAGTCACCAAATCCTTCAACAGAGGCGAATGT
(SEQ ID NO: 30)

31. The amino acid sequence of heavy chain of 26B12H2L2(hG1DM)

DVQLQESGPGLVKPSQTLSLTCTVSGHSFTSDYAWSWIRQPPGKGLEW
IGYISYSDSTNYNPSLKSRVTISRDTSKNQFSLKLSSVTAADTAVYYCAR
LDYGNYGGAMDYWGQGTSVTVSSASTKGPSVFPLAPSSKSTSGGTAAL
GCLVKDYFPEPVTVSWNSGALTSGVHTFPAVLQSSGLYSLSSVVTVPSS
SLGTQTYICNVNHKPSNTKVDKKVEPKSCDKTHTCPPCPAPEAAGGPS
VFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEK
TISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHE
ALHNHYTQKSLSLSPGK (SEQ ID NO: 31)

32. The nucleotide sequence of heavy chain of 26B12H2L2(hG1DM)

GATGTGCAGCTGCAGGAGTCTGGCCCAGGACTGGTGAAGCCAAGC
CAGACCCTGTCCCTGACCTGTACAGTGTCCGGCCACTCTTTTACAA

GCGACTACGCCTGGTCTTGGATCAGGCAGCCCCCTGGCAAGGGAC
TGGAGTGGATCGGCTACATCTCCTATTCTGACAGCACCAACTATAA
TCCCTCCCTGAAGTCTCGGGTGACCATCTCTAGAGATACAAGCAAG
AACCAGTTCTCCCTGAAGCTGAGCTCCGTGACCGCAGCAGACACAG
CCGTGTACTATTGCGCCGGCTGGACTACGGCAATTATGGCGGAGC
CATGGATTACTGGGGCCAGGGCACCAGCGTGACAGTGTCTAGCGC
CTCTACCAAGGGGCCCAGCGTGTTTCCTCTCGCCCCTCCTCCAAA
AGCACCAGCGGAGGAACCGCTGCTCTCGGATGTCTGGTGAAGGAC
TACTTCCCTGAACCCGTCACCGTGAGCTGGAATAGCGGCGCTCTGA
CAAGCGGAGTCCATACATTCCTGCTGTGCTGCAAAGCAGCGGACT
CTATTCCCTGTCCAGCGTCGTCACAGTGCCCAGCAGCAGCCTGGGC
ACCCAGACCTACATCTGTAACGTCAACCACAAGCCCTCCAACACCA
AGGTGGACAAGAAAGTGGAGCCCAAATCCTGCGACAAGACACACA
CCTGTCCCCCCTGTCCTGCTCCCGAAGCTGCTGGAGGCCCTAGCGT
CTTCCTCTTTCCTCCCAAACCCAAGGACACCCTCATGATCAGCAGA
ACCCCTGAAGTCACCTGTGTCGTCGTGGATGTCAGCCATGAGGACC
CCGAGGTGAAATTCAACTGGTATGTCGATGGCGTCGAGGTGCACAA
CGCCAAAACCAAGCCCAGGGAGGAACAGTACAACTCCACCTACAG
GGTGGTGTCCGTGCTGACAGTCCTCCACCAGGACTGGCTGAACGG
CAAGGAGTACAAGTGCAAGGTGTCCAACAAGGCTCTCCCTGCCCCC
ATTGAGAAGACCATCAGCAAGGCCAAGGCCAACCCAGGGAGCCC
CAGGTCTATACTGCCTCCCTCCAGGGACGAACTCACCAAGAACC
AGGTGTCCCTGACCTGCCTGGTCAAGGGCTTTTATCCCAGCGACAT
CGCCGTCGAGTGGGAGTCCAACGGACAGCCCGAGAATAACTACAA
GACCACCCCTCCTGTCCTCGACTCCGACGGCTCCTTCTTCCTGTAC
AGCAAGCTGACCGTGGACAAAAGCAGGTGGCAGCAGGGAAACGTG
TTCTCCTGCAGCGTGATGCACGAAGCCCTCCACAACCACTACACCC
AGAAAAGCCTGTCCCTGAGCCCCGGCAAA (SEQ ID NO: 32)

33. The amino acid sequence of a truncated fragment of TGF-βRII extracellular region

IPPHVQKSVNNDMIVTDNNGAVKFPQLCKFCDVRFSTCDNQKSCMSNC SITSICEKPQEVCVAVWRKNDENITLETVCHDPKLPYHDFILEDAASPK CIMKEKKKPGETFFMCSCSSDECNDNIIFSEEYNTSNPD (SEQ ID NO: 33)

34. The amino acid sequence of TGF-βRI: (the underlined part is the sequence of an extracellular region)

MEAAVAAPRPRLLLLVLAAAAAAAAALLPGATA<u>LQCFCHLCTKDNFT CVTDGLCFVSVTETTDKVIHNSMCIAEIDLIPRDRPFVCAPSSKTGSVTT TYCCNQDHCNKIELPTTVKSSPGLGPVEL</u>AAVIAGPVCFVCISLMLMV YICHNRTVIHHRVPNEEDPSLDRPFISEGTTLKDLIYDMTTSGSGSGLPL LVQRTIARTIVLQESIGKGRFGEVWRGKWRGEEVAVKIFSSREERSWF REAEIYQTVMLRHENILGFIAADNKDNGTWTQLWLVSDYHEHGSLFD YLNRYTVTVEGMIKLALSTASGLAHLHMEIVGTQGKPAIAHRDLKSKN ILVKKNGTCCIADLGLAVRHDSATDTIDIAPNHRVGTKRYMAPEVLDD SINMKHFESFKRADIYAMGLVFWEIARRCSIGGIHEDYQLPYYDLVPSD PSVEEMRKVVCEQKLRPNIPNRWQSCEALRVMAKIMRECWYANGAA RLTALRIKKTLSQLSQQEGIKM (SEQ ID NO: 34)

35. The amino acid sequence of TGF-βRI extracellular region

LQCFCHLCTKDNFTCVTDGLCFVSVTETTDKVIHNSMCIAEIDLIPRDR PFVCAPSSKTGSVTTTYCCNQDHCNKIELPTTVKSSPGLGPVEL (SEQ ID NO: 35)

36. The amino acid sequence of TGF-βRII: (the underlined part is the sequence of an extracellular region)

MGRGLLRGLWPLHIVLWTRIAS<u>TIPPHVQKSVNNDMIVTDNNGAVKFP</u>

QLCKFCDVRFSTCDNQKSCMSNCSITSICEKPQEVCVAVWRKNDENIT LETVCHDPKLPYHDFILEDAASPKCIMKEKKKPGETFFMCSCSSDECN DNIIFSEEYNTSNPDLLLVIFQVTGISLLPPLGVAISVIIIFYCYRVNRQQK LSSTWETGKTRKLMEFSEHCAIILEDDRSDISSTCANNINHNTELLPIEL DTLVGKGRFAEVYKAKLKQNTSEQFETVAVKIFPYEEYASWKTEKDIF SDINLKHENILQFLTAEERKTELGKQYWLITAFHAKGNLQEYLTRHVIS WEDLRKLGSSLARGIAHLHSDHTPCGRPKMPIVHRDLKSSNILVKNDL TCCLCDFGLSLRLDPTLSVDDLANSGQVGTARYMAPEVLESRMNLENV ESFKQTDVYSMALVLWEMTSRCNAVGEVKDYEPPFGSKVREHPCVES MKDNVLRDRGRPEIPSFWLNHQGIQMVCETLTECWDHDPEARLTAQC VAERFSELEHLDRLSGRSCSEEKIPEDGSLNTTK (SEQ ID NO: 36)

37. The amino acid sequence of TGF-βRII extracellular region

TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQ (SEQ ID NO: 37)

38. The amino acid sequence of TGF-βRIII: (the underlined part is the sequence of an extracellular region)

MTSHYVIAIFALMSSCLATAGPEPGALCELSPVSASHPVQALMESFTVL SGCASRGTTGLPQEVHVLNLRTAGQGPGQLQREVTLHLNPISSVHIHH KSVVFLLNSPHPLVWHLKTERLATGVSRLFLVSEGSVVQFSSANFSLTA ETEERNFPHGNEHLLNWARKEYGAVTSFTELKIARNIYIKVGEDQVFP PKCNIGKNFLSLNYLAEYLQPKAAEGCVMSSQPQNEEVHIIELITPNSNP YSAFQVDITIDIRPSQEDLEVVKNLILILKCKKSVNWVIKSFDVKGSLKII APNSIGFGKESERSMTMTKSIRDDIPSTQGNLVKWALDNGYSPITSYTM APVANRFHLRLENNAEEMGDEEVHTIPPELRILLDPGALPALQNPPIRG GEGQNGGLPFPFPDISRRVWNEEGEDGLPRPKDPVIPSIQLFPGLREPE

EVQGSVDIALSVKCDNEKMIVAVEKDSFQASGYSGMDVTLLDPTCKAK
MNGTHFVLESPLNGCGTRPRWSALDGVVYYNSIVIQVPALGDSSGWPD
GYEDLESGDNGFPGDMDEGDASLFTRPEIVVFNCSLQQVRNPSSFQEQP
HGNITFNMELYNTDLFLVPSQGVFSVPENGHVYVEVSVTKAEQELGFA
IQTCFISPYSNPDRMSHYTIIENICPKDESVKFYSPKRVHFPIPQADMDK
KRFSFVFKPVFNTSLLFLQCELTLCTKMEKHPQKLPKCVPPDEACTSL
DASIIWAMMQNKKTFTKPLAVIHHEAESKEKGPSMKEPNPISPPIFHGL
DTLTVMGIAFAAFVIGALLTGALWYIYSHTGETAGRQQVPTSPPASENS
SAAHSIGSTQSTPCSSSSTA (SEQ ID NO: 38)

39. The amino acid sequence of TGF-βRIII extracellular region

GPEPGALCELSPVSASHPVQALMESFTVLSGCASRGTTGLPQEVHVLN
LRTAGQGPGQLQREVTLHLNPISSVHIHHKSVVFLLNSPHPLVWHLKT
ERLATGVSRLFLVSEGSVVQFSSANFSLTAETEERNFPHGNEHLLNWA
RKEYGAVTSFTELKIARNIYIKVGEDQVFPPKCNIGKNFLSLNYLAEYL
QPKAAEGCVMSSQPQNEEVHIIELITPNSNPYSAFQVDITIDIRPSQEDLE
VVKNLILILKCKKSVNWVIKSFDVKGSLKIIAPNSIGFGKESERSMTMT
KSIRDDIPSTQGNLVKWALDNGYSPITSYTMAPVANRFHLRLENNAEE
MGDEEVHTIPPELRILLDPGALPALQNPPIRGGEGQNGGLPFPFPDISRR
VWNEEGEDGLPRPKDPVIPSIQLFPGLREPEEVQGSVDIALSVKCDNEK
MIVAVEKDSFQASGYSGMDVTLLDPTCKAKMNGTHFVLESPLNGCGT
RPRWSALDGVVYYNSIVIQVPALGDSSGWPDGYEDLESGDNGFPGDMD
EGDASLFTRPEIVVFNCSLQQVRNPSSFQEQPHGNITFNMELYNTDLFL
VPSQGVFSVPENGHVYVEVSVTKAEQELGFAIQTCFISPYSNPDRMSHY
TIIENICPKDESVKFYSPKRVHFPIPQADMDKKRFSFVFKPVFNTSLLFL
QCELTLCTKMEKHPQKLPKCVPPDEACTSLDASIIWAMMQNKKTFTK
PLAVIHHEAESKEKGPSMKEPNPISPPIFHGLDTLTV (SEQ ID NO: 39)

40. The amino acid sequence of heavy chain of tiragolumab

EVQLQQSGPGLVKPSQTLSLTCAISGDSVSSNSAAWNWIRQSPSRGLE
WLGKTYYRFKWYSDYAVSVKGRITINPDTSKNQFSLQLNSVTPEDTAV
FYCTRESTTYDLLAGPFDYWGQGTLVTVSSASTKGPSVFPLAPSSKSTS
GGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFS
CSVMHEALHNHYTQKSLSLSPGK (SEQ ID NO: 40)

41. The amino acid sequence of light chain of tiragolumab

DIVMTQSPDSLAVSLGERATINCKSSQTVLYSSNNKKYLAWYQQKPGQ
PPNLLIYWASTRESGVPDRFSGSGSGTDFTLTISSLQAEDVAVYYCQQY
YSTPFTFGPGTKVEIKRTVAAPSVFIFPPSDEQLKSGTASVVCLLNNFYP
REAKVQWKVDNALQSGNSQESVTEQDSKDSTYSLSSTLTLSKADYEKH
KVYACEVTHQGLSSPVTKSFNRGEC (SEQ ID NO: 41)

42. The amino acid sequence of heavy chain variable region of RG6058(hG4)

EVQLQQSGPGLVKPSQTLSLTCAISGDSVSSNSAAWNWIRQSPSRGLE
WLGKTYYRFKWYSDYAVSVKGRITINPDTSKNQFSLQLNSVTPEDTAV
FYCTRESTTYDLLAGPFDYWGQGTLVTVSS (SEQ ID NO: 42)

43. The amino acid sequence of light chain variable region of RG6058(hG4)

DIVMTQSPDSLAVSLGERATINCKSSQTVLYSSNNKKYLAWYQQKPGQ
PPNLLIYWASTRESGVPDRFSGSGSGTDFTLTISSLQAEDVAVYYCQQY
YSTPFTFGPGTKVEIK (SEQ ID NO: 43)

44. The amino acid sequence of a linker fragment GGGGSGGGGSGGGGSGGGGSG (SEQ ID NO: 44)

45. The amino acid sequence of heavy chain of anti-HEL&TGFβ antibody

EVQLEQSGAELMKPGASVKISCKATGYTFTTYWIEWIKQRPGHSLEWI
GEILPGSDSTYYNEKVKGKVTFTADASSNTAYMQLSSLTSEDSAVYYCA
RGDGFYVYWGQGTTLTVSSASTKGPSVFPLAPCSRSTSESTAALGCLV
KDYFPEPVTVSWNSGALTSGVHTFPAVLQSSGLYSLSSVVTVPSSSLGT
KTYTCNVDHKPSNTKVDKRVESKYGPPCPPCPAPEAAGGPSVFLFPPK
PKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKG
QPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYT
QKSLSLSLGGGGGSGGGGSGGGGSGGGGSGIPPHVQKSVNNDMIVTD
NNGAVKFPQLCKFCDVRFSTCDNQKSCMSNCSITSICEKPQEVCVAVW
RKNDENITLETVCHDPKLPYHDFILEDAASPKCIMKEKKKPGETFFMC
SCSSDECNDNIIFSEEYNTSNPD (SEQ ID NO: 45)

46. The amino acid sequence of light chain of anti-HEL& TGFβ antibody

DIELTQSPATLSVTPGDSVSLSCRASQSISNNLHWYQQKSHESPRLLIKY
TSQSMSGIPSRFSGSGSGTDFTLSINSVETEDFGVYFCQQSGSWPRTFGG
GTKLDIKRTVAAPSVFIFPPSDEQLKSGTASVVCLLNNFYPREAKVQWK
VDNALQSGNSQESVTEQDSKDSTYSLSSTLTLSKADYEKHKVYACEVT
HQGLSSPVTKSFNRGEC (SEQ ID NO: 46)

47. The amino acid sequence of heavy chain of RG6058(G1DM)

EVQLQQSGPGLVKPSQTLSLTCAISGDSVSSNSAAWNWIRQSPSRGLE
WLGKTYYRFKWYSDYAVSVKGRITINPDTSKNQFSLQLNSVTPEDTAV
FYCTRESTTYDLLAGPFDYWGQGTLVTVSSASTKGPSVFPLAPSSKSTS
GGTAALGCLVKDYFPEPVTVSWNSGALTSGVHTFPAVLQSSGLYSLSS

VVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKA LPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSC SVMHEALHNHYTQKSLSLSPGK (SEQ ID NO: 47)

48. The amino acid sequence of light chain of RG6058(G1DM)

DIVMTQSPDSLAVSLGERATINCKSSQTVLYSSNNKKYLAWYQQKPGQ PPNLLIYWASTRESGVPDRFSGSGSGTDFTLTISSLQAEDVAVYYCQQY YSTPFTFGPGTKVEIKRTVAAPSVFIFPPSDEQLKSGTASVVCLLNNFYP REAKVQWKVDNALQSGNSQESVTEQDSKDSTYSLSSTLTLSKADYEKH KVYACEVTHQGLSSPVTKSFNRGEC (SEQ ID NO: 48)

DETAILED DESCRIPTION

Embodiments of the present invention will be described in detail below with reference to examples, but those skilled in the art will appreciate that the following examples are only for illustrating the present invention, and should not be construed as limitations to the scope of the present invention. Experimental procedures without specified conditions in the examples are conducted according to conventional conditions or conditions recommended by the manufacturer. Reagents or instruments used are all commercially available conventional products if the manufacturers thereof are not specified.

BALB/c mice were purchased from Guangdong Medical Laboratory Animal Center.

C57 mice were purchased from Laboratory Animal Center of Guangzhou University of Chinese Medicine.

MDA-MB-231 cells and U87-MG cells were purchased from ATCC.

The positive control antibody tiragolumab comprises amino acid sequences of the heavy chain and the light chain which are derived from the antibody described in WHO. Proposed INN: List 117. WHO Drug Information, 31(2): p104, 2017 and which are identical to those of SEQ ID NOs: 40-41, respectively.

The tiragolumab and RG6058(hG1WT) are the same antibody in the present invention.

The positive control antibody RG6058(hG4) comprises sequences which are derived from the antibody described in Publication Patent No. CN108290946A and comprises amino acid sequences of the heavy chain variable region and the light chain variable region which are identical to those of SEQ ID NOs: 42-43, respectively.

The positive control antibody RG6058(hG1DM) was prepared at the laboratory of Akeso Biopharma Inc., with Lot No. 20180816; the amino acid sequence of the heavy chain thereof is set forth in SEQ ID NO: 47, and the amino acid sequence of the light chain thereof is set forth in SEQ ID NO: 48. The variable region sequence of the isotype control antibody in the examples of the present invention, human anti-hen egg lysozyme IgG (anti-HEL, or human IgG, abbreviated as hIgG), is derived from Acierno et al., "Affinity maturation increases the stability and plasticity of the Fv domain of anti-protein antibodies" (Acierno et al., J Mol Biol., 2007; 374(1):130-46). The hIgG1DM and hIgG4WT used in the examples are isotype control antibodies with anti-HEL having an hGIDM and hG4WT constant region sequence, respectively, prepared at the laboratory of Akeso Biopharma Inc.

The anti-HEL&TGFβ antibody was constructed by fusing TGFβRII protein to the C-terminus of a heavy chain of an anti-HEL antibody (human hG4DM) of k subtype, and was prepared at the laboratory of Akeso Biopharma Inc.; the amino acid sequence of the heavy chain thereof is set forth in SEQ ID NO: 45, and the amino acid sequence of the light chain thereof is set forth in SEQ ID NO: 46.

TIGIT-mFc protein (Lot No. 20171110), TGF-βRII-mFc (Lot No. 20200528), CD155-hFc-Biotin (Lot No. 20170721), CD155-mFc (Lot No. 20190726), and CD112-mFc (Lot No. 20190726) were all produced by Akeso Biopharma Inc. according to known sequences, wherein mFc represents an Fc protein fragment of mouse IgG; hFc represents an Fc protein fragment of human IgG. TGF-β1 protein was purchased from Sino Biologcal Inc., with Cat. No. LC13DE3108.

TGF-β2 protein was purchased from peprotech, with Cat. No. 0420345 E0620. TGF-β3 protein was purchased from peprotech, with Cat. No. 0713410 A0219. In the following examples of the present invention, the cell line 293T-TIGIT used was constructed by Akeso Biopharma Inc. The cell line 293T-TIGIT was prepared by viral infection of HEK293T cells (Wuhan University) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was plenti6.3/V5-TIGITFL-BSD (TIGIT was Genebank ID: NP_776160.2; the vector plenti6.3/V5 TOPO was purchased from Invitrogen, with Cat. No. K531520).

In the following examples of the present invention, the cell line CHO-K1-TIGIT used was constructed by Akeso Biopharma Inc. The cell line CHO-K1-TIGIT was prepared by viral infection of CHO-K1 cells (Cell Resource Center, Institute of Basic Medical Sciences, Chinese Academy of Medical Sciences) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was plenti6.3/V5-TIGITFL-BSD (TIGIT was Genebank ID: NP_776160.2; the vector plenti6.3/V5 TOPO was purchased from Invitrogen, with Cat. No. K531520).

In the following examples of the present invention, the cell line Jurkat-TIGIT used was constructed by Akeso Biopharma Inc. The cell line Jurkat-TIGIT was prepared by viral infection of Jurkat cells (Cell Center, Chinese Academy of Sciences) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was plenti6.3/V5-TIGITFL-BSD (TIGIT was Genebank ID: NP_776160.2; the vector plenti6.3/V5 TOPO was purchased from Invitrogen, with Cat. No. K531520).

In the following examples of the present invention, the cell line HT1080-aCD3scFv used was constructed by Akeso Biopharma Inc. The cell line HT1080-aCD3scFv was prepared by viral infection of HT-1080 cells (Cell Resource Center, Shanghai Institutes for Biological Sciences, Chinese Academy of Sciences) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was pCDH-aCD3scFv-Puro (the sequences of anti-CD3scFv were derived from the anti-human CD3 Mus musculus OKT3 hybridoma antibody; the vector pCDH-CMV-MCS-EF1-Puro was purchased from Youbio, with Cat. No. VT1480).

Example 1: Preparation of Anti-TIGIT Antibody

1. Preparation of hybridoma cell line LT019

The antigen used for preparing the anti-TIGIT antibody was human TIGIT-mFc (TIGIT was Genbank ID: NP_776160.2). Spleen cells of immunized mice were fused with myeloma cells of the mice to prepare hybridoma cells. With human TIGIT-hFc taken as antigens, the hybridoma cells were screened by indirect ELISA to obtain hybridoma cells capable of secreting antibodies specifically binding to TIGIT. The hybridoma cells obtained by screening were subjected to limiting dilution to obtain a stable hybridoma cell line. The above hybridoma cell line was designated hybridoma cell line LT019, and the monoclonal antibody secreted by the cell line was designated 26B12.

Hybridoma cell line LT019 (also known as TIGIT-26B12) was deposited at China Center for Type Culture Collection (CCTCC) on Oct. 23, 2020, under CCTCC NO. C2020208, the depository address being Wuhan University, Wuhan, China, postal code: 430072.

2. Preparation of anti-TIGIT antibody 26B12

The cell line LT019 prepared above was cultured with a chemical defined medium (CD medium, containing 1% penicillin-streptomycin) at 37 °C/5% COz. After 7 days, the cell culture supernatant was collected, subjected to high-speed centrifugation and vacuum filtration through a microfiltration membrane, and purified by using a HiTrap protein A HP column to obtain antibody 26B12.

Example 2: Sequence Analysis of Anti-TIGIT Antibody 26B12

mRNA was extracted from the cell line LT019 cultured in Example 1 according to the method described in the manual of the cell/bacterial total RNA extraction kit (Tiangen, Cat. No. DP430).

cDNA was synthesized according to the manual of Invitrogen SuperScript® III First-Strand Synthesis System for RT-PCR and amplified by PCR.

The PCR amplification products were directly subjected to TA cloning according to the manual of the pEASY-T1 Cloning Kit (Transgen CT101).

The TA cloning products were directly sequenced, and the sequencing results are as follows:

The nucleotide sequence of the heavy chain variable region is set forth in SEQ ID NO: 2 with a length of 363 bp; the encoded amino acid sequence is set forth in SEQ ID NO: 1 with a length of 121 amino acids, wherein the sequences of heavy chains HCDR1, HCDR2, and HCDR3 are set forth in SEQ ID NOs: 3, 4, and 5, respectively.

The nucleotide sequence of the light chain variable region is set forth in SEQ ID NO: 7 with a length of 321 bp; the encoded amino acid sequence is set forth in SEQ ID NO: 6 with a length of 107 amino acids, wherein the sequences of light chains LCDR1, LCDR2, and LCDR3 are set forth in SEQ ID NOs: 8, 9, and 10, respectively.

Example 3: Design and Preparation of Anti-Human TIGIT Humanized Antibodies and Mutant Antibodies

1. Design of light and heavy chains of anti-human TIGIT humanized antibodies 26B12H1L1, 26B12H4L1, 26B12H2L2, 26B12H3L2, 26B12H2L3, 26B12H3L3, 26B12H1L4, and 26B12H4L4

Based on the three-dimensional crystal structure of human TIGIT protein and the sequence of antibody 26B12 obtained in Example 2, the variable region sequences of antibodies 26B12H1L1, 26B12H4L1, 26B12H2L2, 26B12H3L2, 26B12H2L3, 26B12H3L3, 26B12H1L4, and 26B12H4L4 (antibody constant region sequences from the NCBI database: the heavy chain constant region is Ig gamma-1 chain C region, ACCESSION: P01857; the light chain constant region is Ig kappa chain C region, ACCESSION: P01834) were designed.

The designed variable region sequences are shown in Table 1 below.

Table 1: Variable region sequences of anti-human TIGIT humanized antibodies

| Name | Amino acid sequence of heavy chain variable region SEQ ID NO: | Nucleotide sequence of heavy chain variable region SEQ ID NO: | Amino acid sequence of light chain variable region SEQ ID NO: | Nucleotide sequence of light chain variable region SEQ ID NO: |
|---|---|---|---|---|
| 26B12H1L1 | 11 | 12 | 19 | 20 |
| 26B12H4L1 | 17 | 18 | 19 | 20 |
| 26B12H2L2 | 13 | 14 | 21 | 22 |
| 26B12H2L3 | 13 | 14 | 23 | 24 |
| 26B12H3L2 | 15 | 16 | 21 | 22 |
| 26B12H3L3 | 15 | 16 | 23 | 24 |
| 26B12H1L4 | 11 | 12 | 25 | 26 |
| 26B12H4L4 | 17 | 18 | 25 | 26 |

For the above 8 antibodies 26B12H1L1, 26B12H4L1, 26B12H2L2, 26B12H3L2, 26B12H2L3, 26B12H3L3, 26B12H1L4, and 26B12H4L4, the length of the nucleotide sequences of the heavy chain variable regions was 363 bp, and the length of the encoded amino acid sequences was 121 aa; the length of the nucleotide sequences of the light chain variable regions was 321 bp, and the length of the encoded amino acid sequences was 107 aa.

Moreover, the above 8 antibodies had the same HCDR1-HCDR3 and LCDR1-LCDR3:

the sequences of the HCDR1, HCDR2, and HCDR3 are set forth in SEQ ID NOs: 3, 4, and 5, respectively; and

the sequences of the LCDR1, LCDR2, and LCDR3 are set forth in SEQ ID NOs: 8, 9, and 10, respectively.

2. Preparation of humanized antibodies 26B12H1L1, 26B12H4L1, 26B12H2L2, 26B12H3L2, 26B12H2L3, 26B12H3L3, 26B12H1L4, and 26B12H4L4

The heavy chain constant regions were all Ig gamma-1 chain C region, ACCESSION: P01857; the light chain constant regions were all Ig kappa chain C region, ACCESSION: P01834.

Heavy chain cDNA and light chain cDNA of 26B12H1L1, heavy chain cDNA and light chain cDNA of 26B12H4L1, heavy chain cDNA and light chain cDNA of 26B12H2L2, heavy chain cDNA and light chain cDNA of 26B12H3L2, heavy chain cDNA and light chain cDNA of 26B12H2L3, heavy chain cDNA and light chain cDNA of 26B12H3L3, heavy chain cDNA and light chain cDNA of 26B12H1L4, heavy chain cDNA and light chain cDNA of 26B12H2L4, and heavy chain cDNA and light chain cDNA of 26B12H4L4 were separately cloned into pUC57simple (provided by GenScript) vectors to obtain:

pUC57simple-26B12H1, pUC57simple-26B12L1;
pUC57simple-26B12H4, pUC57simple-26B12L1;
pUC57simple-26B12H2, pUC57simple-26B12L2;
pUC57simple-26B12H3, pUC57simple-26B12L2;
pUC57simple-26B12H2, pUC57simple-26B12L3;
pUC57simple-26B12H3, pUC57simple-26B12L3;
pUC57simple-26B12H1, pUC57simple-26B12L4; and
pUC57simple-26B12H4, pUC57simple-26B12L4.

With reference to the standard techniques described in Molecular Cloning: A Laboratory Manual (Second Edition), the heavy and light chain full-length genes synthesized by EcoRI&HindIII digestion were subcloned into expression vector pcDNA3.1 by digestion with restriction enzyme EcoRI&HindIII to obtain expression plasmids pcDNA3.1-26B12H1, pcDNA3.1-26B12L1, pcDNA3.1-26B12H4, pcDNA3.1-126B12H2, pcDNA3.1-26B12L2, pcDNA3.1-26B12H3, pcDNA3.1-26B12L3, and pcDNA3.1-26B12L4, and the heavy/light chain genes of the recombinant expression plasmids were further sequenced. Then the designed gene combinations comprising corresponding light and heavy chain recombinant plasmids (pcDNA3.1-26B12H1/pcDNA3.1-26B12L1, pcDNA3.1-26B12H4/pcDNA3.1-26B12L1, pcDNA3.1-26B12H2/pcDNA3.1-26B12L2, pcDNA3.1-26B12H3/pcDNA3.1-26B12L2, pcDNA3.1-26B12H2/pcDNA3.1-26B12L3, pcDNA3.1-26B12H3/pcDNA3.1-26B12L3, pcDNA3.1-26B12H1/pcDNA3.1-26B12L4, and pcDNA3.1-26B12H4/pcDNA3.1-26B12L4) were separately co-transfected into 293F cells, and the cultures were collected and purified. After the sequences were verified, endotoxin-free expression plasmids were prepared, and were transiently transfected into HEK293 cells for antibody expression. After 7 days of culture, the cell cultures were collected and subjected to affinity purification on a Protein A column to obtain humanized antibodies.

Humanized antibody 26B12H2L2 is also referred to herein as 26B12H2L2(hG1WT).

3. Design of humanized antibodies 26B12H2L2(hG1DM) and 26B12H2L2(hG4DM)

The inventor obtained a humanized antibody 26B12H2L2(hG1DM) with mutated constant regions by introducing a leucine-to-alanine point mutation at position 234 (L234A) (according to the EU numbering system, same below) and a leucine-to-alanine point mutation at position 235 (L235A) in the heavy chain constant region of 26B12H2L2. The amino acid sequence of heavy chain 26B12H2(hG1DM) of 26B12H2L2(hG1DM) is set forth in SEQ ID NO: 31; the amino acid sequence of the light chain thereof is set forth in SEQ ID NO: 29. The inventor obtained a humanized antibody 26B12H2L2(hG4DM) with mutated constant regions by using Ig gamma-4 chain C region (ACCESSION: P01861.1) as a heavy chain constant region and introducing a leucine-to-alanine point mutation at position 234 (L234A) and a leucine-to-alanine point mutation at position 235 (L235A) in the heavy chain constant region of 26B12H2L2 while keeping the antibody variable region thereof constant. The amino acid sequence of heavy chain 26B12H2(hG4DM) of 26B12H2L2(hG4DM) is set forth in SEQ ID NO: 27; the amino acid sequence of the light chain thereof is set forth in SEQ ID NO: 29.

Humanized antibodies 26B12H2L2(hG1DM) and 26B12H2L2(hG4DM) were prepared by the method described above in step 2.

Example 4: Sequence Design and Preparation of Fusion Protein of Anti-TIGIT Antibody and TGF-βR

1. Sequence design

The composition of the fusion protein of the anti-TIGIT antibody and TGF-βR is shown in Table 2 below.

Table 2: Composition of heavy chain and light chain of fusion protein of anti-TIGIT antibody and TGF-βR

| Fusion protein name | Heavy chain of IgG moiety | Linker fragment | Truncated fragment of TGF-βRII extracellular region | Light chain |
|---|---|---|---|---|
| TF01 | SEQ ID NO: 27 | SEQ ID NO: 44 | SEQ ID NO: 33 | SEQ ID NO: 29 |
| TF02 | SEQ ID NO: 31 | SEQ ID NO: 44 | SEQ ID NO: 33 | SEQ ID NO: 29 |

The TGF-β receptor moiety has two peptide chains each linked to the C-terminus of the heavy chain of the IgG moiety via a linker fragment.

2. Expression and purification of antibodies

The heavy chain cDNA sequence and the light chain cDNA sequence of TF01 and TF02 were separately cloned into vector pUC57simple (provided by Genscript) to obtain plasmids pUC57simple-TF01H and pUC57simple-TF02H, or pUC57simple-TF01L and pUC57simple-TF02L, respectively.

Plasmids pUC57simple-TF01H/pUC57simple TF01L and pUC57simple-TF02H/pUC57simple TF02L were each digested (HindIII&EcoRI), and the heavy and light chains isolated by electrophoresis were separately subcloned into vectors pcDNA3.1, and recombinant plasmids were extracted to co-transfect 293F cells. After 7 days of cell culture, the cultures were separated by high-speed centrifugation, and the supernatant was concentrated and loaded onto a HiTrap MabSelect SuRe column. The protein was eluted in one step with an elution buffer. The target sample was isolated and the buffer was exchanged into PBS. Therefore, fusion proteins TF01 and TF02 of the anti-TIGIT antibody and TGF-βR were prepared.

Example 5: ELISA Assays for Binding Activity of TF01 and TF02 to Antigen or TGF-β

1. Indirect ELISA assays for binding activity of TF01, TF02, 26B12H2L2(hG4DM), and RG6058(hG4) to antigen TIGIT-mFc

The method is as follows:

A microplate was coated with goat anti-mouse IgG Fc (Jackson, Cat. No. 115-005-071) at 2 μg/mL and incubated at 4 °C overnight. Then the microplate was washed once with PBST, and then blocked with a PBS solution containing 1% BSA as the blocking solution at 37 °C for 2 h. After blocking, the microplate was washed 3 times with PBST. Then 1 μg/mL of TIGIT-mFc was added, and the microplate was incubated at 37 °C for 30 min and then washed 3 times with PBST. The antibodies serially diluted with PBST solution (the dilution gradients for the antibody are shown in Table 3) were added. The microplate containing the test antibodies was incubated at 37 °C for 30 min, and then washed 3 times with PBST. After washing, a secondary antibody working solution of HRP-labeled goat anti-human IgG (H+L) (Jackson, Cat. No. 109-035-098) diluted at a ratio of 1:5000 was added, and then the microplate was incubated at 37 °C for 30 min. After incubation, the plate was washed 4 times with PBST. Then TMB (Neogen, 308177) was added in the dark for chromogenesis for 5 min, and then a stop solution was added to terminate the chromogenic reaction. The microplate was put into a microplate reader immediately, and the OD value of each well in the microplate was read at 450 nm. The data were analyzed and processed by SoftMax Pro 6.2.1.

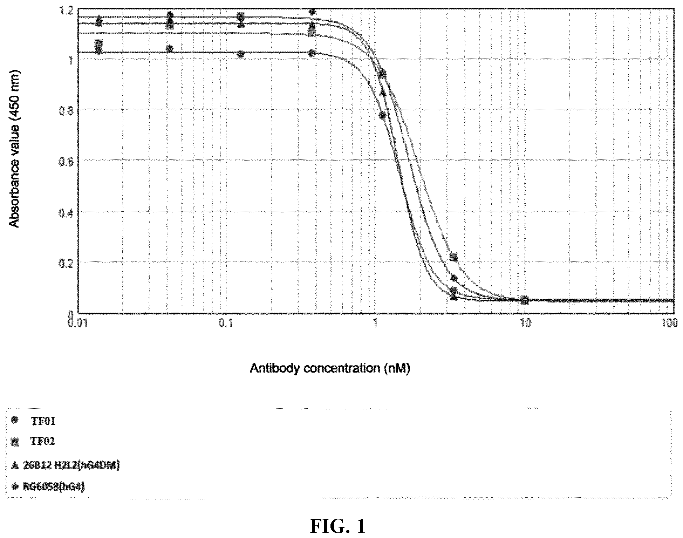

The results are shown in Table 3 and FIG. 1.

Table 3: ELISA assays for binding of TF01, TF02, 26B12H2L2(hG4DM), and RG6058(hG4) to TIGIT-mFc

| TIGIT-mFc (1 μg/mL), 50 μL/well | | | | | | | |
|---|---|---|---|---|---|---|---|
| Antibody dilution | TF01 | | TF02 | | 26B12H2L2(hG4DM) | | RG6058(hG4) | |
| gradient (nM) | Initial antibody concentration (μg/mL) | | | | | | | |
| | 0.399 | | 0.399 | | 0.325 | | 0.332 | |
| 2.240 | 2.965 | 2.902 | 2.950 | 2.911 | 2.952 | 2.999 | 3.032 | 3.017 |
| 0.747 | 2.921 | 2.862 | 2.861 | 2.819 | 2.972 | 2.993 | 3.018 | 3.040 |

(continued)

| TIGIT-mFc (1 μg/mL), 50 μL/well | | | | | | | |
|---|---|---|---|---|---|---|---|
| Antibody dilution | TF01 | | TF02 | | 26B12H2L2(hG4DM) | | RG6058(hG4) | |
| gradient (nM) | Initial antibody concentration (μg/mL) | | | | | | | |
| | 0.399 | | 0.399 | | 0.325 | | 0.332 | |
| 0.249 | 2.625 | 2.473 | 2.374 | 2.350 | 2.703 | 2.650 | 2.691 | 2.739 |
| 0.083 | 1.808 | 1.669 | 1.475 | 1.438 | 2.054 | 2.117 | 1.898 | 2.015 |
| 0.028 | 0.889 | 0.818 | 0.700 | 0.685 | 1.134 | 1.158 | 0.992 | 1.081 |
| 0.009 | 0.404 | 0.362 | 0.305 | 0.307 | 0.529 | 0.546 | 0.460 | 0.505 |
| 0.0031 | 0.210 | 0.178 | 0.165 | 0.167 | 0.252 | 0.265 | 0.223 | 0.261 |
| 0 | 0.109 | 0.102 | 0.097 | 0.105 | 0.098 | 0.117 | 0.113 | 0.131 |
| Secondary antibody | Goat anti-human IgG (H+L), HRP (1:5000) | | | | | | | |
| $EC_{50}$ (nM) | 0.067 | | 0.091 | | 0.046 | | 0.055 | |

It can be seen from the figure that TF01, TF02, 26B12H2L2(hG4DM), and RG6058(hG4) could effectively bind to the antigen TIGIT-mFc in a dose-dependent manner. The $EC_{50}$ values for all the doses are shown in Table 3. By quantitative analysis of the absorbance of the bound antibodies, the binding efficiency $EC_{50}$ values of TF01, TF02, 26B12H2L2(hG4DM), and RG6058(hG4) (as a control) were 0.067 nM, 0.091 nM, 0.046 nM, and 0.055 nM, respectively, as obtained by curve fitting.

The above experimental results show that TF01, TF02, 26B12H2L2(hG4DM), and RG6058(hG4) had the activity of effectively binding to TIGIT-mFc.

2. Indirect ELISA assays for binding activity of TF01, TF02, and TGF-βRII-mFc to TGF-β1, TGF-β2, and TGF-β3

The method is as follows:

Three microplates were coated with TGF-β1, TGF-β2, and TGF-β3 at 1 μg/mL, 2 μg/mL, and 1 μg/mL, respectively, and incubated at 4 °C overnight.

Then the microplates coated with antigens were washed once with PBST, and then blocked with a PBS solution containing 1% BSA as the blocking solution at 37 °C for 2 h. After blocking, the microplates were washed 3 times with PBST. Then the antibodies serially diluted with PBST solution and TGF-βRII-mFc were added. The microplates containing the test antibodies and TGF-βRII-mFc were incubated at 37 °C for 30 min, and then washed 3 times with PBST. After washing, a secondary antibody working solution of HRP-labeled goat anti-human IgG (H+L) (Jackson, Cat. No. 109-035-098) and HRP-labeled goat anti-mouse IgG Fc (Jackson, Cat. No. 115-035-071) diluted at a ratio of 1:5000 was added, and then the microplates were incubated at 37 °C for 30 min. After incubation, the plates were washed 4 times with PBST. Then TMB (Neogen, 308177) was added in the dark for chromogenesis for 5 min, and then a stop solution was added to terminate the chromogenic reaction. The microplates were put into a microplate reader immediately, and the OD value of each well in the microplates was read at 450 nm. The data were analyzed and processed by SoftMax Pro 6.2.1.

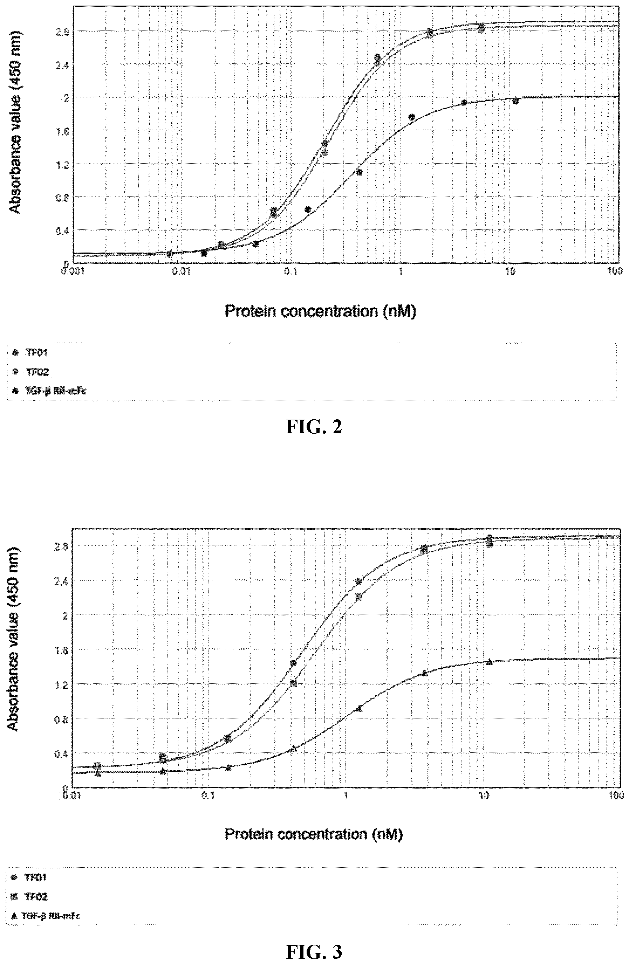

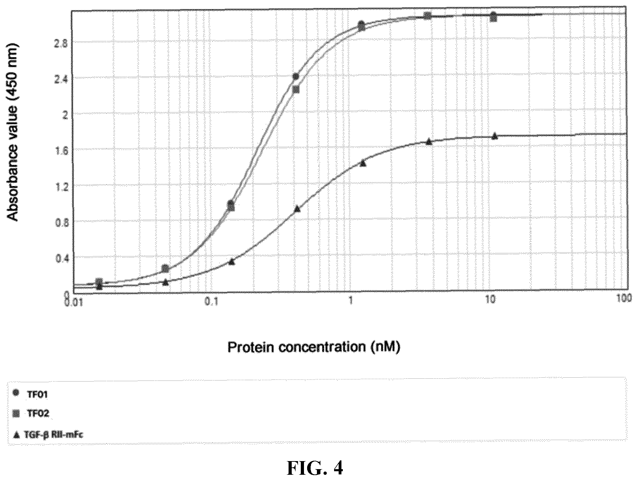

The results are shown in Tables 4, 5, and 6 and FIGs. 2, 3, and 4.

Table 4: ELISA assays for binding of TF01, TF02, and TGF-βRII-mFc to TGF-β1

| Antibody dilution gradient (μg/mL) | TGF-β1 (1 μg/mL), 50 μL/well | | | | | |
|---|---|---|---|---|---|---|
| | TF01 | | TF02 | | TGF-β RII-mFc | |
| 1.000 | 2.885 | 2.823 | 2.786 | 2.819 | 1.896 | 2.007 |
| 0.333 | 2.811 | 2.771 | 2.695 | 2.769 | 1.949 | 1.909 |
| 0.111 | 2.515 | 2.432 | 2.379 | 2.411 | 1.787 | 1.721 |
| 0.037 | 1.525 | 1.352 | 1.358 | 1.304 | 1.136 | 1.044 |

(continued)

| Antibody dilution gradient (μg/mL) | TGF-β1 (1 μg/mL), 50 μL/well | | | | | |
| | TF01 | | TF02 | | TGF-β RII-mFc | |
| 0.012 | 0.663 | 0.611 | 0.612 | 0.548 | 0.673 | 0.602 |
| 0.004 | 0.233 | 0.206 | 0.206 | 0.190 | 0.240 | 0.206 |
| 0.001 | 0.113 | 0.100 | 0.103 | 0.092 | 0.119 | 0.099 |
| 0.000 | 0.063 | 0.056 | 0.057 | 0.060 | 0.053 | 0.224 |
| Secondary antibody | Goat anti-human IgG (H+L), HRP (1:5000), 50 μL/well | | | | Goat anti-mouse IgG mFC, HRP (1:5000), 50 μL/well | |
| $EC_{50}$ (nM) | 0.207 | | 0.224 | | 0.356 | |

Table 5: ELISA assays for binding of TF01, TF02, and TGF-βRII-mFc to TGF-β2

| Antibody dilution gradient (nM) | TGF-β2 (2 μg/mL) | | | | | |
| | TF01 | | TF02 | | TGF-β RII-mFc | |
| Initial protein concentration (μg/mL) | 2.001 | | 2.000 | | 0.971 | |
| 11.23 nM | 2.874 | 2.896 | 2.821 | 2.812 | 1.462 | 1.436 |
| 1:3 | 2.782 | 2.769 | 2.764 | 2.716 | 1.336 | 1.322 |
| 1:9 | 2.411 | 2.353 | 2.224 | 2.186 | 0.908 | 0.925 |
| 1:27 | 1.507 | 1.353 | 1.197 | 1.199 | 0.432 | 0.462 |
| 1:81 | 0.707 | 0.430 | 0.548 | 0.554 | 0.253 | 0.211 |
| 1:243 | 0.364 | 0.350 | 0.314 | 0.316 | 0.202 | 0.158 |
| 1:729 | 0.237 | 0.243 | 0.231 | 0.243 | 0.184 | 0.152 |
| 0 | 0.164 | 0.171 | 0.181 | 0.191 | 0.176 | 0.160 |
| Secondary antibody | Goat anti-human IgG (H+L)-HRP (1:5000) | | | | Goat anti-mouse IgG Fc-HRP (1:5000) | |
| $EC_{50}$ (nM) | 0.477 | | 0.594 | | 1.029 | |

Table 6: ELISA assays for binding of TF01, TF02, and TGF-βRII-mFc to TGF-β3

| Antibody dilution gradient (nM) | TGF-β3 (1 μg/mL) | | | | | |
| | TF01 | | TF02 | | TGF-β RII-mFc | |
| Initial protein concentration (μg/mL) | 2.001 | | 2.000 | | 0.971 | |
| 11.23 nM | 3.065 | 3.042 | 3.009 | 3.035 | 1.663 | 1.765 |
| 1:3 | 3.058 | 3.048 | 3.048 | 3.054 | 1.667 | 1.649 |
| 1:9 | 2.957 | 2.976 | 2.931 | 2.944 | 1.411 | 1.447 |
| 1:27 | 2.382 | 2.409 | 2.269 | 2.213 | 0.912 | 0.934 |
| 1:81 | 0.993 | 0.980 | 0.964 | 0.913 | 0.338 | 0.340 |
| 1:243 | 0.263 | 0.278 | 0.270 | 0.252 | 0.113 | 0.114 |

(continued)

| Antibody dilution gradient (nM) | TGF-$\beta$3(1 $\mu$g/mL) | | | | | |
|---|---|---|---|---|---|---|
| | TF01 | | TF02 | | TGF-$\beta$ RII-mFc | |
| 1:729 | 0.120 | 0.118 | 0.116 | 0.115 | 0.071 | 0.074 |
| 0 | 0.057 | 0.055 | 0.054 | 0.055 | 0.056 | 0.059 |
| Secondary antibody | Goat anti-human IgG (H+L)-HRP (1: 5000) | | | | Goat anti-mouse IgG Fc-HRP (1: 5000) | |
| EC$_{50}$ (nM) | 0.215 | | 0.234 | | 0.402 | |

By quantitative analysis of the absorbance of the bound antibodies, the binding efficiency EC$_{50}$ values of TF01 to TGF-$\beta$1, TGF-$\beta$2, and TGF-$\beta$3 were 0.207 nM, 0.477 nM, and 0.215 nM, respectively, the binding efficiency EC$_{50}$ values of TF02 to TGF-$\beta$1, TGF-$\beta$2, and TGF-$\beta$3 were 0.224 nM, 0.594 nM, and 0.234 nM, respectively, and the binding efficiency EC$_{50}$ values of TGF-$\beta$R2-mFc(as a positive control) to TGF-$\beta$1, TGF-$\beta$2, and TGF-$\beta$3 were 0.356 nM, 1.029 nM, and 0.402 nM, respectively, as obtained by curve fitting. The results show that TF01 and TF02 had the activity of effectively binding to TGF-$\beta$1, TGF-$\beta$2, and TGF-$\beta$3 in a dose-dependent manner. The results also show that TF01, TF02, and TGF-$\beta$RII-mFc each could effectively bind to TGF-$\beta$1, TGF-$\beta$2, and TGF-$\beta$3, respectively, and TF01 and TF02 had a stronger capacity of binding to TGF-$\beta$1, TGF-$\beta$2, and TGF-$\beta$3 than TGF-$\beta$RII-mFc.

Example 6: ELISA Assays for Activity of TF01 and TF02 Competing with TGF-$\beta$RII-His-Biotin for Binding to Human TGF-$\beta$1 and TGF-$\beta$3

Experimental steps: two microplates were coated with TGF-$\beta$1 and TGF-$\beta$3 at 2 $\mu$g/mL, respectively, and incubated at 4 °C overnight. After incubation, the microplates coated with antigens were rinsed once with PBST, and then blocked with a PBS solution containing 1% BSA as the microplate blocking solution for 2 h. After blocking, the microplates were washed 3 times with PBST. The antibodies serially diluted with PBST solution and TGF-$\beta$RII-mFc were added to the microplates. For the TGF-$\beta$1 microplate, after incubation at room temperature for 60 min, the plate was washed 3 times with PBST, and then incubated at room temperature for 10 min with the addition of TGF-$\beta$RII-His-Biotin at 0.01 $\mu$g/mL. For the TGF-$\beta$3 microplate, after incubation at 37 °C for 30 min, the plate was washed 3 times with PBST, and then incubated at 37 °C for 30 min with the addition of TGF-$\beta$RII-His-Biotin at 0.01 $\mu$g/mL. After incubation, the plates were washed 3 times with PBST. After washing, an SA-HRP working solution diluted at a ratio of 1:4000 was added, and the microplates were incubated at 37 °C for 30 min. After incubation, the plates were washed 4 times with PBST. Then TMB (Neogen, 308177) was added in the dark for chromogenesis for 4min, and then a stop solution was added to terminate the chromogenic reaction. The microplates were put into a microplate reader immediately, and the OD value of each well in the microplates was read at 450 nm. The data were analyzed and processed by SoftMax Pro 6.2.1.

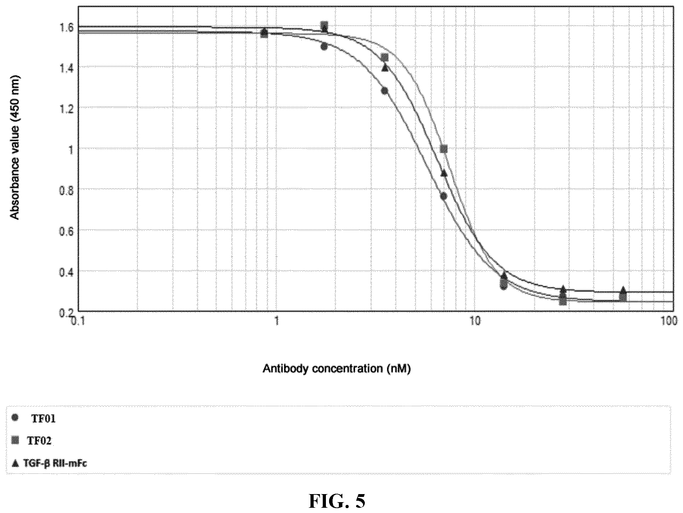

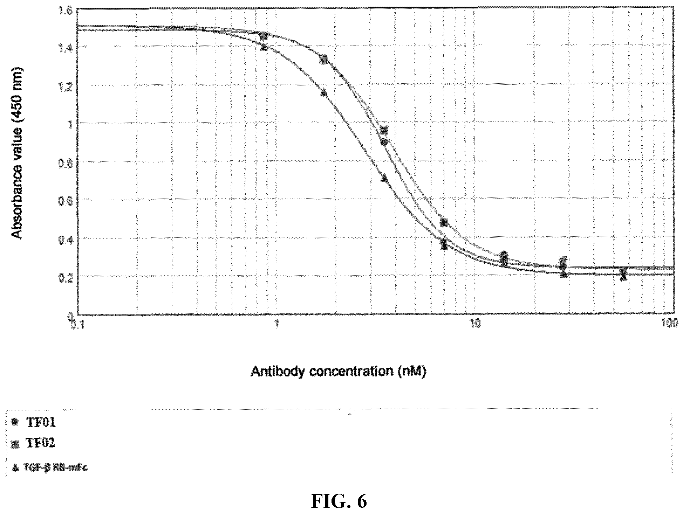

The results of antibodies competing with TGF-$\beta$RII-His-Biotin for binding to TGF-$\beta$1 and TGF-$\beta$3 are shown in Tables 7 and 8, and FIGs. 5 and 6. An absorbance vs. antibody concentration curve was fitted, and the EC$_{50}$ values of the antibodies competing with TGF-$\beta$RII-His-Biotin for binding to TGF-$\beta$1 and TGF-$\beta$3 were calculated. The results are shown in Tables 7 and 8 below.

Table 7: Assays for activity of TF01 and TF02 competing with TGF-RII-His-Biotin for binding to TGF-$\beta$1

| Antibody dilution gradient (nM), 100 $\mu$L/well | TGF-$\beta$1 (2 $\mu$g/mL), 50 $\mu$L/well | | | | | |
|---|---|---|---|---|---|---|
| | TF01 | | TF02 | | TGF-$\beta$ RII-mFc | |
| Initial protein concentration ($\mu$g/mL) | 10.000 | | 9.998 | | 4.851 | |
| 56.134 | 0.252 | 0.281 | 0.256 | 0.279 | 0.294 | 0.317 |
| 28.067 | 0.275 | 0.270 | 0.222 | 0.266 | 0.305 | 0.311 |
| 14.034 | 0.324 | 0.317 | 0.316 | 0.350 | 0.396 | 0.361 |
| 7.017 | 0.714 | 0.815 | 0.961 | 1.032 | 0.933 | 0.828 |
| 3.508 | 1.267 | 1.296 | 1.385 | 1.508 | 1.435 | 1.360 |
| 1.754 | 1.491 | 1.497 | 1.590 | 1.618 | 1.601 | 1.576 |

(continued)

| Antibody dilution gradient (nM), 100 μL/well | TGF-β1 (2 μg/mL), 50 μL/well | | | | | |
|---|---|---|---|---|---|---|
| | TF01 | | TF02 | | TGF-β RII-mFc | |
| 0.877 | 1.561 | 1.573 | 1.524 | 1.595 | 1.609 | 1.543 |
| 0 | 1.629 | 1.561 | 1.570 | 1.545 | 1.609 | 1.603 |
| TGF-βR2-His-Biotin, 0.01 μg/mL, 50 μL/well | | | | | | |
| Secondary antibody: SA-HRP (1:4000), 50 μL/well | | | | | | |
| $EC_{50}$ (nM) | 15.759 | | 17.469 | | 6.475 | |

Table 8: Assays for activity of TF01 and TF02 competing with TGF-βRII-His-Biotin for binding to TGF-β3

| Antibody dilution gradient, 50 μL/well | TGF-β3 (2 μg/mL), 50 μL/well | | | | | |
|---|---|---|---|---|---|---|
| | TF01 | | TF02 | | TGF-β RII-mFc | |
| Initial protein concentration (μg/mL) | 10.000 | | 9.998 | | 4.851 | |
| 56.134 | 0.232 | 0.213 | 0.210 | 0.220 | 0.181 | 0.197 |
| 28.067 | 0.249 | 0.233 | 0.233 | 0.299 | 0.198 | 0.218 |
| 14.034 | 0.307 | 0.293 | 0.299 | 0.279 | 0.255 | 0.277 |
| 7.017 | 0.364 | 0.378 | 0.486 | 0.456 | 0.372 | 0.335 |
| 3.508 | 0.897 | 0.892 | 0.973 | 0.937 | 0.813 | 0.601 |
| 1.754 | 1.294 | 1.344 | 1.327 | 1.323 | 1.272 | 1.040 |
| 0.877 | 1.427 | 1.466 | 1.436 | 1.466 | 1.476 | 1.311 |
| 0 | 1.511 | 1.500 | 1.535 | 1.508 | 1.575 | 1.453 |
| TGF-βRII-His-Biotin, 0.01 μg/mL, 50 μL/well | | | | | | |
| Secondary antibody | SA-HRP(1:4000) | | | | | |
| $EC_{50}$ (nM) | 3.569 | | 3.879 | | 2.808 | |

By quantitative analysis of the absorbance of the bound antibodies, the binding efficiency $EC_{50}$ values of TF01, TF02, and TGF-βRII-mFc competing with TGF-βRII-His-Biotin for binding to TGF-β1 were 5.579 nM, 7.469 nM, and 6.475 nM, respectively, and the binding efficiency $EC_{50}$ values of TF01, TF02, and TGF-βRII-mFc competing with TGF-βRII-His-Biotin for binding to TGF-β3 were 3.569 nM, 3.879 nM, and 2.808 nM, respectively, as obtained by curve fitting.
The results show that TF01 and TF02 could effectively bind to TGF-β1 and TGF-β3 in a dose-dependent manner. In addition, TF01 has a stronger capacity of binding to TGF-β1 than TGF-βRII-His-Biotin. TF01 had a stronger capacity of competing with TGF-βRII-His-Biotin for binding to TGF-β1 than TGF-βRII-mFc.

Example 7: ELISA Assays for Activity of TF01 and TF02 Competing with CD155-hFc-Biotin for Binding to Human TIGIT-mFc

A microplate was coated with human TIGIT-mFc (TIGIT Genbank ID: NP-776160.2) at 2 μg/mL and incubated at 4 °C overnight. After incubation, the microplate was blocked with a PBS solution containing 1% BSA at 37 °C for 2 h. After blocking, the plate was washed three times and dried. The antibody was serially diluted to 7 concentrations at a gradient ratio of 1:3 on a dilution plate with 20.18 nM (at a final concentration of 10.09 nM) as the starting concentration, and a blank control was set. Then an equal volume of 4 μg/mL (at a final concentration of 2 μg/mL) of human CD155-hFc-Biotin solution was added, and the system was mixed well. Then the mixture was incubated at room temperature for 20 min. Then the mixture after reaction was added to the coated microplate, and the microplate was incubated at 37 °C for 30 min. After incubation, the plate was washed three times with PBST and dried. An SA-HRP (KPL, 14-30-00) working solution was added, and the plate was incubated at 37 °C for 30 min. After incubation, the plate was washed four times and dried. Then TMB (Neogen, 308177) was added in the dark for chromogenesis for 5 min, and then a stop solution was added to terminate chromogenic reaction. The microplate was put into a microplate reader immediately, and the OD value of each well in the microplate was read at 450 nm. The data were analyzed and processed by SoftMax Pro 6.2.1.

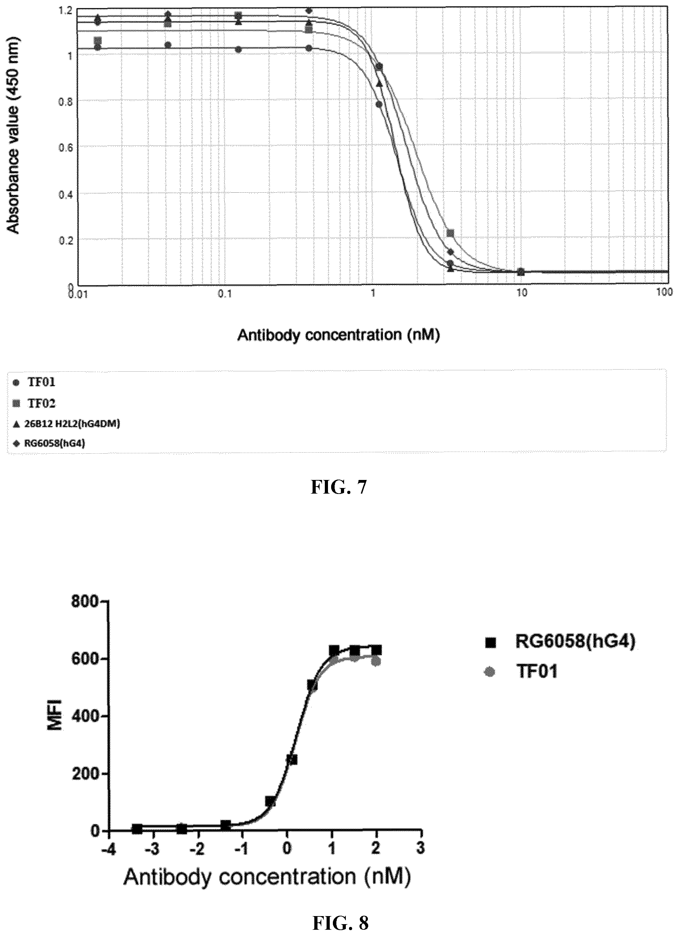

The results are shown in FIG. 7. The OD values for all the dosages are shown in Table 9. By quantitative analysis of the absorbance intensity of the bound antibodies, the binding efficiency $EC_{50}$ values of the antibodies were as obtained by curve fitting (Table 9).

Table 9: Assays for activity of TF01, TF02, 26B12H2L2(hG4DM), and RG6058(hG4) competing with CD155-hFc-Biotin for binding to TIGIT-mFc

| Antibody dilution (nM) | TIGIT-mFc (2 μg/mL), 50 μL/well | | | | | | | |
|---|---|---|---|---|---|---|---|---|
| | TF01 | | TF02 | | 26B12H2L2 (hG4DM) | | RG6058(hG4) | |
| Initial final antibody concentration (μg/mL) | 1.797 | | 1.797 | | 1.463 | | 1.496 | |
| 10.090 | 0.055 | 0.051 | 0.052 | 0.049 | 0.049 | 0.046 | 0.047 | 0.052 |
| 3.363 | 0.085 | 0.086 | 0.221 | 0.212 | 0.066 | 0.065 | 0.151 | 0.127 |
| 1.121 | 0.786 | 0.765 | 0.923 | 0.949 | 0.865 | 0.874 | 0.913 | 0.974 |
| 0.374 | 0.982 | 1.059 | 1.099 | 1.106 | 1.148 | 1.117 | 1.170 | 1.199 |
| 0.125 | 0.993 | 1.042 | 1.148 | 1.180 | 1.123 | 1.150 | 1.153 | 1.169 |
| 0.042 | 1.028 | 1.051 | 1.118 | 1.144 | 1.174 | 1.141 | 1.203 | 1.144 |
| 0.0138 | 1.080 | 0.979 | 1.036 | 1.083 | 1.155 | 1.165 | 1.137 | 1.139 |
| 0 | 1.001 | 1.039 | 1.011 | 1.058 | 1.095 | 1.130 | 1.182 | 1.126 |
| Primary antibody | CD155-hFc-Biotin (2 μg/mL), 50 μL/well | | | | | | | |
| Secondary antibody | SA-HRP(1:4000) | | | | | | | |
| $EC_{50}$ (nM) | 1.463 | | 1.963 | | 1.423 | | 1.675 | |

The $EC_{50}$ values for blocking the binding of TIGIT-mFc to its ligand CD155-hFc-Biotin by TF01, TF02, 26B12H2L2(hG4DM), and RG6058(hG4) (as a control) were 1.463 nM, 1.963 nM, 1.423 nM, and 1.675 nM, respectively. The results show that TF01, TF02, 26B12H2L2(hG4DM), and RG6058(hG4) (as a control) could effectively block the binding of antigen human CD155-hFc-Biotin to its receptor human TIGIT-mFc in a dose-dependent manner; TF01 had a stronger capacity of blocking the binding of TIGIT-mFc to its ligand CD155-hFc-Biotin than the control antibody RG6058(hG4).

Example 8: FACS Assay for Binding Activity of TF01 to TIGIT on 293T-TIGIT Membrane Surface

293T-TIGIT cells in logarithmic growth phase were collected and transferred to a transparent V-bottomed 96-well plate at $3 \times 10^5$ cell/well. 350 g of 1% PBSA was added, and the mixture was centrifuged for 5 min to remove the supernatant. 100 μL of antibodies diluted by 1% PBSA (at the final concentrations of 100 nM, 33.33 nM, 11.11 nM, 3.7 nM, 1.23 nM, 0.41 nM, 0.041 nM, 0.0041 nM, and 0.00041 nM, respectively) were added, respectively. The system was mixed gently and uniformly, and then the mixture was incubated on ice for 1 h. Then 350 g of 1% PBSA was added, and the mixture was centrifuged for 5 min to remove the supernatant. The 350-fold diluted FITC labeled goat anti-human IgG secondary antibody (Jackson, Cat. No. 109-095-098) was added for resuspension and uniform mixing, and the mixture was incubated on ice in the dark for 0.5 h. 350 g of 1% PBSA was added, and the mixture was centrifuged for 5 min to remove the supernatant. At last, 400 μL of PBSA was added to resuspend cell precipitates, and the resulting mixture was transferred to a flow tube for FACS Calibur assay.

The experimental results are shown in Table 10 and FIG. 8 that TF01 and RG6058(hG4) (control antibody) could both specifically bind to TIGIT on the 293T-TIGIT membrane surface.

Table 10: FACS assays for binding activity of TF01 and RG6058(hG4) to TIGIT on 293T-TIGIT cell surface

| Antibody dilution gradient (nM) | TF01 | RG6058 (hG4) |
|---|---|---|
| 0.00041 | 8.83 | 9.07 |
| 0.0041 | 10.00 | 10.30 |
| 0.041 | 18.50 | 19.50 |
| 0.41 | 97.40 | 106.00 |
| 1.23 | 244.00 | 251.00 |
| 3.7 | 497.00 | 508.00 |
| 11.1 | 596.00 | 629.00 |
| 33.3 | 604.00 | 630.00 |
| 100 | 589.00 | 630.00 |
| $EC_{50}$ (nM) | 1.540 | 1.612 |
| R2 | 0.9981 | 0.9981 |

The $EC_{50}$ values of TF01 and RG6058(hG4) binding to 293T-TIGIT cells were 1.540 nM and 1.612 nM, respectively, under the same experimental conditions. The results show that TF01 had the activity of effectively binding to TIGIT on the 293T-TIGIT membrane surface in a dose-dependent manner, and had the stronger binding capacity than the control antibody RG6058(hG4).

Example 9: Assays of TF01 Competing with CD155 or CD112 for Binding to Antigen TIGIT on Cell Membrane Surface by Competitive Flow Cytometry

The 293T-TIGIT cells was digested in a conventional way, and divided into several samples with 300,000 cells for each well, which were then subjected to centrifugation and washing. Then 100 μL of corresponding gradiently diluted antibody was added to each well, and the mixture was incubated on ice for 30 min. 100 μL of CD155/CD112-mFc (prepared by Akeso Biopharma Inc., Lot No. 20190726/20190726) was then added to each well, and the system was mixed well to reach a final concentration of 10 nM/30 nM, and then the mixture was incubated on ice for 1 h. Then 350 g of 1% PBSA was added, and the mixture was centrifuged for 5 min to remove the supernatant. 100 μL of APC Goat anti-mouse IgG antibody (Biolegend, Cat. No. 405308) diluted at a ratio of 1:300 was then added to each well, while 100 μL of 1% PBSA was added to a blank sample, and the system was mixed well. Then the mixture was incubated on ice in the dark for 30 min, then centrifuged, washed and resuspended, and then transferred to a flow cytometry tube for testing.

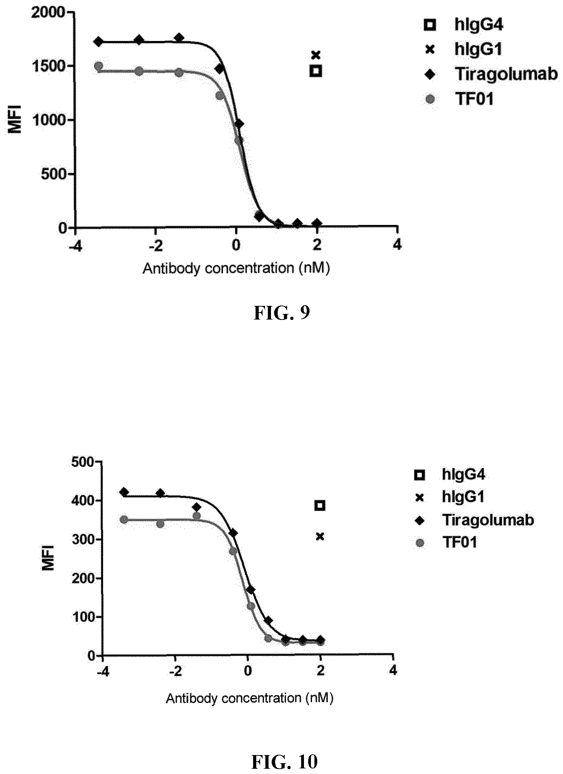

The results are shown in FIGs. 9 and 10, and the $EC_{50}$ values are shown in Table 11. By fluorescence quantification analysis and curve fitting, the $EC_{50}$ values of TF01 competing for binding to antigen were calculated to be 1.2720 nM and 0.7445 nM, respectively.

Table 11: FACS assay for fluorescence intensity analysis of TF01 competing for binding to 293T-TIGIT surface antigen

| Antibody dilution gradient (nM) | CD155 | | CD112 | |
|---|---|---|---|---|
| | TF01 | Tiragolumab | TF01 | Tiragolumab |
| 0.00041 | 1499.0 | 1725.0 | 350.0 | 421.0 |
| 0.0041 | 1449.0 | 1739.0 | 339.0 | 418.0 |
| 0.041 | 1432.0 | 1754.0 | 359.0 | 382.0 |
| 0.41 | 1220.0 | 1469.0 | 267.0 | 314.0 |
| 1.23 | 802.0 | 956.0 | 125.0 | 168.0 |
| 3.7 | 113.0 | 95.8 | 42.7 | 88.1 |
| 11.1 | 25.9 | 30.4 | 33.1 | 40.6 |

(continued)

| Antibody dilution gradient (nM) | CD155 | | CD112 | |
|---|---|---|---|---|
| | TF01 | Tiragolumab | TF01 | Tiragolumab |
| 33.3 | 25.5 | 30.0 | 33.1 | 38.2 |
| 100 | 25.0 | 31.1 | 32.5 | 38.3 |
| EC$_{50}$ (nM) | 1.2720 | 1.2760 | 0.7445 | 0.8370 |
| **R2** | 0.9958 | 0.9957 | 0.9985 | 0.9962 |

The results show that the TF01 antibody could effectively block the binding of CD155 and/or CD112 to TIGIT on the surface of 293T-TIGIT host cells in a dose-dependent manner.

Example 10: Assay for Affinity of TF01 for Fc Receptor FcγRI

The Fc receptor FcγRI (also known as CD64), can bind to the Fc fragment of IgG antibodies and is involved in antibody-dependent cell-mediated cytotoxicity (ADCC). The binding capacity of a therapeutic antibody to an Fc receptor will influence the safety and efficacy of the antibody. In the experiment, the affinity constants of TF01 to FcγRI were determined using a Fortebio Octet system to evaluate the ADCC activity of the antibodies.

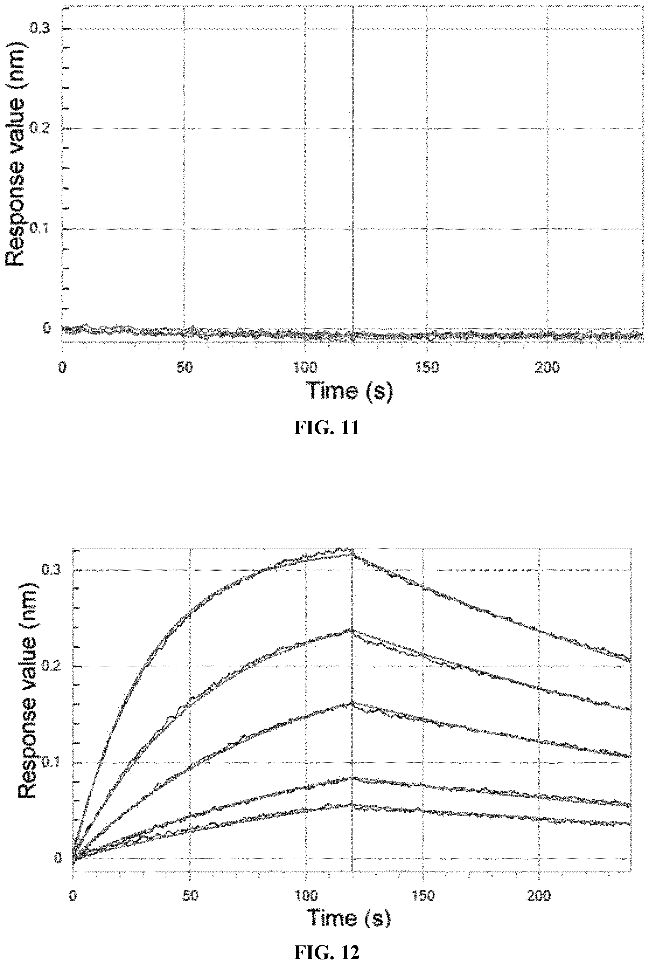

The method for determining the affinity constants of the corresponding antibodies to FcγRI by the Fortebio Octet system is briefly described as follows: The sample dilution buffer was a solution of 0.02% Tween-20 and 0.1% BSA in PBS, pH 7.4. 1 μg/mL of FcγRI solution (purchased from Sinobio) was added to the HISIK sensor to immobilize the FcγRI on the sensor surface for 50 s. The association and dissociation constants of the antibodies to FcγRI were both determined in the buffer with an antibody concentration of 3.12-50 nM (serial two-fold dilution). The shaking speed of the sample plate was 1000 rpm, the temperature was 30 °C and the frequency was 5.0 Hz. The data were fitted and analyzed with a 1:1 model to obtain the affinity constants. The results for the affinity constants of FcγRI to TF01 are shown in Table 12 and FIGs. 11-12.

Table 12: Kinetic parameters for binding of TF01 to FcγRI

| Antibody | K$_D$ (M) | Kon (1/Ms) | SE (kon) | Kdis (1/s) | SE (kdis) | Rmax (nm) |
|---|---|---|---|---|---|---|
| TF01 | N/A | N/A | N/A | N/A | N/A | N/A |
| 26B12H2L2 (hG1WT) | 6.51E-09 | 5.58E+05 | 2.90E+03 | 3.64E-03 | 1.54E-05 | 0.30-0.37 |

N/A indicates that the antibody had no binding or an extremely weak binding signal to the antigen, and thus the results were not analyzed and no corresponding data was obtained.

The results show that 26B12H2L2(hG1WT) could bind to FcγRI with an affinity constant of 6.51E-09M, and that TF01 had no binding or an extremely weak binding signal to FcγRI, and thus the results were not analyzed and no corresponding data was obtained.

The results show that the binding activity of TF01 to FcγRI was effectively eliminated.

Example 11: Assay for Affinity of TF01 for Fc Receptor FcγRIIIa and Subtypes thereof

(1) Assay for affinity constants of FcγRIIIa V158 to TF01

The Fc receptor FcγRIIIa_V158 (also known as CD16a_V158), can bind to the Fc fragment of IgG antibodies and mediate ADCC effects. In the experiment, the affinity constants of TF01 to FcγRIIIa_V158 were determined using a Fortebio Octet system to evaluate the ADCC activity of the antibodies.

The method for determining the affinity constants of the corresponding antibodies by the Fortebio Octet system is briefly described as follows: The sample dilution buffer was a solution of 0.02% Tween-20 and 0.1% BSA in PBS, pH 7.4. 5 μg/mL of FcγRIIIa_V158 was immobilized on the HISIK sensor for 60 s. The sensor was equilibrated in a buffer for 60 s, and the binding of the immobilized FcγRIIIa_V158 on the sensor to the antibodies at concentrations of 31.25-500 nM (serial two-fold dilution) was determined for 60 s. The antibodies were dissociated in the buffer for 60 s. The shaking speed of the sample plate was 1000 rpm, the temperature was 30 °C and the frequency was 5.0 Hz. The data were fitted and analyzed with a 1:1 model to obtain the affinity constants.

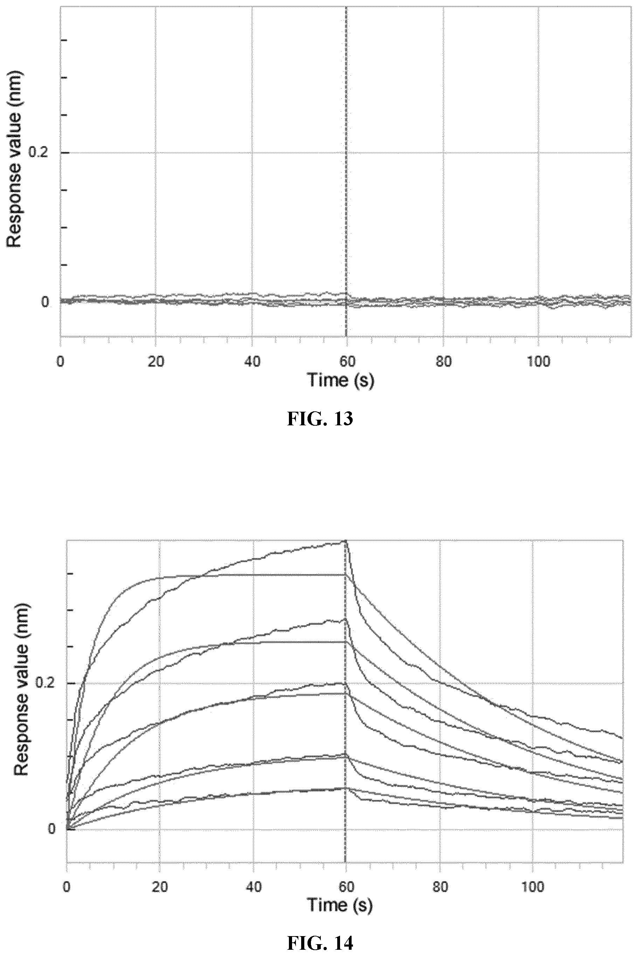

The results for the affinity constants of FcyRIIIa_V158 to TF01 are shown in Table 13 and FIGs. 13-14.

Table 13: Kinetic parameters for binding of TF01 to FcyRIIIa_V158

| Antibody | $K_D$ (M) | Kon (1/Ms) | SE (kon) | Kdis (1/s) | SE (kdis) | Rmax (nm) |
|---|---|---|---|---|---|---|
| TF01 | N/A | N/A | N/A | N/A | N/A | N/A |
| 26B12H2L2 (hG1WT) | 5.54E-08 | 4.02E+05 | 1.68E+04 | 2.23E-02 | 3.84E-04 | 0.18-0.39 |

N/A indicates that the antibody had no binding or an extremely weak binding signal to the antigen, and thus the results were not analyzed and no corresponding data was obtained.

The results show that 26B12H2L2(hG1WT) could bind to FcyRIIIa_V158 with an affinity constant of 5.54E-08M, and that TF01 had no binding or an extremely weak binding signal to FcyRIIIa_V158, and thus the results were not analyzed and no corresponding data was obtained.

The results show that the binding activity of TF01 to FcyR IIIa_V158 was effectively eliminated.

(2) Assay for affinity constants of FcyRIIIa F158 to TF01

The Fc receptor FcyRIIIa_F158 (also known as CD16a_F158), can bind to the Fc fragment of IgG antibodies and mediate ADCC effects. In the experiment, the affinity constants of TF01 to FcyRIIIa_F158 were determined using a Fortebio Octet system to evaluate the ADCC activity of the antibodies.

The method for determining the affinity constants of TF01 to FcyRIIIa_F158 by the Fortebio Octet system is briefly described as follows: The sample dilution buffer was a solution of 0.02% Tween-20 and 0.1% BSA in PBS, pH 7.4. 5 μg/mL of FcyRIIIa_V158 was immobilized on the HISIK sensor for 120 s. The sensor was equilibrated in a buffer for 60 s, and the binding of the immobilized FcyRIIIa_V158 on the sensor to the antibodies at concentrations of 31.25-500 nM (serial two-fold dilution) was determined for 60 s. The antibodies were dissociated in the buffer for 60 s. The shaking speed of the sample plate was 1000 rpm, the temperature was 30 °C and the frequency was 5.0 Hz. The data were fitted and analyzed with a 1:1 model to obtain the affinity constants.

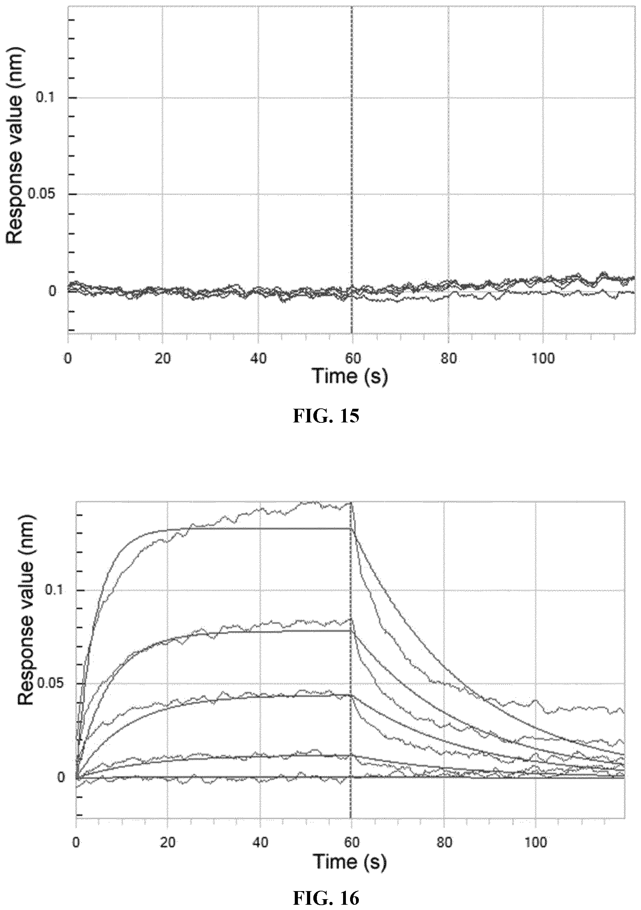

The results for the affinity constants of FcyRIIIa_F158 to TF01 are shown in Table 14 and FIGs. 15-16.

Table 14: Kinetic parameters for binding of TF01 to FcyRIIIa_F158

| Antibody | $K_D$ (M) | Kon (1/Ms) | SE (kon) | Kdis (1/s) | SE (kdis) | Rmax (nm) |
|---|---|---|---|---|---|---|
| TF01 | N/A | N/A | N/A | N/A | N/A | N/A |
| 26B12H2L2 (hG1WT) | 9.97E-08 | 4.03E+05 | 2.28E+04 | 4.02E-02 | 7.95E-04 | 0.00-0.16 |

N/A indicates that the antibody had no binding or an extremely weak binding signal to the antigen, and thus the results were not analyzed and no corresponding data was obtained.

The results show that 26B12H2L2(hG1WT) could bind to FcFcyRIIIa_F158 with an affinity constant of 9.97E-08M, and that TF01 had no binding or an extremely weak binding signal to FcFcyRIIIa_F158, and thus the results were not analyzed and no corresponding data was obtained.

The results show that the binding activity of TF01 to FcyRIIIa_F158 was effectively eliminated.

Example 12: Assay for Affinity of TF01 for Fc Receptor FcvRIIa and Subtypes thereof

(1) Assay for affinity constants of FcyRIIa H131 to TF01

The Fc receptor FcγRIIa_H131 (also known as CD32a_H131), can bind to the Fc fragment of IgG antibodies and is involved in antibody-dependent cell-mediated cytotoxicity (ADCC). The binding capacity of a therapeutic antibody to an Fc receptor will influence the safety and efficacy of the antibody. In the experiment, the affinity constants of TF01 to FcγRIIa_H131 were determined using a Fortebio Octet system to evaluate the binding capacity of the test antibodies to Fc receptors.

The method for determining the affinity constants of TF01 to FcyRIIa_H131 by the Fortebio Octet system is briefly described as follows: The sample dilution buffer was a solution of 0.02% Tween-20 and 0.1% BSA in PBS, pH 7.4. 5 μg/mL of FcγRIIa_H131 was immobilized on the NTA sensor at an immobilization height of about 1.0 nm. The sensor was equilibrated in a buffer for 60 s, and the binding of the immobilized FcγRIIa_H131 on the sensor to the antibodies at concentrations of 12.5-200 nM (serial two-fold dilution) was determined for 60 s. The antibodies were dissociated in the buffer for 60 s. The shaking speed of the sample plate was 1000 rpm, the temperature was 30 °C and the frequency was 5.0 Hz. The data were fitted and analyzed with a 1:1 model to obtain the affinity constants.

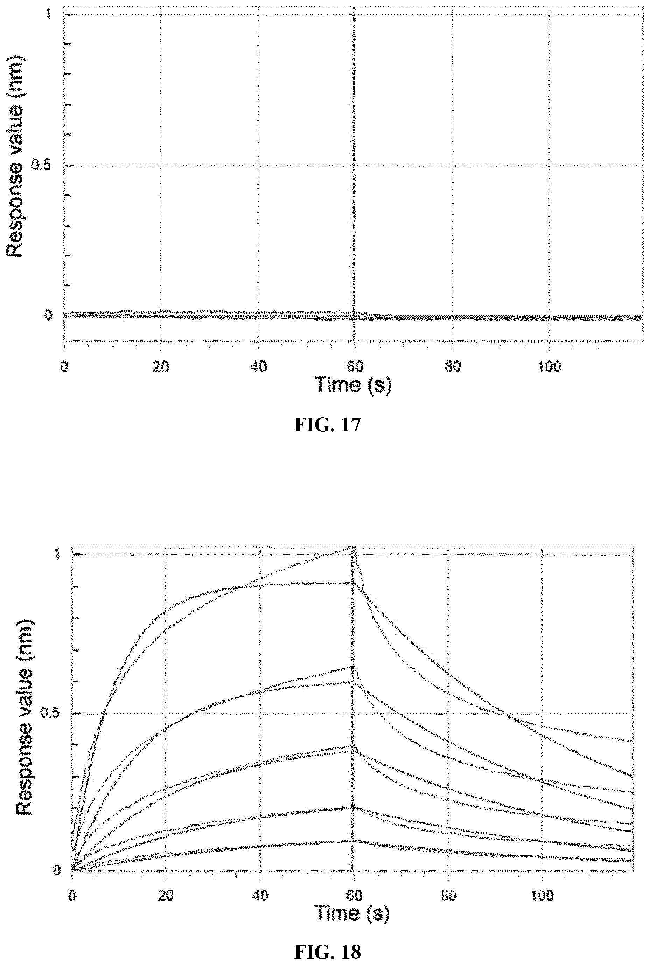

The results for the affinity constants of FcγRIIa_H131 to TF01 are shown in Table 15 and FIGs. 17-18.

Table 15: Kinetic parameters for binding of TF01 to FcγRIIa_H131

| Sample ID | $K_D$ (M) | Kon (1/Ms) | SE (kon) | Kdis (1/s) | SE (kdis) | Rmax (nm) |
|---|---|---|---|---|---|---|
| TF01 | N/A | N/A | N/A | N/A | N/A | N/A |
| 26B12H2L2 (hG1WT) | 3.90E-08 | 4.81E+05 | 1.60E+04 | 1.88E-02 | 2.82E-04 | 0.50-1.01 |

N/A indicates that the antibody had no binding or an extremely weak binding signal to the antigen, and thus the results were not analyzed and no corresponding data was obtained.

The results show that 26B12H2L2(hG1WT) could bind to FcγRIIa_H131 with an affinity constant of 3.90E-08M, and that TF01 had no binding or an extremely weak binding signal to FcγRIIa_H131, and thus the results were not analyzed and no corresponding data was obtained.

The results show that the binding activity of TF01 to FcγRIIa_H131 was effectively eliminated.

(2) Assay for affinity constants of FcγRIIa R131 to TF01

The Fc receptor FcyRIIa_R131 (also known as CD32a_R131) can bind to the Fc fragment of IgG antibodies and is involved in antibody-dependent cell-mediated cytotoxicity (ADCC). The binding capacity of a therapeutic antibody to an Fc receptor will influence the safety and efficacy of the antibody. In the experiment, the affinity constants of TF01 to FcγRIIa_R131 were determined using a Fortebio Octet system to evaluate the binding capacity of the test antibodies to Fc receptors.

The method for determining the affinity constants of TF01 to FcγRIIa_R131 by the Fortebio Octet system is briefly described as follows: The sample dilution buffer was a solution of 0.02% Tween-20 and 0.1% BSA in PBS, pH 7.4. 5 μg/mL of FcγRIIa_R131 was immobilized on the NTA sensor at an immobilization height of about 1.0 nm. The sensor was equilibrated in a buffer for 60 s, and the binding of the immobilized FcγRIIa_R131 on the sensor to the antibodies at concentrations of 12.5-200 nM (serial two-fold dilution) was determined for 60 s. The antibodies were dissociated in the buffer for 60 s. The shaking speed of the sample plate was 1000 rpm, the temperature was 30 °C and the frequency was 5.0 Hz. The data were fitted and analyzed with a 1:1 model to obtain the affinity constants.

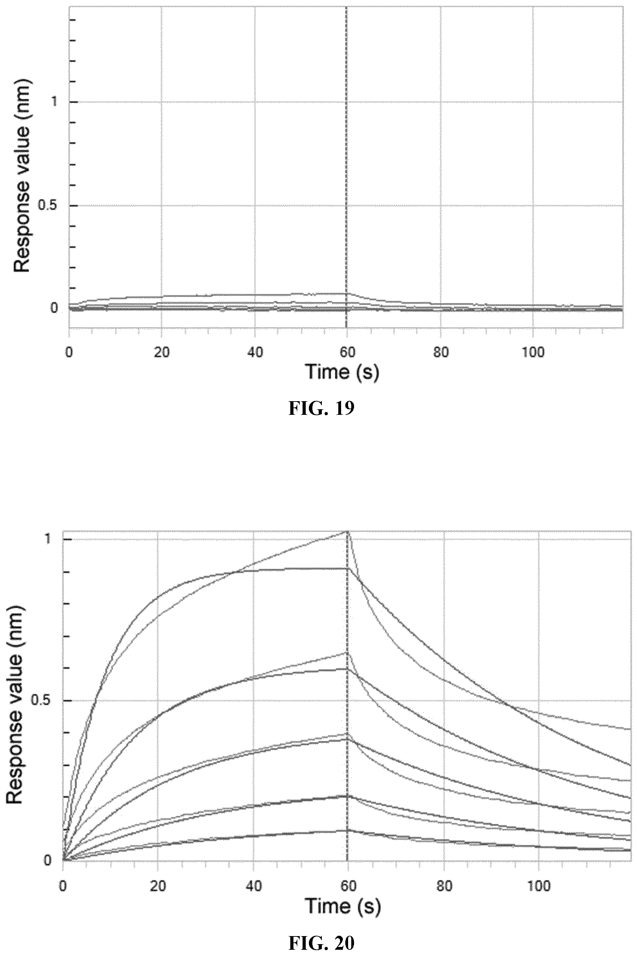

The results for the affinity constants of FcyRIIa_R131 to TF01 are shown in Table 16 and FIGs. 19-20.

Table 16: Kinetic parameters for binding of TF01 to FcyRIIa R131

| Antibody | $K_D$ (M) | Kon (1/Ms) | SE (kon) | Kdis (1/s) | SE (kdis) | Rmax (nm) |
|---|---|---|---|---|---|---|
| TF01 | N/A | N/A | N/A | N/A | N/A | N/A |
| 26B12H2L2 (hG1WT) | 3.22E-08 | 5.11E+05 | 1.38E+04 | 1.65E-02 | 2.14E-04 | 1.08-1.55 |

N/A indicates that the antibody had no binding or an extremely weak binding signal to the antigen, and thus the results were not analyzed and no corresponding data was obtained.

The results show that 26B12H2L2(hG1WT) could bind to FcγRIIa_H131 with an affinity constant of 3.22E-08M, and that TF01 had no binding or an extremely weak binding signal to FcyRIIa_R131, and thus the results were not analyzed and no corresponding data was obtained.

The results show that the binding activity of TF01 to FcyRIIa_R131 was effectively eliminated.

Example 13: Assay for Affinity Constants of FcvRIIb to TF01

The Fc receptor FcyRIIb (also known as CD32b), can bind to the Fc fragment of IgG antibodies. In the experiment, the affinity constants of the test antibodies to FcyRIIb were determined using a Fortebio Octet system to evaluate the binding capacity of TF01 to an Fc receptor.

The method for determining the affinity constants of TF01 to FcyRIIb by the Fortebio Octet system is briefly described as follows: The sample dilution buffer was a solution of 0.02% Tween-20 and 0.1% BSA in PBS, pH 7.4. 5 μg/mL of FcyRIIb was immobilized on the NTA sensor at an immobilization height of about 1.0 nm. The sensor was equilibrated in a buffer for 60 s, and the binding of the immobilized hFCGR2B-his on the sensor to the antibodies at concentrations of 12.5-200 nM (serial two-fold dilution) was determined for 60 s. The antibodies were dissociated in the buffer for 60 s. The shaking speed of the sample plate was 1000 rpm, the temperature was 30 °C and the frequency was 5.0 Hz. The data were fitted and analyzed with a 1:1 model to obtain the affinity constants.

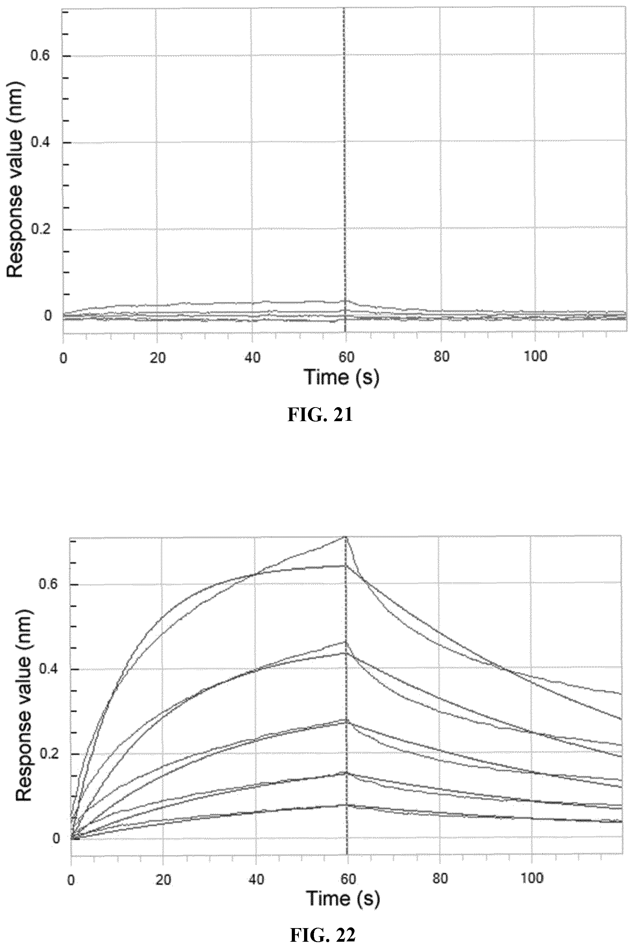

The results for the affinity constants of FcγRIIb to TF01 are shown in Table 17 and FIGs. 21-22.

Table 17: Kinetic parameters for binding of TF01 to FcγRIIb

| Antibody | $K_D$ (M) | Kon (1/Ms) | SE (kon) | Kdis (1/s) | SE (kdis) | Rmax (nm) |
|---|---|---|---|---|---|---|
| TF01 | N/A | N/A | N/A | N/A | N/A | N/A |
| 26B12H2L2 (hG1WT) | 4.12E-08 | 3.42E+05 | 9.61E+03 | 1.41E-02 | 2.04E-04 | 0.50-0.78 |

N/A indicates that the antibody had no binding or an extremely weak binding signal to the antigen, and thus the results were not analyzed and no corresponding data was obtained.

The results show that 26B12H2L2(hG1WT) could bind to FcγRIIb with an affinity constant of 4.12E-08M, and that TF01 had no binding or an extremely weak binding signal to FcγRIIb, and thus the results were not analyzed and no corresponding data was obtained.

The results show that the binding activity of TF01 to FcγRIIb was effectively eliminated.

Example 14: Assay for Affinity of TF01 for C1q

Serum complement C1q can bind to the Fc fragment of IgG antibodies and mediate CDC effects. The binding capacity of a therapeutic antibody to C1q will influence the safety and efficacy of the antibody. In the experiment, the affinity constants of TF01 to C1q were determined using a Fortebio Octet system to evaluate the CDC activity of the antibodies.

The method for determining the affinity constants of the antibodies to C1q by the Fortebio Octet system is briefly described as follows: The sample dilution buffer was a solution of 0.02% Tween-20 and 0.1% BSA in PBS, pH 7.4. 50 μg/mL of antibody was immobilized on the FAB2G sensor at an immobilization height of about 2.0 nm. The sensor was equilibrated in a buffer for 60 s, and the binding of the immobilized antibody on the sensor to the antigen C1q at concentrations of 0.625-10 nM (serial two-fold dilution) was determined for 60 s. The antigen-antibody was dissociated in the buffer for 60 s. The shaking speed of the sample plate was 1000 rpm, the temperature was 30 °C and the frequency was 5.0 Hz. The data were fitted and analyzed with a 1:1 model to obtain the affinity constants. The data acquisition software was Fortebio Data Acquisition 7.0, and the data analysis software was Fortebio Data Analysis 7.0.

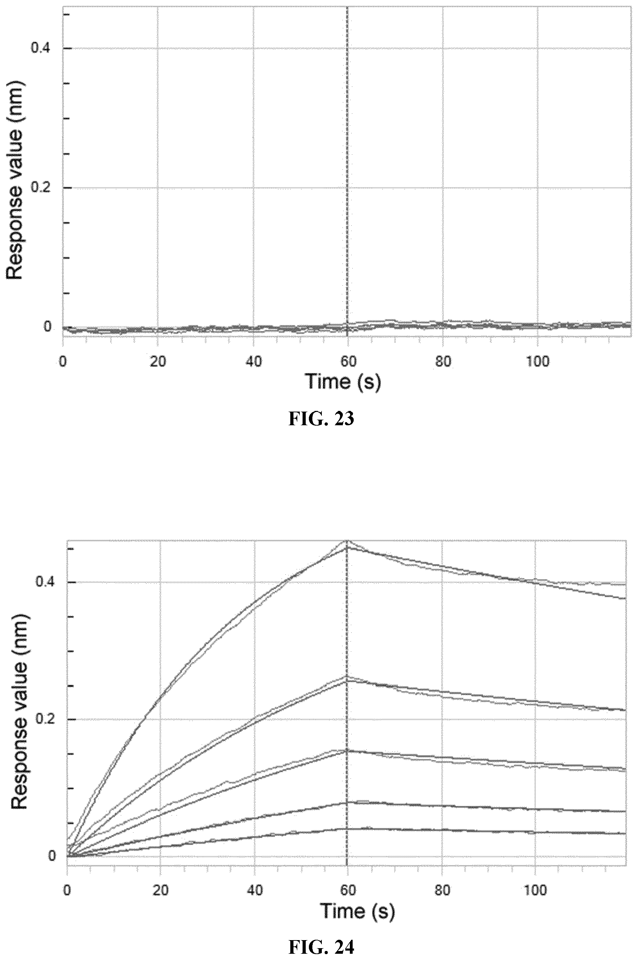

The results for the affinity constants of TF01 to C1q are shown in Table 18 and FIGs. 23-24.

Table 18: Kinetic parameters for binding of TF01 to C1q

| Antibody | $K_D$ (M) | Kon (1/Ms) | SE (kon) | Kdis (1/s) | SE (kdis) | Rmax (nm) |
|---|---|---|---|---|---|---|
| TF01 | N/A | N/A | N/A | N/A | N/A | N/A |
| 26B12H2L2 (hG1WT) | 1.28E-09 | 2.40E+06 | 4.79E+04 | 3.05E-03 | 7.33E-05 | 0.53-0.63 |

N/A indicates that the antibody had no binding or an extremely weak binding signal to the antigen, and thus the results were not analyzed and no corresponding data was obtained.

The results show that 26B12H2L2(hG1WT) could bind to C1q with an affinity constant of 1.28E-09M, and that TF01 had no binding or an extremely weak binding signal to C1q, and thus the results were not analyzed and no corresponding data was obtained.

The results show that the binding activity of TF01 to C1q was effectively eliminated.

Example 15: Antibody-Dependent Cellular Phagocytosis Activity of TF01 on CHO-K1-TIGIT

To detect the antibody-dependent cellular phagocytosis (ADCP) activity, murine macrophages were used as effector cells and cell lines overexpressing TIGIT were used as target cells. The ADCP effects mediated by the cells were tested.

The cryopreserved mouse bone marrow-derived macrophages (MBMMs) (from C57 mice) were taken out, then DMEM+10% FBS+100 ng/mL M-CSF (macrophage colony stimulating factor, peprotech, Cat. No. 315-02) was added, and the mixture was centrifuged at 1200× g for 5 min. The cells were collected, DMEM+10% FBS+100 ng/mL M-CSF culture medium was added, and the cells were thawed overnight.

The target cells (CHO-K1-TIGIT) were collected, centrifuged at 170× g for 5 min, and washed once with PBS.

Trypan blue was added for counting. 5(6)-Carboxyfluorescein N-succinimidyl ester (CFSE, Biolegend, Cat. No. 423801) was diluted to 2.5 $\mu$M with PBS to resuspend the cells (staining density: 10 million cell/mL). A proper amount of the cells were incubated in a cell incubator for 20 min. 6 mL of DMEM complete medium (containing 10% FBS) was added to stop the staining. The mixture was centrifuged at 170$\times$ g for 5 min to remove the supernatant. 1 mL of DMEM complete medium was added, and the cells were incubated in an incubator for 10 min. The antibodies were diluted to the desired concentrations with DMEM complete medium, and isotype control antibodies were designed. The target cells (150,000 cell/well) were added to a 96-well V-bottom plate, then an antibody (100 $\mu$L) was added, and the system was well mixed. The mixture was incubated on ice for 40 min, centrifuged at 170$\times$ g for 5 min, and washed twice. The macrophages (MBMMs) were collected and centrifuged at 750$\times$ g for 5 min to remove the supernatant. The cells were counted and resuspended with DMEM complete medium to adjust the concentration of macrophages (50,000 cell/100 $\mu$L), and then were added to the 96-well V-bottom plate containing the target cells for resuspension and mixing, and the mixture was incubated in an incubator at 37 °C for 2 h. 100 $\mu$L of 1% PBSA at room temperature was added to each well. The mixture was centrifuged at 750$\times$ g for 5 min to remove the supernatant. The cells were washed once with 200 $\mu$L of PBSA. APC anti-mouse/human CD11b antibody (Biolegend, Cat. No. 101212) (500-fold diluted with PBSA) was added to the corresponding samples at 100 $\mu$L/well, and the system was mixed well. Then the mixture was incubated on ice for 40 min. 100 $\mu$L of 1% PBSA was added to each well. The mixture was centrifuged at 750$\times$ g for 5 min to remove the supernatant. The cells were washed once with 200 $\mu$L of PBSA for each well. 200 $\mu$L of 1%PBSA was added to each well for resuspension, followed by testing. Macrophages in the system were APC positive, and macrophages involved in phagocytosis were APC and CFSE dual positive. The phagocytosis rate was determined as the ratio of the number of dual positive cells to the number of APC positive cells, and thus the ADCP activity was evaluated. The ADCP activity of each group, represented by P%, was calculated according to the following formula:

$$\mathbf{P\%} = \text{Virhe.} \times \mathbf{100\%}$$

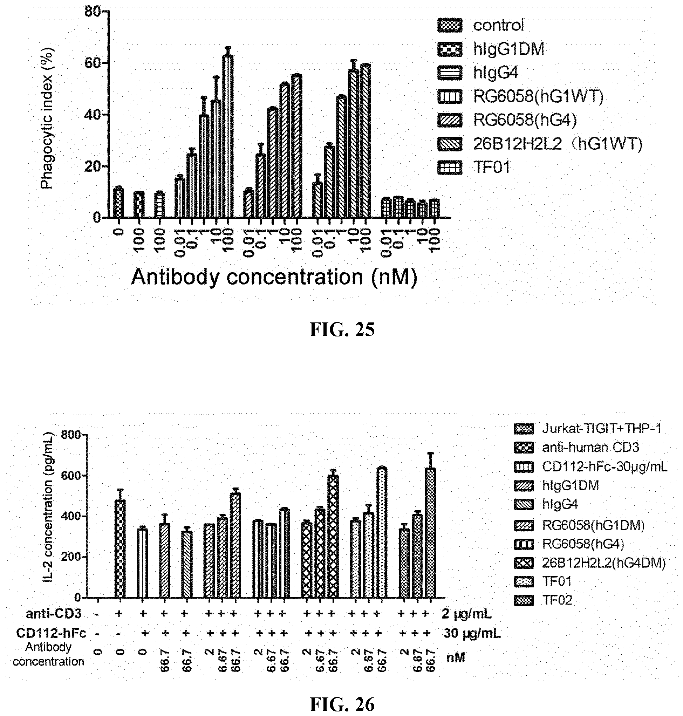

The results are shown in FIG. 25.

The results show that RG6058(hG1WT), RG6058(hG4), and 26B12H2L2(hG1WT) had ADCP effects at the same concentration; the phagocytosis rate of TF01 at the same concentration was comparable to that of the isotype control antibody group, indicating that TF01 had no ADCP effect. The results show that the amino acid mutation introduced by TF01 could effectively eliminate the ADCP effect.

Example 16: Evaluation of Blocking Activity of Fusion Protein of Anti-TIGIT Antibody and TGF-$\beta$R on CD112/CD155 Inhibition Against IL-2 Secretion in Co-Culture System

1. Evaluation of blocking activity of fusion protein of anti-TIGIT antibody and TGF-$\beta$R on CD112 inhibition against IL-2 secretion in Jurkat-TIGIT and THP-1 cell co-culture system

A 96-well plate (Corning, Model No. 3599) was coated with an anti-human CD3 antibody (prepared by Akeso Biopharma Inc., Lot No. 20170830) at 2 $\mu$g/mL at 37 °C for 2 h, then the coating solution was removed, and the plate was washed once with pre-cooled PBS (300 $\mu$L). Jurkat-TIGIT cells (constructed by Akeso Biopharma Inc.) were counted and added to a 96-well plate at 50,000 cell/well. The antibody was added according to the experimental design, and the plate was incubated at 37 °C for 30 min. CD112-hFc (prepared by Akeso Biopharma Inc., Lot No. 20180209) was added according to the experimental design. THP-1 cells (purchased from Chinese Academy of Sciences, Cat. No. 3131C0001000700057) were counted and added to a 96-well plate at 50,000 cell/well. The plate was incubated in an incubator for 48 h. The culture supernatant was collected, and the IL-2 content was tested using IL-2 ELISA kit (Dakewe, Cat. No. 1110202).

The results are shown in FIG. 26. The results show that CD112 had a significant inhibitory effect on IL-2 secretion. Antibodies 26B12H2L2(hG4DM), TF01, TF02, RG6058(hG1DM), and RG6058(hG4) could effectively block CD112 inhibition against IL-2 secretion in the system, and TF01 and TF02 had stronger activity than positive control antibodies RG6058(hG1DM) and RG6058(hG4).

2. Evaluation of blocking activity of fusion protein of anti-TIGIT antibody and TGF-$\beta$R on CD155 inhibition against IL-2 secretion in Jurkat-TIGIT and THP-1 cell co-culture system

A 96-well plate (Corning, Model No. 3599) was coated with an anti-human CD3 antibody (prepared by Akeso Biopharma Inc., Lot No. 20170830) at 2 $\mu$g/mL at 37 °C for 2 h, then the coating solution was removed, and the plate was washed once with pre-cooled PBS (300 $\mu$L). Jurkat-TIGIT cells (constructed by Akeso Biopharma Inc.) were counted and added to a 96-well plate at 50,000 cell/well. The antibody was added according to the experimental design, and the plate was incubated at 37 °C for 30 min. CD155-hFc (prepared by Akeso Biopharma Inc., Lot No. 20180209) was added according to the experimental design. THP-1 cells (purchased from Chinese Academy of Sciences, Cat. No. 3131C0001000700057) were counted and added to a 96-well plate at 50,000 cell/well. The plate was incubated in an incubator for 48 h. The culture supernatant was collected, and the IL-2 content was tested using IL-2 ELISA kit (Dakewe, Cat. No. 1110202).

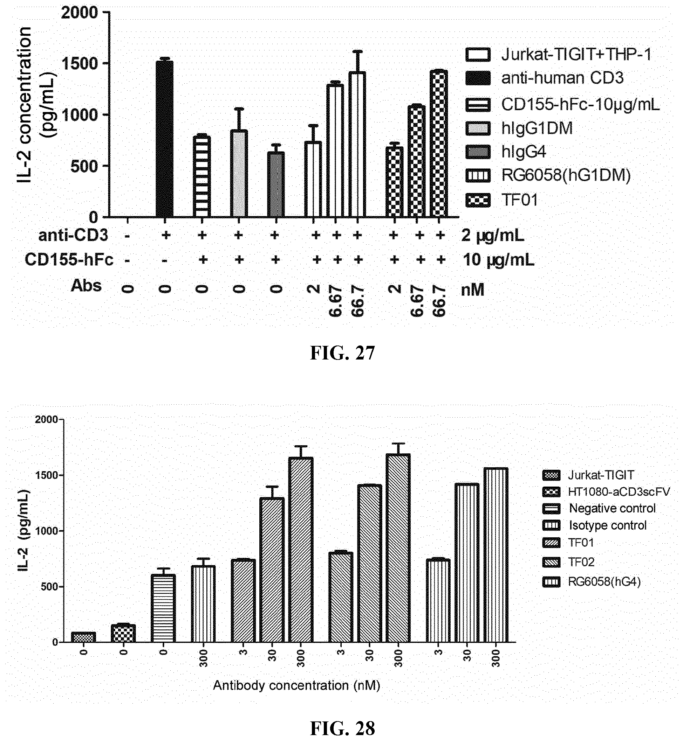

The results are shown in FIG. 27. The results show that CD155 had a significant inhibitory effect on IL-2 secretion. Antibodies TF01 and RG6058(hG1DM) could effectively block CD155 inhibition against IL-2 secretion in the system, and had comparable activity.

Example 17: Evaluation of Bioactivity of Fusion Protein of Anti-TIGIT Antibody and TGF-$\beta$R in Promotion of IL-2 Secretion in Jurkat-TIGIT and HT1080-aCD3scFv Cell Co-culture System

Jurkat-TIGIT and HT1080-aCD3scFv cells (constructed by Akeso Biopharma Inc.) in logarithmic growth phase were collected and counted, with 50,000 Jurkat-TIGIT cells for each well and 10,000 HT1080-aCD3scFv cells for each well. The diluted antibodies (antibody gradients: 3 nM, 30 nM, 300 nM) were added. A soluble anti-human CD28 antibody (3 $\mu$g/mL) (R&D, Cat. No. MAB342-500) was added. The mixture was incubated in an incubator for 48 h. The culture supernatant was collected, and the IL-2 content was tested using IL-2 ELISA kit (Dakewe, Cat. No. 1110202).

The results are shown in FIG. 28.

The results show that antibodies TF01, TF02, and RG6058(hG4) could effectively promote IL-2 secretion in the Jurkat-TIGIT and HT1080-scFv cell co-culture system, and had comparable activity.

Example 18: Evaluation of Blocking Activity of Fusion Protein of Anti-TIGIT Antibody and TGF-$\beta$R on TGF-$\beta$1 Inhibition Against IFN-v Secretion in Process of PBMC Secondary Immune Response to CMV Antigen

The PBMCs (from a healthy donor) were thawed, seeded onto a complete medium (RPMI 1640+10% FBS), and then incubated in an incubator overnight. The next day, the cells were collected and counted for determining the cell viability, and seeded onto a round-bottom 96-well plate (Corning, Cat. No. 3799) at 200,000 cell/well. Meanwhile, the gradiently diluted antibody, CMV (at a final concentration of 0.02 $\mu$g/mL, Mabtech, Cat. No. 3619-1), and TGF-$\beta$1 (at a final concentration of 3 ng/mL, Genscript, Cat. No. Z03411) were added according to the experimental design, with a final volume of 200 $\mu$L. (TGF-$\beta$1 and the antibody were incubated at 37 °C for 10 min). The mixture was incubated in an incubator for 4 days. After 4 days, the cell culture supernatant was collected, and the IFN-y content was tested using IFN-y ELISA kit (Dakewe, Cat. No. 1110002).

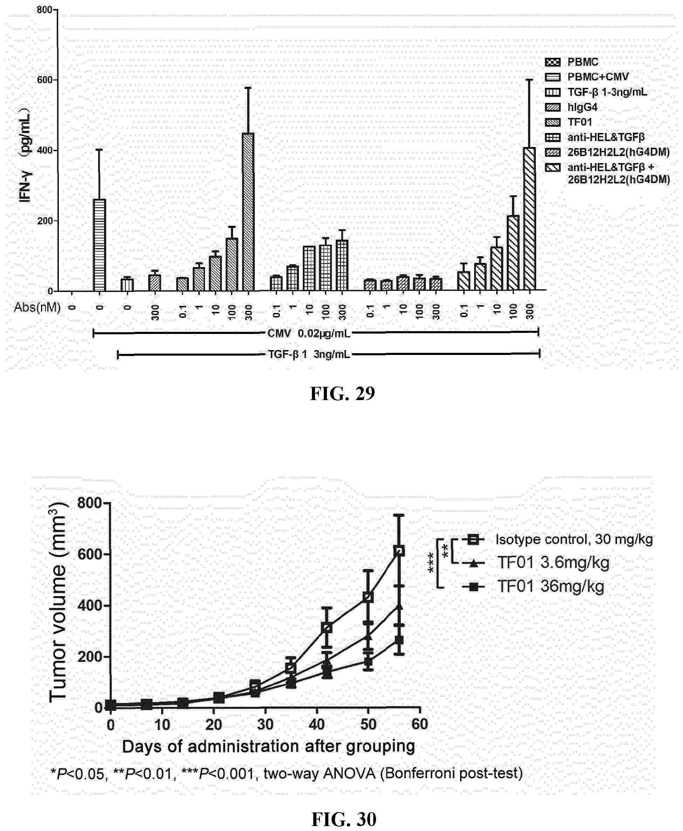

The results are shown in FIG. 29. The results show that the CMV antigen could cause the secretion of IFN-y by the PBMC secondary immune response, and TGF-$\beta$1 had a significant inhibitory effect on IFN-y secretion in the system. The combined use of antibodies TF01, anti-HEL&TGF$\beta$, and anti-HEL&TGF$\beta$+26B12H2L2(hG1DM) could effectively block TGF-$\beta$1 inhibition against IFN-y secretion in the system, and this effect could not be achieved by using 26B12H2L2(hG1DM) alone. The blocking activity of TF01 was superior to that of the control antibody anti-HEL&TGF$\beta$, comparable to that of anti-HEL&TGF$\beta$+26B12H2L2(hG1DM).

Example 19: Pharmacodynamic Evaluation of Fusion Protein of Anti-TIGIT Antibody and TGF-$\beta$R in Mouse Tumor Cell Subcutaneous Xenograft Model

To determine the anti-tumor activity *in vivo* of the fusion protein of anti-TIGIT antibody and TGF-$\beta$R, MDA-MB-231 cells (purchased from ATCC) were first inoculated subcutaneously into 6.71-9.57 week old female Scid Beige mice (purchased from Beijing Vital River Laboratory Animal Technology Co., Ltd.) at their breast fat pads. On Day 17 after inoculation, the mice were randomized into 3 groups of 8 according to tumor volume. Each mouse was injected intraperitoneally with human peripheral blood mononuclear cells (hPBMCs) and dosed, and the day of grouping was defined as D0. The route of administration was intraperitoneal injection, once per week for 6 weeks. hPBMCs were injected intraperitoneally on day 8 after grouping. The modeling and specific regimen are shown in Table 19. After the administration, the length and width of tumors in each group were measured, and the tumor volume was calculated.

Table 19: Dose regimen of fusion protein of anti-TIGIT antibody and TGF-βR for treating MDA-MB-231 xenograft tumor in Scid Beige mouse model

| Grouping | Number of animals | Inoculation | Regimen |
|---|---|---|---|
| Isotype control, 30 mg/kg | 8 | MDA-MB-231, $2 \times 10^6$ cells/Scid Beige mouse, subcutaneously inoculated at breast fat pads; grouping on day 17 after cell inoculation, $2.5 \times 10^6$ million hPBMCs were intraperitoneally injected; 3 million hPBMCs were intraperitoneally injected on day 8 after grouping. | Anti-HEL(hIgG4) (prepared by Akeso Biopharma Inc., Lot No. 20190910), 30 mg/kg; intraperitoneally injected once per week for 6 weeks |
| TF01 3.6mg/kg | 8 | | TF01 (prepared by Akeso Biopharma Inc., Lot No. A130Y20210301), 3.6 mg/kg; intraperitoneally injected once per week for 6 weeks |
| TF01 36mg/kg | 8 | | TF01 (prepared by Akeso Biopharma Inc., Lot No. A130Y20210301), 36 mg/kg; intraperitoneally injected once per week for 6 weeks |

The results are shown in FIG. 30. The results show that, compared with an isotype control antibody, the fusion protein TF01 of the anti-TIGIT antibody and TGF-βR could effectively inhibit the growth of tumors in mice.

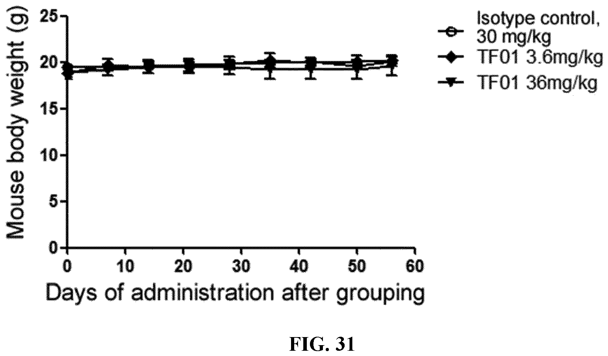

In addition, as shown in FIG. 31, the tumor-bearing mice were well resistant to the tested drug TF01, and each group had no effect on the body weight of the tumor-bearing mice.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

**Claims**

1.  A fusion protein, comprising:

    a first protein functional region targeting TIGIT, and
    a second protein functional region having TGF-β binding activity;
    wherein,
    the first protein functional region is an anti-TIGIT antibody or an antigen-binding fragment thereof;
    a heavy chain variable region of the anti-TIGIT antibody comprises HCDR1-HCDR3 with amino acid sequences set forth in SEQ ID NOs: 3-5, respectively, and a light chain variable region thereof comprises LCDR1-LCDR3 with amino acid sequences set forth in SEQ ID NOs: 8-10, respectively.

2.  The fusion protein according to claim 1, wherein the heavy chain variable region of the anti-TIGIT antibody comprises an amino acid sequence selected from SEQ ID NO: 1, SEQ ID NO: 11, SEQ ID NO: 13, SEQ ID NO: 15, and SEQ ID NO: 17; and

    the light chain variable region of the anti-TIGIT antibody comprises an amino acid sequence selected from SEQ ID NO: 6, SEQ ID NO: 19, SEQ ID NO: 21, SEQ ID NO: 23, and SEQ ID NO: 25.

3.  The fusion protein according to any one of claims 1 to 2, wherein

    the heavy chain variable region of the anti-TIGIT antibody comprises an amino acid sequence set forth in SEQ ID NO: 1, and the light chain variable region thereof comprises an amino acid sequence set forth in SEQ ID NO: 6;
    the heavy chain variable region of the anti-TIGIT antibody comprises an amino acid sequence set forth in SEQ ID NO: 11, and the light chain variable region thereof comprises an amino acid sequence set forth in SEQ ID NO: 19;

the heavy chain variable region of the anti-TIGIT antibody comprises an amino acid sequence set forth in SEQ ID NO: 17, and the light chain variable region thereof comprises an amino acid sequence set forth in SEQ ID NO: 19;

the heavy chain variable region of the anti-TIGIT antibody comprises an amino acid sequence set forth in SEQ ID NO: 13, and the light chain variable region thereof comprises an amino acid sequence set forth in SEQ ID NO: 21;

the heavy chain variable region of the anti-TIGIT antibody comprises an amino acid sequence set forth in SEQ ID NO: 13, and the light chain variable region thereof comprises an amino acid sequence set forth in SEQ ID NO: 23;

the heavy chain variable region of the anti-TIGIT antibody comprises an amino acid sequence set forth in SEQ ID NO: 15, and the light chain variable region thereof comprises an amino acid sequence set forth in SEQ ID NO: 21;

the heavy chain variable region of the anti-TIGIT antibody comprises an amino acid sequence set forth in SEQ ID NO: 15, and the light chain variable region thereof comprises an amino acid sequence set forth in SEQ ID NO: 23;

the heavy chain variable region of the anti-TIGIT antibody comprises an amino acid sequence set forth in SEQ ID NO: 11, and the light chain variable region thereof comprises an amino acid sequence set forth in SEQ ID NO: 25; or

the heavy chain variable region of the anti-TIGIT antibody comprises an amino acid sequence set forth in SEQ ID NO: 17, and the light chain variable region thereof comprises an amino acid sequence set forth in SEQ ID NO: 25.

4. The fusion protein according to any one of claims 1 to 3, wherein the anti-TIGIT antibody or the antigen-binding fragment thereof is selected from Fab, Fab', F(ab')$_2$, Fd, Fv, dAb, a complementarity determining region fragment, a single chain antibody, a humanized antibody, a chimeric antibody, and a diabody.

5. The fusion protein according to any one of claims 1 to 4, wherein
the anti-TIGIT antibody comprises a non-CDR region derived from a species other than murine, such as from a human antibody.

6. The fusion protein according to any one of claims 1 to 5, wherein a heavy chain constant region of the anti-TIGIT antibody is Ig gamma-1 chain C region (e.g., NCBI ACCESSION: P01857) or Ig gamma-4 chain C region (e.g., NCBI ACCESSION: P01861.1); a light chain constant region of the anti-TIGIT antibody is Ig kappa chain C region (e.g., NCBI ACCESSION: P01834).

7. The fusion protein according to any one of claims 1 to 6, wherein the fusion protein has a stronger capacity of binding to a ligand CD155-hFc-Biotin than a control antibody RG6058(hG4).

8. The fusion protein according to any one of claims 1 to 7, wherein the fusion protein binds to TIGIT-mFc with an $EC_{50}$ of less than 0.08 nM or less than 0.10 nM; preferably, the $EC_{50}$ is measured by indirect ELISA.

9. The fusion protein according to any one of claims 1 to 8, wherein the fusion protein binds to TGF-β1, TGF-β2, or TGF-β3 with an $EC_{50}$ of less than 0.3 nM, less than 0.4 nM, less than 0.5 nM, or less than 0.6 nM; preferably, the $EC_{50}$ is measured by indirect ELISA.

10. The fusion protein according to any one of claims 1 to 9, wherein the anti-TIGIT antibody is an antibody produced by a hybridoma cell line LT019 deposited at China Center for Type Culture Collection (CCTCC) under CCTCC NO. C2020208.

11. The fusion protein according to any one of claims 1 to 10, wherein according to the EU numbering system, the heavy chain constant region of the anti-TIGIT antibody has one of the combinations of the following mutations:

L234A and L235A;
L234A and G237A;
L235A and G237A; or
L234A, L235A, and G237A.

12. The fusion protein according to any one of claims 1 to 11, wherein

the second protein functional region is a TGF-β receptor, an extracellular region thereof, or a truncated fragment of a TGF-β receptor extracellular region with TGF-β receptor functions;
preferably, the TGF-β receptor is TGF-βRI, TGF-βRII, or TGF-βRIII, or is an extracellular region of TGF-βRI, TGF-βRII, or TGF-βRIII, or is a truncated fragment of the extracellular region of TGF-βRI, TGF-βRII, or TGF-βRIII;
preferably, the second protein functional region comprises an amino acid sequence set forth in any one of SEQ ID NOs: 33-39.

13. The fusion protein according to any one of claims 1 to 12, wherein
a heavy chain of the anti-TIGIT antibody comprises an amino acid sequence set forth in SEQ ID NO: 27 or SEQ ID NO: 31, and a light chain thereof comprises an amino acid sequence set forth in SEQ ID NO: 29.

14. The fusion protein according to any one of claims 1 to 13, wherein the first protein functional region and the second protein functional region are linked directly or via a linker fragment.

15. The fusion protein according to claim 14, wherein the linker fragment is (GGGGS)n or (GGGGS)nG, n being a positive integer; preferably, n is 1, 2, 3, 4, 5, or 6.

16. The fusion protein according to any one of claims 1 to 15, wherein the numbers of the first protein functional region and the second protein functional region are each independently 1, 2, or more.

17. The fusion protein according to any one of claims 1 to 16, wherein the TGF-β receptor or the extracellular fragment thereof is linked to the C-terminus of the heavy chain of the anti-TIGIT antibody.

18. A fusion protein, comprising:

a first protein functional region targeting TIGIT, and
a second protein functional region having TGF-β binding activity;
wherein,
the number of the first protein functional region is 1, and the number of the second protein functional region is 2;
the first protein functional region is an anti-TIGIT antibody or an antigen-binding fragment thereof, and the second protein functional region is a TGF-β receptor, an extracellular region thereof, or a truncated fragment of a TGF-β receptor extracellular region with TGF-β receptor functions;
a heavy chain of the anti-TIGIT antibody comprises an amino acid sequence set forth in SEQ ID NO: 27 or SEQ ID NO: 31, and a light chain thereof comprises an amino acid sequence set forth in SEQ ID NO: 29;
the second protein functional region comprises an amino acid sequence set forth in SEQ ID NO: 33;
the second protein functional region is linked to the C-terminus of the heavy chain of the anti-TIGIT antibody via a linker fragment;
preferably, the linker fragment comprises an amino acid sequence set forth in SEQ ID NO: 44.

19. An isolated nucleic acid molecule, encoding the fusion protein according to any one of claims 1 to 18.

20. A vector, comprising the isolated nucleic acid molecule according to claim 19.

21. A host cell, comprising the isolated nucleic acid molecule according to claim 19 or the vector according to claim 20.

22. A conjugate, comprising a fusion protein moiety and a conjugated moiety, wherein the fusion protein moiety is the fusion protein according to any one of claims 1 to 18, and the conjugated moiety is a detectable label; preferably, the conjugated moiety is a radioisotope, a fluorescent substance, a luminescent substance, a colored substance, or an enzyme.

23. Use of the fusion protein according to any one of claims 1 to 18 or the conjugate according to claim 22 in the preparation of a kit for detecting the presence or level of TIGIT and/or TGF-β in a sample.

24. A pharmaceutical composition, comprising the fusion protein according to any one of claims 1 to 18 or the conjugate according to claim 22, wherein, optionally, the pharmaceutical composition further comprises a pharmaceutically acceptable carrier and/or excipient.

**25.** The pharmaceutical composition according to claim 24, further comprising one or more anti-tumor chemotherapeutic drugs; wherein

preferably, the anti-tumor chemotherapeutic drug is a tyrosine kinase inhibitor; more preferably, the anti-tumor chemotherapeutic drug is anlotinib or a pharmaceutically acceptable salt thereof (e.g., hydrochloride salt), or lenvatinib or a pharmaceutically acceptable salt thereof (e.g., mesylate salt).

**26.** The pharmaceutical composition according to claim 24 or 25, wherein the unit dose of the pharmaceutical composition is 100 mg-1500 mg, 200 mg-1000 mg, 200 mg-800 mg, 300 mg-600 mg, 400 mg-500 mg, or 450 mg, based on the mass of the fusion protein.

**27.** A combination product, comprising a first product and a second product in separate packages, wherein,

the first product comprises the fusion protein according to any one of claims 1 to 18, the conjugate according to claim 22, or the pharmaceutical composition according to any one of claims 24 to 26; the second product comprises one or more anti-tumor chemotherapeutic drugs; preferably, the anti-tumor chemotherapeutic drug is a tyrosine kinase inhibitor; more preferably, the anti-tumor chemotherapeutic drug is anlotinib or a pharmaceutically acceptable salt thereof (e.g., hydrochloride salt), or lenvatinib or a pharmaceutically acceptable salt thereof (e.g., mesylate salt); preferably, the first product and the second product further independently comprise one or more pharmaceutically acceptable auxiliary materials; preferably, the combination product further comprises a package insert.

**28.** The combination product according to claim 27, wherein the unit dose of the first product is 100 mg-1500 mg, 200 mg-1000 mg, 200 mg-800 mg, 300 mg-600 mg, 400 mg-500 mg, or 450 mg, based on the mass of the fusion protein.

**29.** The combination product according to claim 27 or 28, wherein the unit dose of the second product is 0.1 mg-100 mg, 0.5 mg-50 mg, 1 mg-20 mg, 2 mg-15 mg, 4 mg-12 mg, or 8 mg-12 mg, based on the mass of an active ingredient.

**30.** Use of the fusion protein according to any one of claims 1 to 18 or the conjugate according to claim 22 in the preparation of a medicament for treating and/or preventing a tumor; wherein

preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma; preferably, the non-small cell lung cancer is advanced non-small cell lung cancer.

**31.** The fusion protein according to any one of claims 1 to 18 or the conjugate according to claim 22 for use in the treatment and/or prevention of a tumor; wherein

preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma; preferably, the non-small cell lung cancer is advanced non-small cell lung cancer.

**32.** A method for treating and/or preventing a tumor, comprising a step of administering to a subject in need thereof an effective amount of the fusion protein according to any one of claims 1 to 18 or the conjugate according to claim 22; wherein

preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma; preferably, the non-small cell lung cancer is advanced non-small cell lung cancer.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

**FIG. 15**

**FIG. 16**

**FIG. 17**

**FIG. 18**

**FIG. 19**

**FIG. 20**

**FIG. 21**

**FIG. 22**

**FIG. 23**

**FIG. 24**

**FIG. 25**

**FIG. 26**

**FIG. 27**

**FIG. 28**

**FIG. 29**

*P<0.05, **P<0.01, ***P<0.001, two-way ANOVA (Bonferroni post-test)

**FIG. 30**

**FIG. 31**

## INTERNATIONAL SEARCH REPORT

| International application No. |
| --- |
| **PCT/CN2022/113882** |

### A. CLASSIFICATION OF SUBJECT MATTER

C07K 19/00(2006.01)i; C07K 14/71(2006.01)i; C07K 14/495(2006.01)i; C07K 16/28(2006.01)i; A61K 38/17(2006.01)i; A61P 35/00(2006.01)i; A61K 39/00(2006.01)i

According to International Patent Classification (IPC) or to both national classification and IPC

### B. FIELDS SEARCHED

Minimum documentation searched (classification system followed by classification symbols)

C07K A61K A61P

Documentation searched other than minimum documentation to the extent that such documents are included in the fields searched

Electronic data base consulted during the international search (name of data base and, where practicable, search terms used)

CNABS, CNTXT, SIPOABS, DWPI, VEN, WOTXT, USTXT, EPTXT, CNKI, 万方数据库, WANFANG DATABASE, baidu 学术, BAIDU SCHOLAR, PUBMED, ELSEVIER, SPRINGER, ISI web of knowledge, sciencedirect, NCBI, Genbank, EMBL, STN, 中国专利生物序列检索系统, Chinese Patent Biological Sequence Retrieval System: TIGIT, T cell Ig and ITIM domain, WUCAM, Vstm3, VSIG9, lt019, tigit-26b12, CCTTCC NO: C2020208, TGF-β, 转化生长因子-β, transforming growth factor-β, 中山康方生物医药有限公司, akeso biopharma, 发明人, SEQ ID NOs: 1-39

### C. DOCUMENTS CONSIDERED TO BE RELEVANT

| Category* | Citation of document, with indication, where appropriate, of the relevant passages | Relevant to claim No. |
| --- | --- | --- |
| A | CN 108883164 A (JN BIOSCIENCES LLC et al.) 23 November 2018 (2018-11-23) entire document | 1-32 |
| A | CN 108290946 A (F. HOFFMANN-LA ROCHE AG.) 17 July 2018 (2018-07-17) entire document | 1-32 |
| A | US 2018/0251548 A1 (COMPASS THERAPEUTICS LLC) 06 September 2018 (2018-09-06) entire document | 1-32 |
| A | CN 110050000 A (JIANGSU HENGRUI MEDICINE CO., LTD. et al.) 23 July 2019 (2019-07-23) entire document | 1-32 |
| A | WO 2021/101796 A1 (THE BRIGHAM AND WOMEN'S HOSPITAL, INC.) 27 May 2021 (2021-05-27) entire document | 1-32 |

☑ Further documents are listed in the continuation of Box C.   ☑ See patent family annex.

| | |
| --- | --- |
| \* Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **19 September 2022** | **28 September 2022** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/113882** |

**C.    DOCUMENTS CONSIDERED TO BE RELEVANT**

| Category* | Citation of document, with indication, where appropriate, of the relevant passages | Relevant to claim No. |
|---|---|---|
| A | WO 2021/093760 A1 (JIANGSU HENGRUI MEDICINE CO., LTD. et al.) 20 May 2021 (2021-05-20)<br>    entire document | 1-32 |
| A | LAN, Yan et al. "Enhanced Preclinical Antitumor Activity of M7824, a Bifunctional Fusion Protein Simultaneously Targeting PD-L1 and TGF-β"<br>*Science Translational Medicine,* Vol. 10, 17 January 2018 (2018-01-17),<br>    eaan5488, pp. 1-15 | 1-32 |
| PA | CN 114181310 A (AKESO BIOPHARMA CO., LTD.) 15 March 2022 (2022-03-15)<br>    entire document | 1-32 |
| PA | CN 114478769 A (AKESO BIOPHARMA CO., LTD.) 13 May 2022 (2022-05-13)<br>    entire document | 1-32 |
| PA | CN 114106182 A (AKESO BIOPHARMA CO., LTD.) 01 March 2022 (2022-03-01)<br>    entire document | 1-32 |

Form PCT/ISA/210 (second sheet) (January 2015)

<div style="text-align:center">**INTERNATIONAL SEARCH REPORT**</div>

| International application No. |
| --- |
| **PCT/CN2022/113882** |

---

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

---

Form PCT/ISA/210 (continuation of first sheet) (January 2015)

## INTERNATIONAL SEARCH REPORT

International application No.

**PCT/CN2022/113882**

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **32**
   because they relate to subject matter not required to be searched by this Authority, namely:

   [1]   Claim 32 relates to a method for treating and/or preventing tumors, and relates to the subject matter as defined in PCT Rule 39.1 that does not warrant a search conducted by the international searching authority: (iv) methods for the treatment of a human or animal body by surgery or therapy, as well as diagnostic methods. An international search was formed on the basis of a use of the fusion protein or conjugate in the preparation of a drug for treating and/or preventing the tumors.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

| International application No. |
| --- |
| **PCT/CN2022/113882** |

| Patent document cited in search report | | | Publication date (day/month/year) | Patent family member(s) | | | Publication date (day/month/year) |
| --- | --- | --- | --- | --- | --- | --- | --- |
| CN | 108883164 | A | 23 November 2018 | US | 2020297844 | A1 | 24 September 2020 |
| | | | | EP | 3423089 | A1 | 09 January 2019 |
| | | | | AU | 2017228474 | A1 | 30 August 2018 |
| | | | | TW | 201734043 | A | 01 October 2017 |
| | | | | US | 2017281764 | A1 | 05 October 2017 |
| | | | | MX | 2018010485 | A | 10 January 2019 |
| | | | | KR | 20220024837 | A | 03 March 2022 |
| | | | | UY | 37141 | A | 29 September 2017 |
| | | | | JP | 2019513817 | A | 30 May 2019 |
| | | | | UA | 125898 | C2 | 06 July 2022 |
| | | | | SG | 11201806992V | A | 27 September 2018 |
| | | | | IL | 261188 | A | 31 October 2018 |
| | | | | WO | 2017152088 | A1 | 08 September 2017 |
| | | | | BR | 112018067458 | A2 | 02 January 2019 |
| | | | | CA | 3014934 | A1 | 08 September 2017 |
| | | | | PH | 12018501778 | A1 | 15 May 2019 |
| | | | | KR | 20180119653 | A | 02 November 2018 |
| | | | | MY | 191649 | A | 05 July 2022 |
| CN | 108290946 | A | 17 July 2018 | WO | 2017053748 | A2 | 30 March 2017 |
| | | | | NZ | 739750 | A | 29 November 2019 |
| | | | | CL | 2018000744 | A1 | 06 July 2018 |
| | | | | AU | 2019246814 | A1 | 31 October 2019 |
| | | | | BR | 112018005862 | A2 | 16 October 2018 |
| | | | | UA | 124573 | C2 | 13 October 2021 |
| | | | | CN | 113956358 | A | 21 January 2022 |
| | | | | CA | 2994858 | A1 | 30 March 2017 |
| | | | | AU | 2016325610 | A1 | 01 March 2018 |
| | | | | MX | 2018003633 | A | 01 August 2018 |
| | | | | EP | 3353210 | A2 | 01 August 2018 |
| | | | | CL | 2020000938 | A1 | 14 August 2020 |
| | | | | JP | 2020074778 | A | 21 May 2020 |
| | | | | CR | 20180225 | A | 09 July 2018 |
| | | | | KR | 20180053742 | A | 23 May 2018 |
| | | | | US | 2021253693 | A1 | 19 August 2021 |
| | | | | KR | 20220104289 | A | 26 July 2022 |
| | | | | JP | 2018532397 | A | 08 November 2018 |
| | | | | MA | 42925 | A | 01 August 2018 |
| | | | | RU | 2018114523 | A3 | 25 October 2019 |
| | | | | ZA | 201800941 | B | 29 May 2019 |
| | | | | AU | 2021250946 | A1 | 11 November 2021 |
| | | | | SG | 10202007764T | A | 29 September 2020 |
| | | | | US | 2017088613 | A1 | 30 March 2017 |
| | | | | PH | 12018500608 | A1 | 01 October 2018 |
| | | | | CN | 113912724 | A | 11 January 2022 |
| | | | | IL | 257636 | A | 30 April 2018 |
| | | | | CO | 2018004090 | A2 | 30 November 2018 |
| | | | | PE | 20181046 | A1 | 03 July 2018 |
| | | | | US | 2018186875 | A1 | 05 July 2018 |
| | | | | US | 2021032328 | A1 | 04 February 2021 |
| | | | | JP | 2021166524 | A | 21 October 2021 |

Form PCT/ISA/210 (patent family annex) (January 2015)

| | | | INTERNATIONAL SEARCH REPORT<br>Information on patent family members | | | International application No.<br>**PCT/CN2022/113882** | | |
|---|---|---|---|---|---|---|---|---|

| Patent document<br>cited in search report | | | Publication date<br>(day/month/year) | Patent family member(s) | | | Publication date<br>(day/month/year) |
|---|---|---|---|---|---|---|---|
| | | | | HK | 1258058 | A1 | 01 November 2019 |
| | | | | KR | 20200087283 | A | 20 July 2020 |
| | | | | US | 2022064286 | A1 | 03 March 2022 |
| | | | | RU | 2020120073 | A | 03 July 2020 |
| | | | | US | 2019119376 | A1 | 25 April 2019 |
| | | | | TW | 201718644 | A | 01 June 2017 |
| US | 2018/0251548 | A1 | 06 September 2018 | JP | 2018531914 | A | 01 November 2018 |
| | | | | WO | 2017048824 | A1 | 23 March 2017 |
| | | | | EP | 3349792 | A1 | 25 July 2018 |
| | | | | CA | 2998589 | A1 | 23 March 2017 |
| | | | | AU | 2016322934 | A1 | 12 April 2018 |
| CN | 110050000 | A | 23 July 2019 | TW | 201900674 | A | 01 January 2019 |
| | | | | KR | 20200004801 | A | 14 January 2020 |
| | | | | EP | 3623389 | A1 | 18 March 2020 |
| | | | | MX | 2019013023 | A | 18 December 2019 |
| | | | | JP | 2020519289 | A | 02 July 2020 |
| | | | | WO | 2018205985 | A1 | 15 November 2018 |
| | | | | AU | 2018264455 | A1 | 14 November 2019 |
| | | | | BR | 112019023184 | A2 | 19 May 2020 |
| | | | | CA | 3061791 | A1 | 29 October 2019 |
| | | | | US | 2020157180 | A1 | 21 May 2020 |
| WO | 2021/101796 | A1 | 27 May 2021 | None | | | |
| WO | 2021/093760 | A1 | 20 May 2021 | TW | 202128763 | A | 01 August 2021 |
| CN | 114181310 | A | 15 March 2022 | None | | | |
| CN | 114478769 | A | 13 May 2022 | WO | 2022089392 | A1 | 05 May 2022 |
| CN | 114106182 | A | 01 March 2022 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4816567 A

- CN 108290946 A


**Non-patent literature cited in the description**

- **YU X ; HARDEN K ; GONZALEZ L C et al.** The surface protein TIGIT suppresses T cell activation by promoting the generation of mature immunoregulatory dendritic cells. [J]. *Nature immunology,* 2009, vol. 10 (1), 48
- **MARTINET L ; SMYTH M J.** Balancing natural killer cell activation through paired receptors.[J]. *Nature Reviews Immunology,* 2015, vol. 15 (4), 243-254
- **STANIETSKY N ; SIMIC H ; ARAPOVIC J et al.** The interaction of TIGIT with PVR and PVRL2 inhibits human NK cell cytotoxicity.[J]. *Proceedings of the National Academy of Sciences,* 2009, vol. 106 (42), 17858-17863
- **JOHNSTON R J ; COMPS-AGRAR L ; HACKNEY J et al.** The immunoreceptor TIGIT regulates antitumor and antiviral CD8+ T cell effector function.[J. *Cancer cell,* 2014, vol. 26 (6), 923-937
- **ZHANG Q ; BI J ; ZHENG X et al.** Blockade of the checkpoint receptor TIGIT prevents NK cell exhaustion and elicits potent anti-tumor immunity.[J]. *Nature immunology,* 2018, vol. 19 (7), 723-732
- **LI X-Y ; DAS I ; LEPLETIER A et al.** CD155 loss enhances tumor suppression via combined host and tumor-intrinsic mechanisms. *J Clin Invest,* 2018, vol. 128, 2613-25
- **WHELAN S ; OPHIR E ; KOTTURI MF et al.** PVRIG and PVRL2 Are Induced in Cancer and Inhibit CD8+ T-cell function. *Cancer Immunol Res,* 2019, vol. 7, 257-68
- **J MASSAGUÉ.** TGFbeta in Cancer. [J]. *Cell,* 2008, vol. 134 (2), 215-230
- **CARL-HENRIK HELDIN1 ; ARISTIDIS MOUSTA-KAS.** *Cold Spring Harb Perspect Biol,* 2016
- **SANG XIAOHONG et al.** Advances in researches on small molecule inhibitors targeting TGF-β and receptors. [J]. *Journal of Pharmacy,* 2019, vol. 9
- **EDUARD ; BATLLE ; JOAN et al.** Transforming Growth Factor-β Signaling in Immunity and Cancer. [J. *Immunity,* 2019

- **CHEN DAN ; RAN YAN.** Advance in researches on the regulation and control of tumor cells and tumor-associated immune cells by TGF-β.[J. *Modern Medicine & Health,* 2020, vol. 36 (09), 1354-1358
- **HOGARTH PM ; PIETERSZ GA.** *NATURE REVIEWS DRUG DISCOVERY,* 2012, vol. 11 (4), 311-331
- **EHRENMANN F ; KAAS Q ; LEFRANC M P.** IMGT/3Dstructure-DB and IMGT/DomainGapAlign: a database and a tool for immunoglobulins or antibodies, T cell receptors, MHC, IgSF and MhcSF.[J]. *Nucleic acids research,* 2009, vol. 38 (1), D301-D307
- **KABAT.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **KÖHLER G ; MILSTEIN C.** Continuous cultures of fused cells secreting antibody of predefined specificity.[J]. *Nature,* 1975, vol. 256 (5517), 495
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **CLARK.** *Immunol. Today,* 2000, vol. 21, 397-402
- **HOLLIGER P. et al.** *Proc. Natl. Acad. Sci. USA,* 1993, vol. 90, 6444-6448
- **POLJAK R.J. et al.** *Structure,* 1994, vol. 2, 1121-1123
- Antibodies: A Laboratory Manual. Cold Spring Harbor Press
- **BIRD et al.** *Science,* 1988, vol. 242, 423-426
- **HUSTON et al.** *Proc. Natl. Acad. Sci. USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731

- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunology, Immunotherapy,* 2001, vol. 50 (1), 51-59
- Remington's Pharmaceutical Sciences. Mack Publishing Company, 1995
- **ACIERNO ; ACIERNO et al.** Affinity maturation increases the stability and plasticity of the Fv domain of anti-protein antibodies. *J Mol Biol.,* 2007, vol. 374 (1), 130-46
- **DULL T ; ZUFFEREY R ; KELLY M ; MANDEL RJ ; NGUYEN M ; TRONO D ; NALDINI L.** *J Virol.,* 1998, vol. 72 (11), 8463-8471
- **DULL T ; ZUFFEREY R ; KELLY M ; MANDEL RJ ; NGUYEN M ; TRONO D ; NALDINI L.** A Third Generation Lentivirus Vector with a Conditional Packaging System. *J Virol.,* 1998, vol. 72 (11), 8463-8471
- **DULL T ; ZUFFEREY R ; KELLY M ; MANDEL RJ ; NGUYEN M ; TRONO D ; NALDINI L.** A Third Generation Lentivirus Vector with a Conditional Packaging System. *J Virol.,* 1998, vol. 72 (11), 8463-8471
- Molecular Cloning: A Laboratory Manual